# EUROPEAN PATENT APPLICATION

(11) **EP 3 524 698 A1**
(43) Date of publication of application: **14.08.2019**
(21) Application number: 19165007.6
(22) Date of filing: 07.10.2014
(51) Int. Cl.: C12Q 1/6886

(54) **METHOD FOR THE PROGNOSIS AND TREATMENT OF CANCER METASTASIS**

(30) Priority: 09.10.2013 US 201361888984 P
(62) Divisional of application: 14824529.3
(71) Applicant: Fundació Institut de Recerca Biomèdica IRB (Barcelona), 08028 Barcelona (ES); Institució Catalana de Recerca I Estudis Avançats, 08028 Barcelona (ES)
(72) Inventor: GOMIS, Roger, E-08021 Barcelona (ES); PLANET, Evarist, E-08360 Canet De Mar, Barcelona (ES); PAVLOVIC, Milica, 14224 Lajkovac (RS); ARNAL, Anna, E-08021 Barcelona (ES); TARRAGONA, Maria, E-08006 Barcelona (ES)
(74) Representative: ABG Intellectual Property, S.L.

(57) **Abstract**

The present invention relates to a method for the prognosis of bone metastasis in HER2+ breast cancer, which comprises determining if the MAF gene is amplified in a primary tumor sample. Likewise, the present invention also relates to a method for determining the tendency to develop bone metastasis with respect to metastasis in other organs, which comprise determining the MAF gene expression level, amplification or translocation. The present invention also relates to a method for predicting early bone metastasis in a subject suffering breast cancer. The present invention also relates to a c-Maf inhibitor as therapeutic agent for use in the treatment of HER2+ breast cancer metastasis. The present invention relates to kits for predicting bone metastasis and predicting the clinical outcome of a subject suffering from bone metastasis. Finally, the present invention relates to a method for typing of a subject suffering breast cancer and for classifying a subject from breast cancer into a cohort.

SEQUENCE LISTING

<110>  Fundacio Institut de Recerca Biomedica (IRB Barcelona)
        Institucio Catalana de Recerca i Estudis Avancats

<120>  METHOD FOR THE PROGNOSIS AND TREATMENT OF CANCER METASTASIS

<130>  P13212EPPC01

<150>  61/888,984
<151>  2013-10-09

<160>  13

<170>  PatentIn version 3.5

<210>  1
<211>  6878
<212>  DNA
<213>  Homo sapiens

<400>  1
agaggcttta aaatcttttt tcatcttcta gctgtagctc gggctgcttg tcggcttggc      60

ctccccctcc cccctttgct ctctgcctcg tctttcccca ggacttcgct attttgcttt     120

tttaaaaaaa ggcaagaaag aactaaactc ccccctccct ctcctccagt cgggctgcac     180

ctctgccttg cactttgcac agaggtagag agcgcgcgag ggagagagag gaaagaaaaa     240

aaataataaa gagagccaag cagaagagga ggcgagaagc atgaagtgtt aactcccccg     300

tgccaaggcc cgcgccgccc ggacagacgc ccgccgcgcc tccagccccg agcggacgcc     360

gcgcgcgccc tgcctgcagc ccgggccggc gaggcgagcc cttccttatg caaagcgcgc     420

agcggagcgg cgagcggggg acgccgcgca ccgggccggg ctcctccagc ttcgccgccg     480

cagccaccac cgccgccacc gcagctcgcg gaggatcttc ccgagcctga agccgccggc     540

tcggcgcgca aggaggcgag cgagcaagga ggggccgggg cgagcgaggg agcacattgg     600

cgtgagcagg ggggagggag ggcgggcgcg gggggcgcgg gcagggcggg ggggtgtgtg     660

tgtgagcgcg ctcggaggtt tcgggccagc caccgccgcg caagctagaa gcgccccagc     720

ccggcaagct ggctcacccg ctggccaccc agcacagccc gctggcccct ctcctgcagc     780

ccatctggcg gagcggcggc ggcggcggcg gcggcggcag gagaatggca tcagaactgg     840

caatgagcaa ctccgacctg cccaccagtc ccctggccat ggaatatgtt aatgacttcg     900

atctgatgaa gtttgaagtg aaaaaggaac cggtggagac cgaccgcatc atcagccagt     960

gcggccgtct catcgccggg ggctcgctgt cctccacccc catgagcacg ccgtgcagct    1020

cggtgccccc ttcccccagc ttctcggcgc ccagcccggg ctcgggcagc gagcagaagg    1080

cgcacctgga agactactac tggatgaccg gctacccgca gcagctgaac cccgaggcgc    1140

tgggcttcag ccccgaggac gcggtcgagg cgctcatcag caacagccac cagctccagg    1200

gcggcttcga tggctacgcg cgcggggcgc agcagctggc cgcggcggcc ggggccggtg 1260

ccggcgcctc cttgggcggc agcggcgagg agatgggccc cgccgccgcc gtggtgtccg 1320

ccgtgatcgc cgcggccgcc gcgcagagcg gcgcgggccc gcactaccac caccaccacc 1380

accacgccgc cggccaccac caccacccga cggccggcgc gcccggcgcc gcgggcagcg 1440

cggccgcctc ggccggtggc gctgggggcg cgggcggcgg tggcccggcc agcgctgggg 1500

gcggcggcgg cggcggcggc ggcggaggcg gcgggggcgc ggcgggggcg gggggcgccc 1560

tgcacccgca ccacgccgcc ggcggcctgc acttcgacga ccgcttctcc gacgagcagc 1620

tggtgaccat gtctgtgcgc gagctgaacc ggcagctgcg cggggtcagc aaggaggagg 1680

tgatccggct gaagcagaag aggcggaccc tgaaaaaccg cggctatgcc cagtcctgcc 1740

gcttcaagag ggtgcagcag agacacgtcc tggagtcgga gaagaaccag ctgctgcagc 1800

aagtcgacca cctcaagcag gagatctcca ggctggtgcg cgagagggac gcgtacaagg 1860

agaaatacga gaagttggtg agcagcggct tccgagaaaa cggctcgagc agcgacaacc 1920

cgtcctctcc cgagtttttc atgtgagtct gacacgcgat tccagctagc caccctgata 1980

agtgctccgc gggggtccgg ctcgggtgtg ggcttgctag ttctagagcc atgctcgcca 2040

ccacctcacc acccccaccc ccaccgagtt tggccccctt ggccccctac acacacacaa 2100

acccgcacgc acacaccaca cacacacaca cacacacaca cacaccccac accctgctcg 2160

agtttgtggt ggtggtggct gttttaaact ggggagggaa tgggtgtctg gctcatggat 2220

tgccaatctg aaattctcca taacttgcta gcttgttttt tttttttttt tacacccccc 2280

cgccccaccc ccggacttgc acaatgttca atgatctcag cagagttctt catgtgaaac 2340

gttgatcacc tttgaagcct gcatcattca catatttttt cttcttcttc cccttcagtt 2400

catgaactgg tgttcatttt ctgtgtgtgt gtgtgtttta ttttgtttgg attttttttt 2460

ttaattttac ttttagagct tgctgtgttg cccacctttt ttccaacctc caccctcact 2520

ccttctcaac ccatctcttc cgagatgaaa gaaaaaaaaa agcaaagttt ttttttcttc 2580

tcctgagttc ttcatgtgag attgagcttg caaaggaaaa aaaaatgtga aatgttatag 2640

acttgcagcg tgccgagttc catcgggttt tttttttagc attgttatgc taaaatagag 2700

aaaaaaatcc tcatgaacct tccacaatca agcctgcatc aaccttctgg gtgtgacttg 2760

tgagttttgg ccttgtgatg ccaaatctga gagtttagtc tgccattaaa aaaactcatt 2820

ctcatctcat gcattattat gcttgctact ttgtcttagc aacaatgaac tataactgtt 2880

tcaaagactt tatggaaaag agacattata ttaataaaaa aaaaaagcct gcatgctgga 2940

catgtatggt ataattattt tttccttttt ttttcctttt ggcttggaaa tggacgttcg 3000

aagacttata gcatggcatt catacttttg ttttattgcc tcatgacttt tttgagttta 3060

gaacaaaaca gtgcaaccgt agagccttct tcccatgaaa ttttgcatct gctccaaaac 3120

tgctttgagt tactcagaac ttcaacctcc caatgcactg aaggcattcc ttgtcaaaga 3180

taccagaatg ggttacacat ttaacctggc aaacattgaa gaactcttaa tgttttcttt 3240

ttaataagaa tgacgcccca ctttggggac taaaattgtg ctattgccga gaagcagtct 3300

aaaatttatt ttttaaaaag agaaactgcc ccattatttt tggtttgttt tatttttatt 3360

ttatattttt tggcttttgg tcattgtcaa atgtggaatg ctctgggttt ctagtatata 3420

atttaattct agtttttata atctgttagc ccagttaaaa tgtatgctac agataaagga 3480

atgttataga taaatttgaa agagttaggt ctgtttagct gtagattttt taaacgattg 3540

atgcactaaa ttgtttacta ttgtgatgtt aaggggggta gagtttgcaa ggggactgtt 3600

taaaaaaagt agcttataca gcatgtgctt gcaacttaaa tataagttgg gtatgtgtag 3660

tctttgctat accactgact gtattgaaaa ccaaagtatt aagaggggaa acgcccctgt 3720

ttatatctgt aggggtattt tacattcaaa aatgtatgtt tttttttctt ttcaaaatta 3780

aagtatttgg gactgaattg cactaagata taacctgcaa gcatataata caaaaaaaaa 3840

ttgcaaaact gtttagaacg ctaataaaat ttatgcagtt ataaaaatgg cattactgca 3900

cagttttaag atgatgcaga tttttttaca gttgtattgt ggtgcagaac tggattttct 3960

gtaacttaaa aaaaaatcca cagttttaaa ggcaataatc agtaaatgtt attttcaggg 4020

actgacatcc tgtctttaaa aagaaatgaa aagtaaatct taccacaata aatataaaaa 4080

aatcttgtca gttacttttc ttttacatat tttgctgtgc aaaattgttt tatatcttga 4140

gttactaact aaccacgcgt gttgttccta tgtgcttttc tttcattttc aattctggtt 4200

atatcaagaa aagaataatc tacaataata aacggcattt ttttttgatt ctgtactcag 4260

tttcttagtg tacagtttaa ctgggcccaa caacctcgtt aaaagtgtaa aatgcatcct 4320

tttctccagt ggaaggattc ctggaggaat agggagacag taattcaggg tgaaattata 4380

ggctgttttt tgaagtgagg aggctggccc catatactga ttagcaatat ttaatataga 4440

tgtaaattat gacctcattt ttttctcccc aaagttttca gttttcaaat gagttgagcc 4500

ataattgccc ttggtaggaa aaacaaaaca aaacagtgga actaggcttc ctgagcatgg 4560

ccctacactt ctgatcagga gcaaagccat ccatagacag aggagccgga caaatatggc 4620

gcatcagagg tggcttgcgc acatatgcat tgaacggtaa agagaaacag cgcttgcctt 4680

ttcactaaag ttgactattt ttccttcttc tcttacacac cgagattttc ttgttagcaa 4740

ggcctgacaa gatttaacat aaacatgaca aatcatagtt gtttgttttg ttttgctttt 4800

ctctttaaca ctgaagatca tttgtcttaa ataggaaaaa gaaaatccac tccttacttc 4860

catatttcca agtacatatc tggtttaaac tatgttatca aatcatattt caccgtgaat 4920

attcagtgga gaacttctct acctggatga gctagtaatg atttcagatc atgctatccc 4980

cagaaataaa agcaaaaaat aatacctgtg tggaatatag gctgtgcttt gatttactgg 5040

tatttacccc aaaataggct gtgtatgggg gctgacttaa agatcccttg gaaagactca 5100

aaactacctt cactagtagg actcctaagc gctgacctat ttttaaatga cacaaattca 5160

tgaaactaat gttacaaatt catgcagttt gcactcttag tcatcttccc ctagcacacc 5220

aatagaatgt tagacaaagc cagcactgtt ttgaaaatac agccaaacac gatgactttt 5280

gttttgtttt ctgccgttct taaaagaaaa aaagataata ttgcaactct gactgaaaga 5340

cttattttta agaaaacagg ttgtgtttgg tgctgctaag ttctggccag tttatcatct 5400

ggccttcctg cctatttttt acaaaacacg aagacagtgt gtaacctcga cattttgacc 5460

ttcctttatg tgctagttta gacaggctcc tgaatccaca cttaatttgg cttaacaaaa 5520

gtcttaatag taaacctccc ctcatgagct tgaagtcaag tgttcttgac ttcagatatt 5580

tctttccttt tttttttttt ttcctcatca caactaagag atacacaaac tctgaagaag 5640

cagaaatgga gagaatgctt ttaacaaaaa agcatctgat gaaagatttt aggcaaacat 5700

tctcaaaata agagtgatat tctggatgta gttattgcag ttatctcatg acaaatgagg 5760

cctggattgg aaggaaaata tagttgtgta gaattaagca ttttgatagg aatctacaag 5820

gtagttgaat ataataagca ggtttgggcc cccaaacttt agaaaatcaa atgcaaaggt 5880

gctggcaaaa atgaggtttg agtggctggc tgtaagagaa ggttaactcc tagtaaaagg 5940

catttttaga aataacaatt actgaaaact ttgaagtata gtgggagtag caaacaaata 6000

catgtttttt ttttcttaca aagaactcct aaatcctgag taagtgccat tcattacaat 6060

aagtctctaa atttaaaaaa aaaaaaatca tatgaggaaa tctagctttc ccctttacgc 6120

tgcgtttgat ctttgtctaa atagtgttaa aattcctttc attccaatta cagaactgag 6180

cccactcgca agttggagcc atcagtggga tacgccacat tttggaagcc ccagcatcgt 6240

gtacttacca gtgtgttcac aaaatgaaat ttgtgtgaga gctgtacatt aaaaaaaatc 6300

atcattatta ttattatttg cagtcatgga gaaccaccta cccctgactt ctgtttagtc 6360

tcctttttaa ataaaaatta ctgtgttaga gaagaaggct attaaatgta gtagttaact 6420

atgcctcttg tctgggggtt tcatagagac cggtaggaaa gcgcactcct gcttttcgat 6480

ttatggtgtg tgcaagtaaa caggtgcatt gctttcaacc tgccatacta gttttaaaaa 6540

ttcactgaaa ttacaaagat acatatatat gcatatatat aatggaaagt ttcccggaat 6600

gcaacaatta gcattttaaa atcatatata ggcatgcaca ttctaaatag tactttttca 6660

tgcttcattg tttctctggc agataatttt actaagaaga aaaatagata ttcgactccc 6720

cttccctaaa caaatccacg ggcagaggct ccagcggagc cgagccccct ggttttctcg 6780

taggccctag acggtgttgc atttatcagt gatgtcaaac gtgctcattt gtcagacata 6840

gctgtaaatg aaaacaatgt gtggcaaaat acaaagtt 6878

<210>  2
<211>  2656
<212>  DNA
<213>  Homo sapiens

<400>  2
gaggctttaa aatctttttt catcttctag ctgtagctcg ggctgcttgt cggcttggcc      60

tccccctccc ccctttgctc tctgcctcgt ctttccccag gacttcgcta ttttgctttt     120

ttaaaaaaag gcaagaaaga actaaactcc cccctccctc tcctccagtc gggctgcacc     180

tctgccttgc actttgcaca gaggtagaga gcgcgcgagg gagagagagg aaagaaaaaa     240

aataataaag agagccaagc agaagaggag gcgagaagca tgaagtgtta actcccccgt     300

gccaaggccc gcgccgcccg gacagacgcc cgccgcgcct ccagccccga gcggacgccg     360

cgcgcgccct gcctgcagcc cgggccggcg aggcgagccc ttccttatgc aaagcgcgca     420

gcggagcggc gagcggggga cgccgcgcac cgggccgggc tcctccagct tcgccgccgc     480

agccaccacc gccgccaccg cagctcgcgg aggatcttcc cgagcctgaa gccgccggct     540

cggcgcgcaa ggaggcgagc gagcaaggag gggccggggc gagcgaggga gcacattggc     600

gtgagcaggg gggagggagg gcgggcgcgg ggggcgcggg cagggcgggg gggtgtgtgt     660

gtgagcgcgc tcggaggttt cgggccagcc accgccgcgc aagctagaag cgccccagcc     720

cggcaagctg gctcacccgc tggccaccca gcacagcccg ctggcccctc tcctgcagcc     780

catctggcgg agcggcggcg gcggcggcgg cggcggcagg agaatggcat cagaactggc     840

aatgagcaac tccgacctgc ccaccagtcc cctggccatg gaatatgtta atgacttcga     900

tctgatgaag tttgaagtga aaaaggaacc ggtggagacc gaccgcatca tcagccagtg     960

cggccgtctc atcgccgggg gctcgctgtc ctccaccccc atgagcacgc cgtgcagctc    1020

ggtgccccct tcccccagct tctcggcgcc cagcccgggc tcgggcagcg agcagaaggc    1080

gcacctggaa gactactact ggatgaccgg ctacccgcag cagctgaacc ccgaggcgct    1140

gggcttcagc cccgaggacg cggtcgaggc gctcatcagc aacagccacc agctccaggg    1200

cggcttcgat ggctacgcgc gcggggcgca gcagctggcc gcggcggccg gggccggtgc    1260

cggcgcctcc ttgggcggca gcggcgagga gatgggcccc gccgccgccg tggtgtccgc    1320

cgtgatcgcc gcggccgccg cgcagagcgg cgcgggcccg cactaccacc accaccacca    1380

ccacgccgcc ggccaccacc accacccgac ggccggcgcg cccggcgccg cgggcagcgc    1440

ggccgcctcg gccggtggcg ctgggggcgc gggcggcggt ggcccggcca gcgctggggg    1500

cggcggcggc ggcggcggcg gcggaggcgg cgggggcgcg gcgggggcgg ggggcgccct    1560

gcacccgcac cacgccgccg gcggcctgca cttcgacgac cgcttctccg acgagcagct    1620

ggtgaccatg tctgtgcgcg agctgaaccg gcagctgcgc ggggtcagca aggaggaggt    1680

gatccggctg aagcagaaga ggcggaccct gaaaaaccgc ggctatgccc agtcctgccg 1740

cttcaagagg gtgcagcaga gacacgtcct ggagtcggag aagaaccagc tgctgcagca 1800

agtcgaccac ctcaagcagg agatctccag gctggtgcgc gagagggacg cgtacaagga 1860

gaaatacgag aagttggtga gcagcggctt ccgagaaaac ggctcgagca gcgacaaccc 1920

gtcctctccc gagtttttca taactgagcc cactcgcaag ttggagccat cagtgggata 1980

cgccacattt tggaagcccc agcatcgtgt acttaccagt gtgttcacaa aatgaaattt 2040

gtgtgagagc tgtacattaa aaaaaatcat cattattatt attatttgca gtcatggaga 2100

accacctacc cctgacttct gtttagtctc ctttttaaat aaaaattact gtgttagaga 2160

agaaggctat taaatgtagt agttaactat gcctcttgtc tgggggtttc atagagaccg 2220

gtaggaaagc gcactcctgc ttttcgattt atggtgtgtg caagtaaaca ggtgcattgc 2280

tttcaacctg ccatactagt tttaaaaatt cactgaaatt acaaagatac atatatatgc 2340

atatatataa tggaaagttt cccggaatgc aacaattagc attttaaaat catatatagg 2400

catgcacatt ctaaatagta ctttttcatg cttcattgtt tctctggcag ataattttac 2460

taagaagaaa aatagatatt cgactcccct tccctaaaca aatccacggg cagaggctcc 2520

agcggagccg agccccctgg ttttctcgta ggccctagac ggtgttgcat ttatcagtga 2580

tgtcaaacgt gctcatttgt cagacatagc tgtaaatgaa aacaatgtgt ggcaaaatac 2640

aaagttaaaa aaaaaa 2656

<210> 3
<211> 6887
<212> DNA
<213> Homo sapiens

<400> 3
gaggctttaa aatctttttt catcttctag ctgtagctcg ggctgcttgt cggcttggcc 60

tccccctccc ccctttgctc tctgcctcgt ctttccccag gacttcgcta ttttgctttt 120

ttaaaaaaag gcaagaaaga actaaactcc cccctccctc tcctccagtc gggctgcacc 180

tctgccttgc actttgcaca gaggtagaga gcgcgcgagg gagagagagg aaagaaaaaa 240

aataataaag agagccaagc agaagaggag gcgagaagca tgaagtgtta actcccccgt 300

gccaaggccc gcgccgcccg gacagacgcc cgccgcgcct ccagccccga gcggacgccg 360

cgcgcgccct gcctgcagcc cgggccggcg aggcgagccc ttccttatgc aaagcgcgca 420

gcggagcggc gagcggggga cgccgcgcac cgggccgggc tcctccagct tcgccgccgc 480

agccaccacc gccgccaccg cagctcgcgg aggatcttcc cgagcctgaa gccgccggct 540

cggcgcgcaa ggaggcgagc gagcaaggag gggccggggc gagcgaggga gcacattggc 600

gtgagcaggg gggagggagg gcgggcgcgg ggggcgcggg cagggcgggg gggtgtgtgt 660

gtgagcgcgc tcggaggttt cgggccagcc accgccgcgc aagctagaag cgccccagcc 720

cggcaagctg gctcacccgc tggccaccca gcacagcccg ctggcccctc tcctgcagcc 780

catctggcgg agcggcggcg gcggcggcgg cggcggcagg agaatggcat cagaactggc 840

aatgagcaac tccgacctgc ccaccagtcc cctggccatg gaatatgtta atgacttcga 900

tctgatgaag tttgaagtga aaaaggaacc ggtggagacc gaccgcatca tcagccagtg 960

cggccgtctc atcgccgggg gctcgctgtc ctccaccccc atgagcacgc cgtgcagctc 1020

ggtgccccct tcccccagct tctcggcgcc cagcccgggc tcgggcagcg agcagaaggc 1080

gcacctggaa gactactact ggatgaccgg ctacccgcag cagctgaacc ccgaggcgct 1140

gggcttcagc cccgaggacg cggtcgaggc gctcatcagc aacagccacc agctccaggg 1200

cggcttcgat ggctacgcgc gcggggcgca gcagctggcc gcggcggccg gggccggtgc 1260

cggcgcctcc ttgggcggca gcggcgagga gatgggcccc gccgccgccg tggtgtccgc 1320

cgtgatcgcc gcggccgccg cgcagagcgg cgcgggcccg cactaccacc accaccacca 1380

ccacgccgcc ggccaccacc accacccgac ggccggcgcg cccggcgccg cgggcagcgc 1440

ggccgcctcg gccggtggcg ctgggggcgc gggcggcggt ggcccggcca gcgctggggg 1500

cggcggcggc ggcggcggcg gcggaggcgg cgggggcgcg gcgggggcgg ggggcgccct 1560

gcacccgcac cacgccgccg gcggcctgca cttcgacgac cgcttctccg acgagcagct 1620

ggtgaccatg tctgtgcgcg agctgaaccg gcagctgcgc ggggtcagca aggaggaggt 1680

gatccggctg aagcagaaga ggcggaccct gaaaaaccgc ggctatgccc agtcctgccg 1740

cttcaagagg gtgcagcaga gacacgtcct ggagtcggag aagaaccagc tgctgcagca 1800

agtcgaccac ctcaagcagg agatctccag gctggtgcgc gagagggacg cgtacaagga 1860

gaaatacgag aagttggtga gcagcggctt ccgagaaaac ggctcgagca gcgacaaccc 1920

gtcctctccc gagtttttca tgtgagtctg acacgcgatt ccagctagcc accctgataa 1980

gtgctccgcg ggggtccggc tcgggtgtgg gcttgctagt tctagagcca tgctcgccac 2040

cacctcacca cccccacccc caccgagttt ggcccccttg gccccctaca cacacacaaa 2100

cccgcacgca cacaccacac acacacacac acacacacac acaccccaca ccctgctcga 2160

gtttgtggtg gtggtggctg ttttaaactg gggagggaat gggtgtctgg ctcatggatt 2220

gccaatctga aattctccat aacttgctag cttgtttttt tttttttttt acaccccccc 2280

gccccacccc cggacttgca caatgttcaa tgatctcagc agagttcttc atgtgaaacg 2340

ttgatcacct ttgaagcctg catcattcac atattttttc ttcttcttcc ccttcagttc 2400

atgaactggt gttcattttc tgtgtgtgtg tgtgttttat tttgtttgga tttttttttt 2460

taattttact tttagagctt gctgtgttgc ccaccttttt tccaacctcc accctcactc 2520

cttctcaacc catctcttcc gagatgaaag aaaaaaaaaa gcaaagtttt tttttcttct 2580

cctgagttct tcatgtgaga ttgagcttgc aaaggaaaaa aaaatgtgaa atgttataga 2640

cttgcagcgt gccgagttcc atcgggtttt ttttttagca ttgttatgct aaaatagaga 2700

aaaaaatcct catgaacctt ccacaatcaa gcctgcatca accttctggg tgtgacttgt 2760

gagttttggc cttgtgatgc caaatctgag agtttagtct gccattaaaa aaactcattc 2820

tcatctcatg cattattatg cttgctactt tgtcttagca acaatgaact ataactgttt 2880

caaagacttt atggaaaaga gacattatat taataaaaaa aaaaagcctg catgctggac 2940

atgtatggta taattatttt ttcctttttt tttccttttg gcttggaaat ggacgttcga 3000

agacttatag catggcattc atacttttgt tttattgcct catgactttt ttgagtttag 3060

aacaaaacag tgcaaccgta gagccttctt cccatgaaat tttgcatctg ctccaaaact 3120

gctttgagtt actcagaact tcaacctccc aatgcactga aggcattcct tgtcaaagat 3180

accagaatgg gttacacatt taacctggca aacattgaag aactcttaat gtttcttttt 3240

taataagaat gacgccccac tttggggact aaaattgtgc tattgccgag aagcagtcta 3300

aaatttattt tttaaaaaga gaaactgccc cattattttt ggtttgtttt atttttattt 3360

tatatttttt ggcttttggt cattgtcaaa tgtggaatgc tctgggtttc tagtatataa 3420

tttaattcta gtttttataa tctgttagcc cagttaaaat gtatgctaca gataaaggaa 3480

tgttatagat aaatttgaaa gagttaggtc tgtttagctg tagatttttt aaacgattga 3540

tgcactaaat tgtttactat tgtgatgtta aggggggtag agtttgcaag gggactgttt 3600

aaaaaaagta gcttatacag catgtgcttg caacttaaat ataagttggg tatgtgtagt 3660

ctttgctata ccactgactg tattgaaaac caaagtatta agaggggaaa cgcccctgtt 3720

tatatctgta ggggtatttt acattcaaaa atgtatgttt ttttttcttt tcaaaattaa 3780

agtatttggg actgaattgc actaagatat aacctgcaag catataatac aaaaaaaaat 3840

tgcaaaactg tttagaacgc taataaaatt tatgcagtta taaaaatggc attactgcac 3900

agttttaaga tgatgcagat ttttttacag ttgtattgtg gtgcagaact ggattttctg 3960

taacttaaaa aaaaatccac agttttaaag gcaataatca gtaaatgtta ttttcaggga 4020

ctgacatcct gtctttaaaa agaaatgaaa agtaaatctt accacaataa atataaaaaa 4080

atcttgtcag ttacttttct tttacatatt ttgctgtgca aaattgtttt atatcttgag 4140

ttactaacta accacgcgtg ttgttcctat gtgcttttct ttcattttca attctggtta 4200

tatcaagaaa agaataatct acaataataa acggcatttt tttttgattc tgtactcagt 4260

ttcttagtgt acagtttaac tgggcccaac aacctcgtta aaagtgtaaa atgcatcctt 4320

ttctccagtg gaaggattcc tggaggaata gggagacagt aattcagggt gaaattatag 4380

gctgtttttt gaagtgagga ggctggcccc atatactgat tagcaatatt taatatagat 4440

gtaaattatg acctcatttt tttctcccca aagttttcag ttttcaaatg agttgagcca 4500

taattgccct tggtaggaaa aacaaaacaa aacagtggaa ctaggcttcc tgagcatggc 4560

cctacacttc tgatcaggag caaagccatc catagacaga ggagccggac aaatatggcg 4620

catcagaggt ggcttgcgca catatgcatt gaacggtaaa gagaaacagc gcttgccttt 4680

tcactaaagt tgactatttt tccttcttct cttacacacc gagattttct tgttagcaag 4740

gcctgacaag atttaacata aacatgacaa atcatagttg tttgttttgt tttgcttttc 4800

tctttaacac tgaagatcat ttgtcttaaa taggaaaaag aaaatccact ccttacttcc 4860

atatttccaa gtacatatct ggtttaaact atgttatcaa atcatatttc accgtgaata 4920

ttcagtggag aacttctcta cctggatgag ctagtaatga tttcagatca tgctatcccc 4980

agaaataaaa gcaaaaaata atacctgtgt ggaatatagg ctgtgctttg atttactggt 5040

atttacccca aaataggctg tgtatggggg ctgacttaaa gatcccttgg aaagactcaa 5100

aactaccttc actagtagga ctcctaagcg ctgacctatt tttaaatgac acaaattcat 5160

gaaactaatg ttacaaattc atgcagtttg cactcttagt catcttcccc tagcacacca 5220

atagaatgtt agacaaagcc agcactgttt tgaaaataca gccaaacacg atgacttttg 5280

ttttgttttc tgccgttctt aaaagaaaaa aagataatat tgcaactctg actgaaagac 5340

ttatttttaa gaaaacaggt tgtgtttggt gctgctaagt tctggccagt ttatcatctg 5400

gccttcctgc ctatttttta caaaacacga agacagtgtg taacctcgac attttgacct 5460

tcctttatgt gctagtttag acaggctcct gaatccacac ttaattttgc ttaacaaaag 5520

tcttaatagt aaacctcccc tcatgagctt gaagtcaagt gttcttgact tcagatattt 5580

ctttcctttt tttttttttt tcctcatcac aactaagaga tacacaaact ctgaagaagc 5640

agaaatggag agaatgcttt taacaaaaaa gcatctgatg aaagatttta ggcaaacatt 5700

ctcaaaataa gagtgatatt ctggatgtag ttattgcagt tatctcatga caaatgaggc 5760

ctggattgga aggaaaatat agttgtgtag aattaagcat tttgatagga atctacaagg 5820

tagttgaata taataagcag gtttgggccc ccaaacttta gaaaatcaaa tgcaaaggtg 5880

ctggcaaaaa tgaggtttga gtggctggct gtaagagaag gttaactcct agtaaaaggc 5940

atttttagaa ataacaatta ctgaaaactt tgaagtatag tgggagtagc aaacaaatac 6000

atgttttttt tttcttacaa agaactccta aatcctgagt aagtgccatt cattacaata 6060

agtctctaaa tttaaaaaaa aaaaaatcat atgaggaaat ctagctttcc cctttacgct 6120

gcgtttgatc tttgtctaaa tagtgttaaa attcctttca ttccaattac agaactgagc 6180

ccactcgcaa gttggagcca tcagtgggat acgccacatt ttggaagccc cagcatcgtg 6240

tacttaccag tgtgttcaca aaatgaaatt tgtgtgagag ctgtacatta aaaaaaatca 6300

tcattattat tattatttgc agtcatggag aaccacctac ccctgacttc tgtttagtct 6360

cctttttaaa taaaaattac tgtgttagag aagaaggcta ttaaatgtag tagttaacta 6420

tgcctcttgt ctgggggttt catagagacc ggtaggaaag cgcactcctg cttttcgatt 6480

tatggtgtgt gcaagtaaac aggtgcattg ctttcaacct gccatactag ttttaaaaat 6540

tcactgaaat tacaaagata catatatatg catatatata atggaaagtt tcccggaatg 6600

caacaattag cattttaaaa tcatatatag gcatgcacat tctaaatagt actttttcat 6660

gcttcattgt ttctctggca gataatttta ctaagaagaa aaatagatat tcgactcccc 6720

ttccctaaac aaatccacgg gcagaggctc cagcggagcc gagccccctg gttttctcgt 6780

aggccctaga cggtgttgca tttatcagtg atgtcaaacg tgctcatttg tcagacatag 6840

ctgtaaatga aaacaatgtg tggcaaaata caaagttaaa aaaaaaa 6887

<210> 4
<211> 403
<212> PRT
<213> Homo sapiens

<400> 4

<210> 5
<211> 373
<212> PRT
<213> Homo sapiens

<400> 5

Met Ala Ser Glu Leu Ala Met Ser Asn Ser Asp Leu Pro Thr Ser Pro
1               5                   10                  15

Leu Ala Met Glu Tyr Val Asn Asp Phe Asp Leu Met Lys Phe Glu Val
            20                  25                  30

Lys Lys Glu Pro Val Glu Thr Asp Arg Ile Ile Ser Gln Cys Gly Arg
        35                  40                  45

Leu Ile Ala Gly Gly Ser Leu Ser Ser Thr Pro Met Ser Thr Pro Cys
    50                  55                  60

Ser Ser Val Pro Pro Ser Pro Ser Phe Ser Ala Pro Ser Pro Gly Ser
65                  70                  75                  80

Gly Ser Glu Gln Lys Ala His Leu Glu Asp Tyr Tyr Trp Met Thr Gly
                85                  90                  95

Tyr Pro Gln Gln Leu Asn Pro Glu Ala Leu Gly Phe Ser Pro Glu Asp
            100                 105                 110

Ala Val Glu Ala Leu Ile Ser Asn Ser His Gln Leu Gln Gly Gly Phe
        115                 120                 125

Asp Gly Tyr Ala Arg Gly Ala Gln Gln Leu Ala Ala Ala Ala Gly Ala
    130                 135                 140

Gly Ala Gly Ala Ser Leu Gly Gly Ser Gly Glu Glu Met Gly Pro Ala
145                 150                 155                 160

Ala Ala Val Val Ser Ala Val Ile Ala Ala Ala Ala Ala Gln Ser Gly
                165                 170                 175

Ala Gly Pro His Tyr His His His His His His Ala Ala Gly His His
            180                 185                 190

His His Pro Thr Ala Gly Ala Pro Gly Ala Ala Gly Ser Ala Ala Ala
        195                 200                 205

Ser Ala Gly Gly Ala Gly Gly Ala Gly Gly Gly Gly Pro Ala Ser Ala
    210                 215                 220

Gly Gly Gly Gly Gly Gly Gly Gly Gly Gly Gly Gly Gly Gly Ala Ala
225                 230                 235                 240

Gly Ala Gly Gly Ala Leu His Pro His His Ala Ala Gly Gly Leu His
                245                 250                 255

Phe Asp Asp Arg Phe Ser Asp Glu Gln Leu Val Thr Met Ser Val Arg
            260                 265                 270

Glu Leu Asn Arg Gln Leu Arg Gly Val Ser Lys Glu Glu Val Ile Arg
        275                 280                 285

Leu Lys Gln Lys Arg Arg Thr Leu Lys Asn Arg Gly Tyr Ala Gln Ser
    290                 295                 300

Cys Arg Phe Lys Arg Val Gln Gln Arg His Val Leu Glu Ser Glu Lys
305                 310                 315                 320

Asn Gln Leu Leu Gln Gln Val Asp His Leu Lys Gln Glu Ile Ser Arg
                325                 330                 335

Leu Val Arg Glu Arg Asp Ala Tyr Lys Glu Lys Tyr Glu Lys Leu Val
            340                 345                 350

Ser Ser Gly Phe Arg Glu Asn Gly Ser Ser Ser Asp Asn Pro Ser Ser
        355                 360                 365

Pro Glu Phe Phe Met
    370

<210>  6
<211>  19
<212>  RNA
<213>  Artificial Sequence

<220>
<223>  c-MAF specific siRNA

<400>  6
acggcucgag cagcgacaa                                                    19

<210>  7
<211>  19
<212>  RNA
<213>  Artificial Sequence

<220>
<223>  c-MAF specific siRNA

<400>  7
cuuaccagug uguucacaa                                                    19


<210>  8
<211>  20
<212>  RNA
<213>  Artificial Sequence

<220>
<223>  c-MAF specific siRNA

<400>  8
uggaagacua cuacuggaug                                                   20


<210>  9
<211>  20
<212>  RNA
<213>  Artificial Sequence

<220>
<223>  c-MAF specific siRNA

<400>  9
auuugcaguc auggagaacc                                                   20


<210>  10
<211>  20
<212>  RNA
<213>  Artificial Sequence

<220>
<223>  c-MAF specific siRNA

<400>  10
caaggagaaa uacgagaagu                                                   20


<210>  11
<211>  20
<212>  RNA
<213>  Artificial Sequence

<220>
<223>  c-MAF specific siRNA

<400>  11
acaaggagaa auacgagaag                                                   20


<210>  12
<211>  20
<212>  RNA
<213>  Artificial Sequence

<220>
<223>  c-MAF specific siRNA

<400>  12
accuggaaga cuacuacugg                                                    20


<210>  13
<211>  13878
<212>  DNA
<213>  Homo sapiens

<400>  13
aactatatat taaacacctc cggtctgaga ggccgtgttg ggtgtctttg tcaggtgaag        60

aaagagaaga aggctggtac accttcccag gaattctcac tgaagaaaac atctggattt       120

tttacatctc ttgtgcaaaa caaacaaaga tttcattaag tgatgtatat tgttttccaa       180

ggaagaaacc tgcagagaca aaaacaaata agcaaataat tgaaacaaaa atatgataaa       240

cccccaaatt cttccagtgc taatttactt gttatcatgg ttctctacaa aggcagagat       300

cactaattac aggtttttcc agaattcaca tttcacgtca agatcatcca atccaaacag       360

tgtacggaaa gcctagggcc ttcttcactt tgccccctac cccaccctac acacacgccc       420

ccatctaaat gatacccttg gaaagaaacc tacacatctc atttgtctat attttgcttc       480

ctccctcgcc tcccggtaac caaatgtgag ttgttctcta actgcactgg agaatcagaa       540

tttattgtac atatgtttgt gttccactta ataaaaaaac ctatatttta agataaactt       600

tgttagtaat tcatgaggta agtgactatt tatgctaatc aggcagaaat atattctcaa       660

gcataatgca ttacataaat ttgaatgtaa aatgttcaat tatgaagtaa atacaggtaa       720

tgcaaataat aaattacctc taataaaaat tataaaagat gtgccttgaa agagagagcg       780

gctttaactt acaactgtga attgcttaaa gagaaaagaa ttaataaatg ctgaattact       840

ctgatgatta tttagcacat aattcaccta ttcataacga ctcctagtaa tcagactgtt       900

gtttcacatc ctccaacatg aggcaagact gtttcctcag caattttgcc cttatcagat       960

tatctcgtct gattctatta attttcttcc atgaatctgc taacagtgat ttgtgattta      1020

cttaccctgc taactgaaga ctgttaaaag gatttatcta acactggacc taagaacagt      1080

gtacgcctta tcgttcagtt actctgaaga actctttctc aaatcaattt agttggtttc      1140

atagtgaaat ttagtggaca ctggttagtt ctgccccata aaatcagccc ctaaacaaag      1200

agtccagaca ccatacctga tgcatcccat tctattcaga ttatggatgt ctgattccaa      1260

catgatatat ttgagttgct ataactcaca atcggggaaa atatattcct ttaagctttt      1320

aatctttgta atttggacat gaacaggggt tttgtttttc atttttgcat gaagtcatta      1380

tgtatgtact gacgtgaaac tataattgtg tttctgatgt tactgtgtca caatattcta      1440

tgcgatgtaa cccatgtcct cctccccctc acaaatctcc tataaatatt cattgctttc      1500

aaaaacttta atactactgg tccgaattgg tcaataatga caaatgcatg gtttctaaat      1560

tactgtatat tgttctacag agattactag agtatatata gcaaggggat gttaagcagt      1620

aagaaaacac agttcacatt gtatttggat tagattggct tggatagaag tgaaacaaac 1680

aatgttagca aagaagtcta aagacatgtg gcccactgta attgtacaga atcaaaaacc 1740

tgaatagtac tcattaaaat gagagagctc aattgttata aaagaaatgc tgctaacaga 1800

gaactgtaaa tgtttagaca cccctgtgaa tcactaaata ataatgtaaa aaggataaaa 1860

atgagaatta agttataagc ctgagagcat tactgctaca catctaaaaa aataattctg 1920

atcctctctt ttttttttcc aagagaaaat gggcgactat aaaagacctt gcaataagag 1980

aaataaaaat accatgtctt cacagcagtg tacataaata aaccataaaa atgtgcagat 2040

aataatatat ttagctgccc aaacatgggc atttaatttc tagaaatgat atataacaat 2100

gtaacaatta gatactcagc catgaatgtg tatggcacag tcttcatcat tagcaaactt 2160

tgtgtataaa atattattta ttatttatta taatactgct ttcagaggca atgatcatac 2220

cttacagctt ttaacacaaa tatgatgcaa aaggattaaa agtatatcat aaacaaacaa 2280

taaattcttt ctaaatacac ttaaattcat attttacatg aaaaatataa acttcctaca 2340

tttgtgacta ctgactttta aaaagaccta gaaaactatt gttacgggca atgttaaatg 2400

acataatgct tatgtaatgg aaagtgtgga ttttcctcta aataaactat aatcccttaa 2460

cttcattact agggaaaata ttgttaaaga gaaggaaagc aagggaattc tgctaggttg 2520

cataaatatt gacataatct tcactctttc ttccccaaac tggtaataga catagtttat 2580

tccacccaac aaaatgctct tataagacca aaactaccct tattaacaac ttctctgcag 2640

tcacgatgaa aagaaacact acttgtctga aaaataccga cagcgctgcc cttttcagat 2700

tagggtgtgc ctacgaatct tttgggaagt cttccattaa ggattcctgg gtttgctgaa 2760

actgaagtct actaggatca gagaaattaa cacaggtcta atatggtgca aggaacgagt 2820

gagagacacc tgaggttata aatagcaaag catgctgcgg ggtggggaag accattctga 2880

agtgcaatgt tcaagacgct ggcttaatat atgactaagt gtcagaagtc aggttttctg 2940

agaattactt tccagataaa caactttata gcactgcact taatcttact tactagagac 3000

atctcattta tcactgaatt acaagtaact ttaatcctat tgatattgcc ataaagcccg 3060

ttgaaaatcc atcctggcac ttttaaaggg tttggggccc tgttacatgg ggatcctctt 3120

gcaaaggtct cagccagaaa ttacaccccg agggtgtctg tatcccctgg cctctttgtc 3180

aacaatcaag gagaagagga ggggcaaaaa tgatctctgc atctgccagc actttcttcg 3240

gcccctttcc tatagggtcg ggttctccca cttcagtcaa actaactttg tgtgtctctt 3300

tcctcctccc acactgggta accagctgct tttcacttca tcgacaaaac tggacacgga 3360

tcaatttcaa ctgacctttg ccgaaaggtg gcgctgttga ggtaaaaacc aactcgctcc 3420

aacaatagtt tccactcttc gatccttttg caggcttttc agaatttttt ttttttttta 3480

atgcaccctc ctagcgtctc ccccttctca taaagtaaaa taaatacgat taaaaacacc 3540

aaatgcattt cattaattga aggaatcaac agtcccaact tctaagcaga cgggctggtc 3600

ttccaaaggc tgggtcggtt tcaggagctt tctctccaaa taaatctctg cttcttcgac 3660

ttgcctatcg ctttaaaatc ttagaaacag agttagttgt tggtttcctt ctttttcttt 3720

tttctttttt atttcttttt tgcataaact tttagagaat caatctagaa atttgaacta 3780

cttattagca tttgcaactg ggggtggggg gagcagcctc ccccacccca ccccccactc 3840

tgcgtttccg gactagttcc agaaaccgcg gtttaaaatt taacccttcg agggtagctg 3900

gtgagggctg gggtattgtt tttccccctt gctccctgcc acgatcaagt ccgaaataat 3960

taaaggaaac gtaaaagtgc aaagggcgcg cctgaccctg ataaacagag gtcagatttc 4020

gtaaggggac gggtgagtgt gagtgtgtgt gtgtttgtgt gtgtgtgtgt aagagagaga 4080

gagagcgagc gcgcaatatg agtctcaaag gccaaactcc ggccagtcag gagccggaag 4140

gctgagcccg gctgacctga ctttgagctt ccccggagtt atctcgcata ggcgctcgct 4200

ctgtccaagg gcacgcgacg ccagcgggca gccggtctcc gtgaagaatg gcctctaaac 4260

aacttatttt acctcgttgt aaagagaggg ataaaatggg ctttccctct ccacggatgc 4320

ccagccttct gggcaggcgc atggccgggc ggcgcccagc ccgcagcccc gatccggaca 4380

ccccactgca tccctccctt cccggtccct tccccgcacg ggcgcccgag agacggacaa 4440

agagttgggg ccaagtttga gcgccgggca cggccaggct cagggaagga aggtccccgg 4500

cagacacctg ggtaccagag ttggtgcgag gaggaaaagc tgggaggcga attcacaatc 4560

ctgggggtgg agggcaggca ggggagggga atcaggccaa tcccagccga gtgagccccc 4620

agcgagctgg ggctccggat gggaggcctg tctcgcgctc caaagaaaag caaaccgccc 4680

tcccaggtcc gcccggattg ccgaagcccc tctggaaaaa ctccttcccc tcttacacca 4740

aactttgcgc cgggcctcgt tccctcccgg gtaggcagcg gcgcaggaag ggttaagcca 4800

gcccgtccca gctgacagtc agctgattgg gccctgattg acagctccga aaagtttcct 4860

tgtttctata ctattatgct aatcgcggcc gctctcgccg cctcccattg gcccggagtg 4920

ccagtcaatt tctcatttgg acctgacgtc acgagtgcta taaaactcag caattgcttt 4980

aaactcttct tgctggatca gaggctttaa aatctttttt catcttctag ctgtagctcg 5040

ggctgcttgt cggcttggcc tccccctccc ccctttgctc tctgcctcgt ctttccccag 5100

gacttcgcta ttttgctttt ttaaaaaaag gcaagaaaga actaaactcc cccctccctc 5160

tcctccagtc gggctgcacc tctgccttgc actttgcaca gaggtagaga gcgcgcgagg 5220

gagagagagg aaagaaaaaa aataataaag agagccaagc agaagaggag gcgagaagca 5280

tgaagtgtta actcccccgt gccaaggccc gcgccgcccg gacagacgcc cgccgcgcct 5340

ccagccccga gcggacgccg cgcgcgccct gcctgcagcc cgggccggcg aggcgagccc 5400

ttccttatgc aaagcgcgca gcggagcggc gagcggggga cgccgcgcac cgggccgggc 5460

tcctccagct tcgccgccgc agccaccacc gccgccaccg cagctcgcgg aggatcttcc 5520

cgagcctgaa gccgccggct cggcgcgcaa ggaggcgagc gagcaaggag gggccggggc 5580

gagcgaggga gcacattggc gtgagcaggg gggagggagg gcgggcgcgg ggggcgcggg 5640

cagggcgggg gggtgtgtgt gtgagcgcgc tcggaggttt cgggccagcc accgccgcgc 5700

aagctagaag cgccccagcc cggcaagctg gctcacccgc tggccaccca gcacagcccg 5760

ctggcccctc tcctgcagcc catctggcgg agcggcggcg gcggcggcgg cggcggcagg 5820

agaatggcat cagaactggc aatgagcaac tccgacctgc ccaccagtcc cctggccatg 5880

gaatatgtta atgacttcga tctgatgaag tttgaagtga aaaaggaacc ggtggagacc 5940

gaccgcatca tcagccagtg cggccgtctc atcgccgggg gctcgctgtc ctccaccccc 6000

atgagcacgc cgtgcagctc ggtgccccct tcccccagct tctcggcgcc cagcccgggc 6060

tcgggcagcg agcagaaggc gcacctggaa gactactact ggatgaccgg ctacccgcag 6120

cagctgaacc ccgaggcgct gggcttcagc cccgaggacg cggtcgaggc gctcatcagc 6180

aacagccacc agctccaggg cggcttcgat ggctacgcgc gcggggcgca gcagctggcc 6240

gcggcggccg gggccggtgc cggcgcctcc ttgggcggca gcggcgagga gatgggcccc 6300

gccgccgccg tggtgtccgc cgtgatcgcc gcggccgccg cgcagagcgg cgcgggcccg 6360

cactaccacc accaccacca ccacgccgcc ggccaccacc accacccgac ggccggcgcg 6420

cccggcgccg cgggcagcgc ggccgcctcg gccggtggcg ctgggggcgc gggcggcggt 6480

ggcccggcca gcgctggggg cggcggcggc ggcggcggcg gcggaggcgg cgggggcgcg 6540

gcgggggcgg ggggcgccct gcacccgcac cacgccgccg gcggcctgca cttcgacgac 6600

cgcttctccg acgagcagct ggtgaccatg tctgtgcgcg agctgaaccg gcagctgcgc 6660

ggggtcagca aggaggaggt gatccggctg aagcagaaga ggcggaccct gaaaaaccgc 6720

ggctatgccc agtcctgccg cttcaagagg gtgcagcaga gacacgtcct ggagtcggag 6780

aagaaccagc tgctgcagca agtcgaccac ctcaagcagg agatctccag gctggtgcgc 6840

gagagggacg cgtacaagga gaaatacgag aagttggtga gcagcggctt ccgagaaaac 6900

ggctcgagca gcgacaaccc gtcctctccc gagtttttca tgtgagtctg acacgcgatt 6960

ccagctagcc accctgataa gtgctccgcg ggggtccggc tcgggtgtgg gcttgctagt 7020

tctagagcca tgctcgccac cacctcacca cccccacccc caccgagttt ggcccccttg 7080

gccccctaca cacacacaaa cccgcacgca cacaccacac acacacacac acacacacac 7140

acaccccaca ccctgctcga gtttgtggtg gtggtggctg ttttaaactg gggagggaat 7200

gggtgtctgg ctcatggatt gccaatctga aattctccat aacttgctag cttgtttttt 7260

tttttttttt acaccccccc gccccacccc cggacttgca caatgttcaa tgatctcagc 7320

agagttcttc atgtgaaacg ttgatcacct ttgaagcctg catcattcac atattttttc 7380

ttcttcttcc ccttcagttc atgaactggt gttcattttc tgtgtgtgtg tgtgttttat 7440

tttgtttgga tttttttttt taattttact tttagagctt gctgtgttgc ccaccttttt 7500

tccaacctcc accctcactc cttctcaacc catctcttcc gagatgaaag aaaaaaaaaa 7560

gcaaagtttt tttttcttct cctgagttct tcatgtgaga ttgagcttgc aaaggaaaaa 7620

aaaatgtgaa atgttataga cttgcagcgt gccgagttcc atcgggtttt ttttttagca 7680

ttgttatgct aaaatagaga aaaaaatcct catgaacctt ccacaatcaa gcctgcatca 7740

accttctggg tgtgacttgt gagttttggc cttgtgatgc caaatctgag agtttagtct 7800

gccattaaaa aaactcattc tcatctcatg cattattatg cttgctactt tgtcttagca 7860

acaatgaact ataactgttt caaagacttt atggaaaaga gacattatat taataaaaaa 7920

aaaaagcctg catgctggac atgtatggta taattatttt ttcctttttt tttccttttg 7980

gcttggaaat ggacgttcga agacttatag catggcattc atacttttgt tttattgcct 8040

catgactttt ttgagtttag aacaaaacag tgcaaccgta gagccttctt cccatgaaat 8100

tttgcatctg ctccaaaact gctttgagtt actcagaact tcaacctccc aatgcactga 8160

aggcattcct tgtcaaagat accagaatgg gttacacatt taacctggca aacattgaag 8220

aactcttaat gttttctttt taataagaat gacgccccac tttggggact aaaattgtgc 8280

tattgccgag aagcagtcta aaatttattt tttaaaaaga gaaactgccc cattattttt 8340

ggtttgtttt atttttattt tatatttttt ggcttttggt cattgtcaaa tgtggaatgc 8400

tctgggtttc tagtatataa tttaattcta gtttttataa tctgttagcc cagttaaaat 8460

gtatgctaca gataaaggaa tgttatagat aaatttgaaa gagttaggtc tgtttagctg 8520

tagatttttt aaacgattga tgcactaaat tgtttactat tgtgatgtta aggggggtag 8580

agtttgcaag ggactgtttt aaaaaaagta gcttatacag catgtgcttg caacttaaat 8640

ataagttggg tatgtgtagt ctttgctata ccactgactg tattgaaaac caaagtatta 8700

agaggggaaa cgcccctgtt tatatctgta ggggtatttt acattcaaaa atgtatgttt 8760

ttttttcttt tcaaaattaa agtatttggg actgaattgc actaagatat aacctgcaag 8820

catataatac aaaaaaaaat tgcaaaactg tttagaacgc taataaaatt tatgcagtta 8880

taaaaatggc attactgcac agttttaaga tgatgcagat ttttttacag ttgtattgtg 8940

gtgcagaact ggattttctg taacttaaaa aaaaatccac agttttaaag gcaataatca 9000

gtaaatgtta ttttcaggga ctgacatcct gtctttaaaa agaaatgaaa agtaaatctt 9060

accacaataa atataaaaaa atcttgtcag ttacttttct tttacatatt ttgctgtgca 9120

aaattgtttt atatcttgag ttactaacta accacgcgtg ttgttcctat gtgcttttct 9180

ttcattttca attctggtta tatcaagaaa agaataatct acaataataa acggcatttt 9240

tttttgattc tgtactcagt ttcttagtgt acagtttaac tgggcccaac aacctcgtta 9300

aaagtgtaaa atgcatcctt ttctccagtg gaaggattcc tggaggaata gggagacagt 9360

aattcagggt gaaattatag gctgtttttt gaagtgagga ggctggcccc atatactgat 9420

tagcaatatt taatatagat gtaaattatg acctcatttt tttctcccca aagttttcag 9480

ttttcaaatg agttgagcca taattgccct tggtaggaaa aacaaaacaa aacagtggaa 9540

ctaggcttcc tgagcatggc cctacacttc tgatcaggag caaagccatc catagacaga 9600

ggagccggac aaatatggcg catcagaggt ggcttgcgca catatgcatt gaacggtaaa 9660

gagaaacagc gcttgccttt tcactaaagt tgactatttt tccttcttct cttacacacc 9720

gagattttct tgttagcaag gcctgacaag atttaacata aacatgacaa atcatagttg 9780

tttgttttgt tttgcttttc tctttaacac tgaagatcat ttgtcttaaa taggaaaaag 9840

aaaatccact ccttacttcc atatttccaa gtacatatct ggtttaaact atgttatcaa 9900

atcatatttc accgtgaata ttcagtggag aacttctcta cctggatgag ctagtaatga 9960

tttcagatca tgctatcccc agaaataaaa gcaaaaaata atacctgtgt ggaatatagg 10020

ctgtgctttg atttactggt atttacccca aaataggctg tgtatggggg ctgacttaaa 10080

gatcccttgg aaagactcaa aactaccttc actagtagga ctcctaagcg ctgacctatt 10140

tttaaatgac acaaattcat gaaactaatg ttacaaattc atgcagtttg cactcttagt 10200

catcttcccc tagcacacca atagaatgtt agacaaagcc agcactgttt tgaaaataca 10260

gccaaacacg atgacttttg ttttgttttc tgccgttctt aaaagaaaaa aagataatat 10320

tgcaactctg actgaaagac ttatttttaa gaaaacaggt tgtgtttggt gctgctaagt 10380

tctggccagt ttatcatctg gccttcctgc ctatttttta caaaacacga agacagtgtg 10440

taacctcgac attttgacct tcctttatgt gctagtttag acaggctcct gaatccacac 10500

ttaattttgc ttaacaaaag tcttaatagt aaacctcccc tcatgagctt gaagtcaagt 10560

gttcttgact tcagatattt ctttcctttt tttttttttt tcctcatcac aactaagaga 10620

tacacaaact ctgaagaagc agaaatggag agaatgcttt taacaaaaaa gcatctgatg 10680

aaagatttta ggcaaacatt ctcaaaataa gagtgatatt ctggatgtag ttattgcagt 10740

tatctcatga caaatgaggc ctggattgga aggaaaatat agttgtgtag aattaagcat 10800

tttgatagga atctacaagg tagttgaata taataagcag gtttgggccc ccaaacttta 10860

gaaaatcaaa tgcaaaggtg ctggcaaaaa tgaggtttga gtggctggct gtaagagaag 10920

gttaactcct agtaaaaggc atttttagaa ataacaatta ctgaaaactt tgaagtatag 10980

tgggagtagc aaacaaatac atgttttttt tttcttacaa agaactccta aatcctgagt 11040

aagtgccatt cattacaata agtctctaaa tttaaaaaaa aaaaaatcat atgaggaaat 11100

ctagctttcc cctttacgct gcgtttgatc tttgtctaaa tagtgttaaa attcctttca 11160

ttccaattac agaactgagc ccactcgcaa gttggagcca tcagtgggat acgccacatt 11220

ttggaagccc cagcatcgtg tacttaccag tgtgttcaca aaatgaaatt tgtgtgagag 11280

ctgtacatta aaaaaaatca tcattattat tattatttgc agtcatggag aaccacctac 11340

ccctgacttc tgtttagtct cctttttaaa taaaaattac tgtgttagag aagaaggcta 11400

ttaaatgtag tagttaacta tgcctcttgt ctgggggttt catagagacc ggtaggaaag 11460

cgcactcctg cttttcgatt tatggtgtgt gcaagtaaac aggtgcattg ctttcaacct 11520

gccatactag ttttaaaaat tcactgaaat tacaaagata catatatatg catatatata 11580

atggaaagtt tcccggaatg caacaattag cattttaaaa tcatatatag gcatgcacat 11640

tctaaatagt actttttcat gcttcattgt ttctctggca gataatttta ctaagaagaa 11700

aaatagatat tcgactcccc ttccctaaac aaatccacgg gcagaggctc cagcggagcc 11760

gagccccctg gttttctcgt aggccctaga cggtgttgca tttatcagtg atgtcaaacg 11820

tgctcatttg tcagacatag ctgtaaatga aaacaatgtg tggcaaaata caaagttagt 11880

taaatacaca ccctctgtgt gattttttgc tcccttttct tttttgctcc tactcaaaaa 11940

aaaaaaaatc acctccttta catttccctg gcttcttgca tgtttccctt ttcaaaaacc 12000

atgtaataat tttttacaat gtatctgaca cattaatata ttgacatcaa ataggcagac 12060

attctacttt tgcctggcaa ataaatctgc tacggagaca tcatttcctc actgtctcaa 12120

agccataact acctgggagt ctttcaacac agacccctcc gatgggaaat gctgtttatt 12180

actgaatgca ggatgctcac gctctgatct tttctccctt gtgcctttac cccagtcatt 12240

tttacttagc aacaccaatt ctagatactt ctgttctgaa gtagaaccac ccccttgcca 12300

cactgccagt tttcctgcta aaagcagtgg acagaagaca gatcatggtc accctcacaa 12360

acatggcaca cagctgtctc ggtagctgca ttcccagcat gtcctggtct aaatatctag 12420

agttgcctat gacacgttca aaggttccca agcacagtac attgggaggc ttttgctgct 12480

gtggccgttg ttttcgttta ggccaactta cttccgtatt cacatactct tggctttacg 12540

aaatacactc ctccagtcta ctaggccaat caatatattt aaaagtctga ttgccacata 12600

agtctctctc tctctctttt tgttttttgt ttgtttgttt ttttctgttt tggctgccgg 12660

tagttaaaga ctgagatagg ttggaagact aaaatacagg agtacatgag tgacaacctt 12720

cagccgtctg atttccatgc cggtaaaaca cacaaccaag ctcttcttag cgctgctaat 12780

ataaacattc actaagaggg aataggaagt gagatttacc agcttcactt tgctgatttg 12840

caaggttccc cactacgatt cactgtcatt tgatttttga aaaataattt tgtccgtctc 12900

tttgaagaaa tgtcttagtt cttttatttt gtttgtttgg ttttttttag agaagtttta 12960

tctgcagtga taggctacaa tttttatctc cgctgattat ttgtcaggat gctgaatgaa 13020

taatttggtc ctgtgccttc cttgttgttc tgaggaaaat aagagaaact tggaagtttg 13080

tttcactctt agcccatcct aaatctaaaa gaagatgtcc caggtccagg caggccatgt 13140

agtagttata aaggaggtgg tccaggtcca gccacctcaa tcaggatttg tttgttttga 13200

agcatttgct taaaagcgga gcaagagtct taacccaact tgccataaca ctgcttttct 13260

cgcttttgat gtaaatcttc aaaattcaga catcaaacag ccccagaaaa ggggaattct 13320

ctccaggcat tgctccgccc cagctcctga acaaacccag ctctgtctag catttttttc 13380

cctagcgggg gtaggggaca gggtgagaga atttcagtct cccaggctgt ctcatgattg 13440

ttagggcata aagaaacaca gtcctgccac aaattgggag catctttacc ctttagagag 13500

aaacaaaaca aaactaaaca aacaaatcaa attgctttgc atgaaggcgt agcaaataaa 13560

atctcgggct ccctgttccc tgcaccattt gtaggaggtg agaaatgagg gaaacaagag 13620

aaaggggaac tttaaaagcg ggaggcccag aaataatccc tgttaccagt ctgaatttca 13680

cttgctccgt ggctaacgtc agacctagtg tgcatgtatg ccagaagtaa actaggctcg 13740

gctgtccatt tctttaaaat atgttcacat gtttcctttt tgaaaacaat tttggggact 13800

aaacccaaat ggagagattt gaggaaatcg ttaatgtctt aacatttgag tatatttata 13860

aatgtatcag tctgtgat 13878

## Description

### REFERENCE TO SEQUENCE LISTING

The content of the electronically submitted sequence listing ("3190_011PC01_SequenceListing_ascii.txt", 48,430 bytes, created on October 7, 2014) filed with the application is incorporated herein by reference in its entirety.

### BACKGROUND OF THE INVENTION

### Field of the Invention

In one embodiment, he present invention relates to the prognosis of bone metastasis in ERBB2 positive (human epidermal receptor 2 positive, HER2+) breast cancer, based on determining the levels of the MAF gene, and/or 16q23 or 16q22-24 locus amplification or translocation, in a primary tumor sample. Likewise, in one embodiment, the present invention also relates to a method for designing a customized therapy in a subject with ERBB2 positive (human epidermal receptor 2 positive, HER2+) breast cancer, which comprises determining the MAF gene expression level, and/or 16q23 or 16q22-24 locus amplification or translocation. Finally, in another embodiment, the present invention relates to the use of a c-Maf inhibitor as a therapeutic agent in the treatment of HER2+ breast cancer metastasis, in particular bone metastasis.

### Background Art

Breast cancer is the second most common type of cancer worldwide (10.4%; after lung cancer) and the fifth most common cause of death by cancer (after lung cancer, stomach cancer, liver cancer, and colon cancer). Among women, breast cancer is the most common cause of death by cancer. In 2005, breast cancer caused 502,000 deaths worldwide (7% of the deaths by cancer; almost 1% of all deaths). The number of cases worldwide has increased significantly from the 1970s, a phenomenon which is partly due to the modern lifestyle in the western world.

Breast cancer is classified into stages according to the TNM system. (*See* American Joint Committee on Cancer. AJCC Cancer Staging Manual. 6th ed. New York, NY: Springer, 2002, which is incorporated herein by reference in its entirety.) The prognosis is closely related to the results of the stage classification, and the stage classification is also used to assign patients to treatments both in clinical trials and in the medical practice. The information for classifying into stages is as follow:
- TX: The primary tumor cannot be assessed. T0: there is no evidence of tumor. Tis: *in situ* carcinoma, no invasion. T1: The tumor is 2 cm or less. T2: The tumor is more than 2 cm but less than 5 cm. T3: The tumor is more than 5 cm. T4: Tumor of any size growing in the wall of the breast or skin, or inflammatory breast cancer.
- NX: The nearby lymph nodes cannot be assessed. N0: The cancer has not spread to the regional lymph nodes. N1: The cancer has spread to 1 to 3 axillary lymph nodes or to one internal mammary lymph node. N2: The cancer has spread to 4 to 9 axillary lymph nodes or to multiple internal mammary lymph nodes. N3: One of the followings applies:
   ▪ The cancer has spread to 10 or more axillary lymph nodes, or the cancer has spread to the infraclavicular lymph nodes, or the cancer has spread to the supraclavicular lymph nodes or the cancer affects the axillary lymph nodes and has spread to the internal mammary lymph nodes, or the cancer affects 4 or more axillary lymph nodes and minimum amounts of cancer are in the internal mammary nodes or in sentinel lymph node biopsy.
- MX: The presence of distant spread (metastasis) cannot be assessed. M0: There is no distant spread. M1: spreading to distant organs which do not include the supraclavicular lymph node has been produced.

The fact that most of the patients with solid tumor cancer die after metastasis means that it is crucial to understand the molecular and cellular mechanisms allowing a tumor to metastasize. Recent publications have demonstrated how the metastasis is caused by means of complex yet little known mechanisms and also how the different metastatic cell types have a tropism towards specific organs These tissue specific metastatic cells have a series of acquired functions allowing them to colonize specific organs.

All cells have receptors on their surface, in their cytoplasm and in the cell nucleus. Certain chemical messengers such as hormones bind to said receptors and this causes changes in the cell. There are three significant receptors which may affect the breast cancer cells: estrogen receptor (ER), progesterone receptor (PR) and HER2/neu. For the purpose of naming the cells having any of these receptors, a positive sign is placed thereto when the receptor is present and a negative sign if it is absent: ER positive (ER+), ER negative (ER-), PR positive (PR+), PR negative (PR-), HER2 positive (HER2+) and HER2 negative (HER2-). The receptor state has become a critical assessment for all breast cancers since it determines the suitability of using specific treatments, for example, tamoxifen or trastuzumab.

Unsupervised gene expression array profiling has provided biological evidence for the heterogeneity of breast cancer through the identification of intrinsic subtypes such as luminal A, luminal B, HER2+/ER- and the basal-like subtype.

HER2+ cancers are defined as tumors that over-express the gene HER2, including different levels of overexpression. This subgroup accounts for 30% of all types of breast cancer. It is strongly associated with increased disease recurrence and a poor prognosis. Over-expression is also known to occur in ovarian, stomach, and aggressive forms of uterine cancer, such as uterine serous endometrial cancer

The keystone for treating breast cancer is surgery when the tumor is localized with possible adjuvant hormone therapy (with tamoxifen or an aromatase inhibitor), chemotherapy, and/or radiotherapy. Currently, the suggestions for treatment after the surgery (adjuvant therapy) follow a pattern. This pattern is subject to change because every two years a world conference takes place in St. Gallen, Switzerland to discuss the actual results of the worldwide multi-center studies. Likewise, said pattern is also reviewed according to the consensus criterion of the National Institute of Health (NIH). Based on these criteria, more than 85-90% of the patients not having metastasis in lymph nodes would be candidates to receive adjuvant systemic therapy. HER2+ patients are susceptible to an adjuvant targeted therapy against HER2. HER2 is the target of the monoclonal antibody trastuzumab (marketed as Herceptin). Trastuzumab is effective only in cancers where HER2 is over-expressed. Alternative monoclonal antibodies, such as Pertuzumab, which inhibits dimerization of HER2 and HER3 receptors, antibodies combined to drugs, T-DM1, and small inhibitors are potential targeted therapeutic options. HER2 testing is performed in breast cancer patients to assess prognosis and to determine suitability for trastuzumab therapy. It is important that trastuzumab is restricted to HER2-positive individuals as it is expensive and has been associated with cardiac toxicity. For HER2- tumors, the risks of trastuzumab clearly outweigh the benefits.

Tests are usually performed on biopsy samples obtained by either fine-needle aspiration, core needle biopsy, vacuum-assisted breast biopsy, or surgical excision. Immunohistochemistry is used to measure the amount of HER2 protein present in the sample. Alternatively, fluorescence in situ hybridization (FISH) can be used to measure the number of copies of the gene, which are present.

Currently, PCR assays such as Oncotype DX or microarray assays such as MammaPrint can predict the risk of breast cancer relapse based on the expression of specific genes. In February 2007, the MammaPrint assay became the first breast cancer indicator in achieving official authorization from the Food and Drug Administration.

Patent application EP1961825-A1 describes a method for predicting the occurrence of breast cancer metastasis to bone, lung, liver or brain, which comprises determining in a tumor tissue sample the expression level of one or more markers with respect to their corresponding expression level in a control sample, among which include MAF. However, this document requires determining several genes simultaneously to enable determining the survival of breast cancer patients and the correlation between the capacities of the gene signature for predicting the survivability free from bone metastasis was not statistically significant.

Patent application US2011/0150979 describes a method for predicting a prognosis of a basal like breast cancer comprising detecting the level of FOXC1.

Patent application US2010/0210738 relates to a method for prognosing cancer in a subject with triple negative breast cancer comprising detecting in a sample the expression levels of a series of genes which are randomly up-regulated or downregulated.

Patent application US2011/0130296 relates to the identification of marker genes useful in the diagnosis and prognosis of triple negative breast cancer.

There is the need of identifying new markers which allow predicting the probability of a subject suffering triple negative breast cancer to develop metastasis. The identification of new prognosis factors will serve as a guide in selecting the most suitable treatments.

### SUMMARY OF THE INVENTION

In one aspect, the present invention relates to an *in vitro* method for predicting bone metastasis of a Her2+ breast cancer, in a subject suffering said cancer which comprises
i) determining the expression level of the MAF gene in a sample of said subject and
ii) comparing the expression level obtained in step i) with a reference value,
wherein increased expression level of said gene with respect to said reference value is indicative of increased risk of developing bone metastasis. In some embodiments, the increased expression level is covered through locus amplification. In some embodiments, the increased expression level is covered through locus translocation.

In another aspect, the present invention relates to an *in vitro* method for predicting the clinical outcome of a patient suffering from bone metastatic HER2+ breast cancer, which comprises
i) quantifying the expression level of the MAF gene in a sample of said subject and
ii) comparing the expression level obtained in step i) with a reference value,
wherein increased expression level of said gene with respect to said reference value is indicative of a poor clinical outcome.

In another aspect, the present invention relates to an *in vitro* method for designing a customized therapy for a subject suffering from HER2+ breast cancer, which comprises
i) quantifying the MAF gene expression level in a sample of said subject and
ii) comparing the expression level obtained in i) with a reference value,
wherein if the expression level is increased with respect to said reference value, then said subject is susceptible to receive a therapy aiming to prevent, inhibit and/or treat the bone metastasis. In a particular aspect of this method, the subject is then administered at least one therapeutic drug that prevents, inhibits and/or treats the bone metastasis. If the expression level is not increased with respect to said reference value, then said subject is not susceptible to receive a therapy aiming to prevent, inhibit and/or treat the bone metastasis. In a particular aspect of this method, the subject is then not administered at least one therapeutic drug that prevents, inhibits and/or treats the bone metastasis.

In another aspect, the present invention relates to a method for determining the risk of bone metastasis in a subject suffering from breast cancer, for example HER2+ breast cancer, which comprises determining the expression level of the MAF gene in a sample of said subject wherein expression levels of said gene above the average value plus one standard deviation is indicative of an increased risk of early bone metastasis. In a particular aspect of this method, the subject is then administered at least one therapeutic drug that prevents or inhibits the bone metastasis.

In another aspect, the present invention relates to an *in vitro* method for designing a customized therapy for a subject with HER2+ breast cancer with bone metastasis which comprises
i) quantifying the MAF gene expression level in a bone metastatic sample of said subject and
ii) comparing the expression level obtained in step (i) with a reference value,
wherein if the MAF gene expression level is increased with respect to said reference value, then said subject is susceptible to receive a therapy for preventing the bone degradation. In a particular aspect of this method, the subject is then administered at least one therapeutic drug that prevents, inhibits and/or treats the bone metastasis.
If the MAF gene expression level is not increased with respect to said reference value, then said subject is not susceptible to receive a therapy for preventing the bone degradation. In a particular aspect of this method, the subject is then not administered at least one therapeutic drug that prevents, inhibits and/or treats the bone metastasis.

In another aspect, the present invention relates to an *in vitro* method for predicting bone metastasis of a HER2+ breast cancer, in a subject suffering said cancer which comprises determining if the MAF gene is amplified in a sample of said subject relative to a reference gene copy number wherein an amplification of the MAF gene with respect to said reference gene copy number is indicative of increased risk of developing bone metastasis. In a particular aspect of this method, the subject is then administered at least one therapeutic drug that prevents or inhibits the bone metastasis.

In another aspect, the present invention relates to an *in vitro* method for predicting bone metastasis of breast cancer, for example HER2+ breast cancer, in a subject suffering said cancer which comprises determining if the MAF gene is translocated in a sample of said subject wherein a translocation of the MAF gene is indicative of increased risk of developing bone metastasis. In a particular aspect of this method, the subject is then administered at least one therapeutic drug that prevents, or inhibits the bone metastasis.

In another aspect, the present invention relates to an *in vitro* method for predicting the clinical outcome of a patient suffering HER2+ breast cancer, which comprises determining if the MAF gene is amplified in a sample of said subject relative to a reference gene copy number wherein an amplification of the MAF gene with respect to said reference gene copy number is indicative of a poor clinical outcome. In a particular aspect of this method, the subject is then administered at least one therapeutic drug that prevents, inhibits and/or treats the bone metastasis. If such amplification is not observed then the subject is then not administered at least one therapeutic drug that prevents, inhibits and/or treats the bone metastasis. In another embodiment, the present invention relates to an *in vitro* method for predicting the clinical outcome of a patient suffering HER2+ breast cancer which comprises determining if the MAF gene is translocated in a sample of said subject wherein a translocation of the MAF gene (*i.e.* t(14,16)) is indicative of a poor clinical outcome. In some embodiments, the present invention relates to designing a customized therapy for patients with the amplification or translocation of MAF. In some embodiments, the customized therapy is at least one therapeutic drug that prevents, inhibits and/or treats the bone metastasis.

In another aspect, the present invention relates to a c-Maf inhibitory agent for use in the prevention of bone metastasis from HER2+ breast cancer.

In another aspect, the present invention relates to a c-Maf inhibitory agent or an agent capable of avoiding or preventing bone degradation for use in the treatment of bone metastasis in a subject suffering from HER2+ breast cancer, and having elevated MAF levels in a metastatic sample with respect to a control sample.

In another aspect, the present invention relates to a kit for predicting bone metastasis of a Her2+ breast cancer in a subject suffering from said cancer, the kit comprising: a) means for quantifying the expression level of MAF in a sample of said subject; and b) means for comparing the quantified level of expression of MAF in said sample to a reference MAF expression level. The present invention also relates to the use of such kit to predict bone metastasis of a Her2+ breast cancer in a subject suffering from said cancer. In one embodiment, the subject is then administered or not administered at least one therapeutic drug that prevents, inhibits and/or treats the bone metastasis based on the results of using the kit.

For example, KRAS mutant patients do not receive anti-EGFR therapy (*i.e.* Cetuximab) as it targets a receptor upstream from KRAS, thus the mutation makes any action upstream useless, and patients do not benefit from the therapy. Patient who are wildtype for KRAS do benefit from the use of the drug as it blocks the RAS pathway. In another aspect, the present invention relates to a kit for predicting bone metastasis of a Her2+ breast cancer in a subject suffering from said cancer, the kit comprising: a) means for determining translocation of the MAF gene, 16q23 or 16q22-24 locus amplification or translocation in a sample of said subject; and b) means for comparing the translocation of MAF gene, 16q23 or 16q22-24 locus amplification or translocation, in said sample to a reference MAF sample. The present invention also relates to the use of such kit to predict bone metastasis of a breast cancer in a subject suffering from said cancer. In one embodiment, the subject is then administered or excluded at least one therapeutic drug that prevents, inhibits and/or treats the bone metastasis based on the results of using the kit.

In another aspect, the present invention relates to a kit for predicting bone metastasis of a Her2+ breast cancer in a subject suffering from said cancer, the kit comprising: a) means for quantifying the amplification of MAF gene, 16q23 or 16q22-24 locus amplification or translocation in a sample of said subject; and b) means for comparing the amplified level of MAF gene, 16q23 or 16q22-24 locus amplification or translocation in said sample to a reference.

In another aspect, the present invention relates to a kit for predicting the clinical outcome of a subject suffering from bone metastasis from a HER2+ breast cancer, the kit comprising: a) means for quantifying the expression level of MAF in a sample of said subject; and b) means for comparing the quantified expression level of MAF in said sample to a reference MAF expression level. The present invention also relates to the use of such kit to predict the clinical outcome of a subject suffering from bone metastasis from a HER2+ breast cancer. In one embodiment, the subject is then administered or not administered at least one therapeutic drug that prevents, inhibits and/or treats the bone metastasis based on the results of using the kit.

In another aspect, the present invention relates to a kit for determining a therapy for a subject suffering from Her2+ breast cancer, the kit comprising: a) means for quantifying the expression level of MAF in a sample of said subject; b) means for comparing the quantified expression level of MAF in said sample to a reference MAF expression level; and c) means for determining a therapy for preventing and/or reducing bone metastasis in said subject based on the comparison of the quantified expression level to the reference expression level. The present invention also relates to the use of such kit to determine a therapy for a subject suffering from breast cancer. In one embodiment, the subject is then administered or not administered at least one therapeutic drug that prevents, inhibits and/or treats the bone metastasis based on the results of using the kit.

In another aspect, the present invention relates to a kit comprising: i) a reagent for quantifying the expression level of MAF in a sample of a subject suffering from HER2+ breast cancer, and ii) one or more MAF gene expression level indices that have been predetermined to correlate with the risk of bone metastasis. The present invention also relates to the use of such kit to predict bone metastasis of a Her2+ breast cancer in a subject suffering from said cancer. In one embodiment, the subject is then administered or not administered at least one therapeutic drug that prevents, inhibits and/or treats the bone metastasis based on the results of using the kit.

In another aspect, the present invention relates to an *in vitro* method for typing a sample of a subject suffering from Her2+ breast cancer, the method comprising:
a) providing a sample from said subject;
b) quantifying the expression level of MAF in said sample;
c) typing said sample by comparing the quantified expression level of MAF to a predetermined reference level of MAF expression;
wherein said typing provides prognostic information related to the risk of bone metastasis in said subject. In one embodiment, the subject is administered or not administered at least one therapeutic agent based on the prognostic information provided by the typing.

In another aspect, the present invention relates to a method for preventing or reducing the risk of bone metastasis in a subject suffering from HER2+ breast cancer, said method comprising administering to said subject an agent that prevents or reduces bone metastasis, wherein said agent is administered in accordance with a treatment regimen determined from quantifying the expression level of MAF in said subject.

In another aspect, the present invention relates to a method of classifying a subject suffering from Her2+ breast cancer into a cohort, comprising: a) determining the expression level of MAF in a sample of said subject; b) comparing the expression level of MAF in said sample to a predetermined reference level of MAF expression; and c) classifying said subject into a cohort based on said expression level of MAF in the sample. In a particular aspect, the cohort is used for conducting a clinical trial.

### BRIEF DESCRIPTION OF THE DRAWINGS

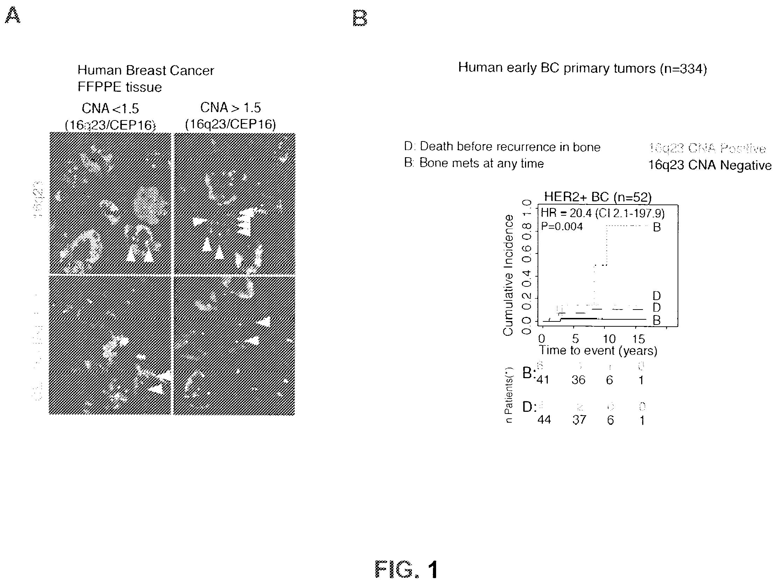

**Figure 1****:** 16q22-24, including MAF gene association with bone metastasis incidence in breast cancer primary tumors.
a) FISH using a 16q23 region probe, within the 16q22-24 DNA amplified region, and CEP16(16q11.2) probes to measure 16q22-24 DNA amplification in HER2+ primary breast cancer tissues. Representative images of patient sample without 16q23 amplification (Case1, 16q23/CEP16 CNA < than 1.5 copies) and with 16q23 amplification (Case2, 16q23/CEP16 CNA> than 1.5 copies).
b) Cumulative incidence of bone metastasis at any time, using death before recurrence in bone as a competing event in stage I, II and III BC human primary tumor data set (n=334). Patients were stratified according to 16q23/CEP16 CNA negative (<1.5) and 16q23/CEP16 CNA positive (> or = 1.5) group based on cut-off of 1.5 16q23/CEP16 copies per cell. A minimum of 50 cells per core and 3 cores per tumor were scored. HR-hazard ratio, CI-Confidence Interval.

### DETAILED DESCRIPTION OF THE INVENTION

### Definitions of general terms and expressions

"And/or" where used herein is to be taken as specific disclosure of each of the two specified features or components with or without the other. For example 'A and/or B' is to be taken as specific disclosure of each of (i) A, (ii) B and (iii) A and B, just as if each is set out individually herein.

As used herein, "agent for avoiding or preventing bone degradation" refers to any molecule capable of preventing, inhibiting, treating, reducing, or stopping bone degradation either by stimulating the osteoblast proliferation or inhibiting the osteoclast proliferation or fixing the bone structure.

As used herein, the term "amplification of a gene" refers to a process through which various copies of a gene or of a gene fragment are formed in an individual cell or a cell line. The copies of the gene are not necessarily located in the same chromosome. The duplicated region is often called an "amplicon". Normally, the amount of mRNA produced, *i.e.,* the gene expression level, also increases in proportion to the copy number of a particular gene.

As used herein, "HER2+" refers to a breast cancer which is characterized by tumor cells with detectable expression of epidermal growth factor receptor type 2 (HER2 or ErbB2) and/or amplification for the HER2 gene, a receptor normally located on the cell surface. As used herein, tumor cells are considered negative for HER2 overexpression if they yield a test result score of 0 or 1+, or 2+ when tested with a HercepTest™ Kit (Code K5204, Dako North America, Inc., Carpinteria, CA), a semiquantitative immunohistochemical assay using a polyclonal anti-HER2 primary antibody or if they are HER2 FISH negative.

As used herein, "c-MAF gene" or "MAF" (v-maf musculoaponeurotic fibrosarcoma oncogene homologue (avian) also known as MAF or MGC71685) is a transcription factor containing a leucine zipper which acts like a homodimer or a heterodimer. Depending on the DNA binding site, the encoded protein can be a transcriptional activator or repressor. The DNA sequence encoding MAF is described in the NCBI database under accession number NG_016440 (SEQ ID NO: 1 (coding)). The genomic sequence of MAF is set forth in SEQ ID NO:13. The methods of the present invention may utilize either the coding sequence or the genomic DNA sequence. Two messenger RNA are transcribed from said DNA sequence, each of which will give rise to one of the two MAF protein isoforms, the α isoform and the β isoform. The complementary DNA sequences for each of said isoforms are described, respectively, in the NCBI database under accession numbers NM_005360.4 (SEQ ID NO: 2) and NM_001031804.2 (SEQ ID NO: 3). Use of the MAF gene to predict the prognosis of cancer is found in, for example, Int'l. Appl. Nos. PCT/IB2013/001204 and PCT/ES2011/070693 and U.S. Appl. Nos. 13/878,114 and 13/878,114 (triple-negative breast cancer and ER+ breast cancer), Int'l Appl. No. PCT/US2014/026154 (renal cell carcinoma), Int'l Appl. No. PCT/US2014/028722 (breast cancer), Int'l Appl. No. PCT/US2013/044584 (lung cancer), U.S.. Appl. No. 14/050,262 and Int'l Appl. No. PCT/IB2013/002866 (prostate cancer), U.S. Appl. No. 14/213,670 and Int'l Appl. No. PCT/US2014/028569 (metastatic cancer), each of which are incorporated here by reference in its entirety.

As used herein, a "MAF inhibitory agent" refers to any molecule capable of completely or partially inhibiting the MAF gene expression, both by preventing the expression product of said gene from being produced (interrupting the MAF gene transcription and/or blocking the translation of the mRNA coming from the MAF gene expression) and by directly inhibiting the c-Maf protein activity. MAF gene expression inhibitors can be identified using methods based on the capacity of c-Maf for promoting *in vitro* cell proliferation as shown in Int'l Pat. Publ. WO2005/046731 (incorporated herein by reference in its entirety), based on the capacity of an inhibitor or test compound for blocking the transcription capacity of a reporter gene under the control of cyclin D2 promoter or of a promoter containing the c-Maf responsive region (MARE or c-Maf responsive element) in cells expressing c-Maf as described in Int'l Pat. Publ. WO2008/098351 (incorporated herein by reference in its entirety). Variants of c-Maf can also be identified based on the capacity of an inhibitor for blocking reporter gene expression under the control of the IL-4 promoter in response to the stimulation with PMA/ionomycin in cells expressing NFATc2 and c-Maf as described in U.S. Publ. No. US2009/048117A (incorporated herein by reference in its entirety).

As used herein, Mammalian target of rapamycin (mTOR) or "mTor" refers to those proteins that correspond to EC 2.7.11.1. mTor enzymes are serine/threonine protein kinases and regulate cell proliferation, cell motility, cell growth, cell survival, and transcription.

As used herein, an "mTor inhibitor" refers to any molecule capable of completely or partially inhibiting the mTor gene expression, both by preventing the expression product of said gene from being produced (interrupting the mTor gene transcription and/or blocking the translation of the mRNA coming from the mTor gene expression) and by directly inhibiting the mTor protein activity. Including inhibitors that have a dual or more targets and among them mTor protein activity.

As used herein, "Src" refers to those proteins that correspond to EC 2.7.10.2. Src is a non-receptor tyrosine kinase and a proto-oncogene. Src may play a role in cell growth and embryonic development.

As used herein, a "Src inhibitor" refers to any molecule capable of completely or partially inhibiting Src gene expression, both by preventing the expression product of said gene from being produced (interrupting the Src gene transcription and/or blocking the translation of the mRNA coming from the Src gene expression) and by directly inhibiting the Src protein activity.

As used herein, "Prostaglandin-endoperoxide synthase 2", "cyclooxygenase-2" or "COX-2" refers to those proteins that correspond to EC 1.14.99.1. COX-2 is responsible for converting arachidonic acid to prostaglandin endoperoxide H2.

As used herein, a "COX-2 inhibitor" refers to any molecule capable of completely or partially inhibiting COX-2 gene expression, both by preventing the expression product of said gene from being produced (interrupting the COX-2 gene transcription and/or blocking the translation of the mRNA coming from the COX-2 gene expression) and by directly inhibiting the COX-2 protein activity.

As used herein "outcome" or "clinical outcome" refers to the resulting course of disease and/or disease progression and can be characterized for example by recurrence, period of time until recurrence, metastasis, period of time until metastasis, number of metastases, number of sites of metastasis and/or death due to disease. For example a good clinical outcome includes cure, prevention of recurrence, prevention of metastasis and/or survival within a fixed period of time (without recurrence), and a poor clinical outcome includes disease progression, metastasis and/or death within a fixed period of time.

As used herein, the term "expression level" of a gene refers to the measurable quantity of gene product produced by the gene in a sample of the subject, wherein the gene product can be a transcriptional product or a translational product. Accordingly, the expression level can pertain to a nucleic acid gene product such as mRNA or cDNA or a polypeptide gene product. The expression level is derived from a subject's sample and/or a reference sample or samples, and can for example be detected de novo or correspond to a previous determination. The expression level can be determined or measured, for example, using microarray methods, PCR methods (such as qPCR), and/or antibody based methods, as is known to a person of skill in the art.

As used herein, the term "gene copy number" refers to the copy number of a nucleic acid molecule in a cell. The gene copy number includes the gene copy number in the genomic (chromosomal) DNA of a cell. In a normal cell (non-tumoral cell), the gene copy number is normally two copies (one copy in each member of the chromosome pair). The gene copy number sometimes includes half of the gene copy number taken from samples of a cell population.

"Increased expression level" is understood as the expression level when it refers to the levels of the MAF gene greater than those in a reference sample or control sample. Increased levels can be caused without excluding other mechanisms by a gene or 16q23 or 16q22-24 chromosomal locus amplification or translocation. Particularly, a sample can be considered to have high MAF expression level when the expression level in the sample isolated from the patient is at least about 1.1 times, about 1.5 times, about 5 times, about 10 times, about 20 times, about 30 times, about 40 times, about 50 times, about 60 times, about 70 times, about 80 times, about 90 times, about 100 times or even more with respect to the reference or control.

"Probe", as used herein, refers to an oligonucleotide sequence that is complementary to a specific nucleic acid sequence of interest. In some embodiments, the probes may be specific to regions of chromosomes which are known to undergo translocations. In some embodiments, the probes have a specific label or tag. In some embodiments, the tag is a fluorophore. In some embodiments, the probe is a DNA *in situ* hybridization probe whose labeling is based on the stable coordinative binding of platinum to nucleic acids and proteins. In some embodiments, the probe is described in U.S. Patent Appl. 12/067532 and U.S. Patent Appl. 12/181,399, which are incorporated by reference in their entirety, or as described in Swennenhuis et al. "Construction of repeat-free fluorescence in situ hybridization probes" Nucleic Acids Research 40(3):e20 (2012).

"Tag" or "label", as used herein, refers to any physical molecule which is directly or indirectly associated with a probe, allowing the probe or the location of the probed to be visualized, marked, or otherwise captured.

"Translocation", as used herein, refers to the exchange of chromosomal material in unequal or equal amounts between chromosomes. In some cases, the translocation is on the same chromosome. In some cases, the translocation is between different chromosomes. Translocations occur at a high frequency in many types of cancer, including breast cancer and leukemia. Translocations can be either primary reciprocal translocations or the more complex secondary translocations. There are several primary translocations that involve the immunoglobin heavy chain (IgH) locus that are believed to constitute the initiating event in many cancers. (Eychène, A., Rocques, N., and Puoponnot, C., A new MAFia in cancer. 2008. Nature Reviews: Cancer. 8: 683-693.)

"Polyploid" or "polyploidy", as used herein, indicates that the cell contains more than two copies of a gene of interest. In some instances, the gene of interest is MAF. In some embodiments, polyploidy is associated with an accumulation of expression of the gene of interest. In some embodiments, polyploidy is associated with genomic instability. In some embodiments, the genomic instability may lead to chromosome translocations.

"Whole genome sequencing", as used herein, is a process by which the entire genome of an organism is sequenced at a single time. *See, e.g.,* Ng., P.C. amd Kirkness, E.F., Whole Genome Sequencing. 2010. Methods in Molecular Biology. 628: 215-226.

"Exome sequencing", as used herein, is a process by which the entire coding region of the DNA of an organism is sequenced. In exome sequencing, the mRNA is sequenced. The untranslated regions of the genome are not included in exome sequencing. *See, e.g.,* Choi, M. et al., Genetic diagnosis by whole exome capture and massively parallel DNA sequencing. 2009. PNAS. 106(45): 19096-19101.

"Metastasis", as used herein, is understood as the propagation of a cancer from the organ where it started to a different organ. It generally occurs through the blood or lymphatic system. When the cancer cells spread and form a new tumor, the latter is called a secondary or metastatic tumor. The cancer cells forming the secondary tumor are like those of the original tumor. If a breast cancer, for example, spreads (metastasizes) to the lung, the secondary tumor is formed of malignant breast cancer cells. The disease in the lung is metastatic breast cancer and not lung cancer. In a particular embodiment of the method of the present invention, the metastasis is HER2+ breast cancer which has spread (metastasized) to the bone.

"Predicting", as used herein, refers to the determination of the likelihood that the subject suffering from HER2+ breast cancer will develop metastasis to a distant organ. As used herein, "good prognosis" indicates that the subject is expected (*e.g.* predicted) to survive and/or have no, or is at low risk of having, recurrence or distant metastases within a set time period. The term "low" is a relative term and, in the context of this application, refers to the risk of the "low" expression group with respect to a clinical outcome (recurrence, distant metastases, etc.). A "low" risk can be considered as a risk lower than the average risk for an heterogeneous cancer patient population. In the study of Paik et al. (2004), an overall "low" risk of recurrence was considered to be lower than 15 percent. The risk will also vary in function of the time period. The time period can be, for example, five years, ten years, fifteen years or even twenty years after initial diagnosis of cancer or after the prognosis was made.

As used herein, "poor prognosis" indicates that the subject is expected *e.g*. predicted to not survive and/or to have, or is at high risk of having, recurrence or distant metastases within a set time period. The term "high" is a relative term and, in the context of this application, refers to the risk of the "high" expression group with respect to a clinical outcome (recurrence, distant metastases, etc.). A "high" risk can be considered as a risk higher than the average risk for a heterogeneous cancer patient population. In the study of Paik et al. (2004), an overall "high" risk of recurrence was considered to be higher than 15 percent. The risk will also vary in function of the time period. The time period can be, for example, five years, ten years, fifteen years or even twenty years of initial diagnosis of cancer or after the prognosis was made.

"Reference value", as used herein, refers to a laboratory value used as a reference for values/data obtained by laboratory examinations of patients or samples collected from patients. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested.

As used herein, "Subject" or "patient" refers to all animals classified as mammals and includes but is not limited to domestic and farm animals, primates and humans, for example, human beings, non-human primates, cows, horses, pigs, sheep, goats, dogs, cats, or rodents. Preferably, the subject is a human man or woman of any age or race.

The term "treatment", as used herein, refers to any type of therapy, which aims at terminating, preventing, ameliorating or reducing the susceptibility to a clinical condition as described herein. In a preferred embodiment, the term treatment relates to prophylactic treatment (*i.e.* a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

As used herein, "sample" or "biological sample" means biological material isolated from a subject. The biological sample may contain any biological material suitable for determining the expression level of the MAF gene. The sample can be isolated from any suitable biological tissue or fluid such as, for example, tumor tissue, blood, blood plasma, serum, urine or cerebral spinal fluid (CSF).

"Tumor tissue sample" is understood as the tissue sample originating from the primary HER2+ breast cancer tumor. Said sample can be obtained by conventional methods, for example biopsy, using methods well known by the persons skilled in related medical techniques.

"Osteolytic bone metastasis" refers to a type of metastasis in which bone resorption (progressive loss of the bone density) is produced in the proximity of the metastasis resulting from the stimulation of the osteoclast activity by the tumor cells and is characterized by severe pain, pathological fractures, hypercalcaemia, spinal cord compression and other syndromes resulting from nerve compression.

### Method for predicting bone metastasis of a HER2+ breast cancer, based on the expression level of MAF

The expression level of MAF in samples of a HER2+ breast cancer, correlates with the risk of suffering bone metastasis. Moreover, gene expression of MAF in HER2+ primary tumors, correlates significantly with bone metastasis recurrence, and inversely with bone metastasis-free survival and survival. Moreover, the MAF expression levels predict bone metastasis in a dose-dependent manner.

In a first aspect, the present invention relates to an *in vitro* method (hereinafter first method of the present invention) for predicting bone metastasis of a HER2+ breast cancer, in a subject suffering said cancer which comprises:
i) determining the expression level of the MAF gene in a sample of said subject and
ii) comparing the expression level obtained in step i) with a reference value,
wherein increased expression level of said gene with respect to said reference value is indicative of increased risk of developing bone metastasis.

The method of the present invention comprises in a first step determining the MAF gene expression level in a sample from a subject. In a preferred embodiment, the sample is a tumor tissue sample.

The methods for obtaining a biopsy sample include splitting a tumor into large pieces, or microdissection, or other cell separating methods known in the art. The tumor cells can additionally be obtained by means of cytology through aspiration with a small gauge needle. To simplify sample preservation and handling, samples can be fixed in formalin and soaked in paraffin or first frozen and then soaked in a tissue freezing medium such as OCT compound by means of immersion in a highly cryogenic medium which allows rapid freezing.

In a preferred embodiment, the first method of the present invention comprises quantifying only the MAF gene expression level as a single marker, *i.e.,* the method does not involve determining the expression level of any additional marker.

As understood by the person skilled in the art, the gene expression level can be quantified by measuring the messenger RNA levels of said gene or of the protein encoded by said gene, as well as the number of genomic region copies or translocations containing said gene.

For this purpose, the biological sample can be treated to physically or mechanically break up the tissue or cell structure, releasing the intracellular components into an aqueous or organic solution for preparing nucleic acids. The nucleic acids are extracted by means of commercially available methods known by the person skilled in the art (Sambrook, J., et al., "Molecular cloning: a Laboratory Manual", 3rd ed., Cold Spring Harbor Laboratory Press, N.Y., Vol. 1-3.)

Thus, the MAF gene expression level can be quantified from the RNA resulting from the transcription of said gene (messenger RNA or mRNA) or, alternatively, from the complementary DNA (cDNA) of said gene. Therefore, in a particular embodiment of the present invention, the quantification of the MAF gene expression level comprises the quantification of the messenger RNA of the MAF gene or a fragment of said mRNA, complementary DNA of the MAF gene or a fragment of said cDNA or the mixtures thereof.

Virtually any conventional method can be used within the scope of the present invention for detecting and quantifying the mRNA levels encoded by the MAF gene or of the corresponding cDNA thereof. By way of non-limiting illustration, the mRNA levels encoded by said gene can be quantified using conventional methods, for example, methods comprising mRNA amplification and the quantification of said mRNA amplification product, such as electrophoresis and staining, or alternatively, by Southern blot and using suitable probes, Northern blot and using specific probes of the mRNA of the gene of interest (MAF) or of the corresponding cDNA thereof, mapping with S1 nuclease, RT-PCR, hybridization, microarrays, etc., preferably by means of real time quantitative PCR using a suitable marker. Likewise, the cDNA levels corresponding to said mRNA encoded by the MAF gene can also be quantified by means of using conventional techniques; in this case, the method of the present invention includes a step for synthesizing the corresponding cDNA by means of reverse transcription (RT) of the corresponding mRNA followed by the amplification and quantification of said cDNA amplification product. Conventional methods for quantifying expression level can be found, for example, in Sambrook *et al.,* 2001. (cited *ad supra*). These methods are known in the art and a person skilled in the art would be familiar with the normalizations necessary for each technique. For example, the expression measurements generated using multiplex PCR should be normalized by comparing the expression of the genes being measured to so called "housekeeping" genes, the expression of which should be constant over all samples, thus providing a baseline expression to compare against or other control genes whose expression are known to be modulated with cancer.

In a particular embodiment, the MAF gene expression level is quantified by means of quantitative polymerase chain reaction (PCR) or a DNA/RNA array or nucleotide hybridization technique.

In addition, the MAF gene expression level can also be quantified by means of quantifying the expression level of the protein encoded by said gene, *i.e.,* the c-Maf protein (c-Maf) [NCBI, accession number O75444], or any functionally equivalent variant of the c-Maf protein. There are two c-Maf protein isoforms, the α isoform (NCBI, NP_005351.2) made up of 403 amino acids (SEQ ID NO: 4) and the β isoform (NCBI, NP_001026974.1) made up of 373 amino acids (SEQ ID NO: 5). The MAF gene expression level can be quantified by means of quantifying the expression level of any of the c-Maf protein isoforms. Thus, in a particular embodiment, the quantification of the level of the protein encoded by the MAF gene comprises the quantification of the c-Maf protein.

In the context of the present invention, "functionally equivalent variant of the c-Maf protein" is understood as (i) variants of the c-Maf protein (SEQ ID NO: 4 or SEQ ID NO: 5) in which one or more of the amino acid residues are substituted by a conserved or non-conserved amino acid residue (preferably a conserved amino acid residue), wherein such substituted amino acid residue may or may not be one encoded by the genetic code, or (ii) variants comprising an insertion or a deletion of one or more amino acids and having the same function as the c-Maf protein, *i.e.,* to act as a DNA binding transcription factor. Variants of the c-Maf protein can be identified using methods based on the capacity of c-Maf for promoting *in vitro* cell proliferation as shown in international patent application WO2005/046731 (incorporated herein by reference in its entirety), based on the capacity of the so-called inhibitor for blocking the transcription capacity of a reporter gene under the control of cyclin D2 promoter or of a promoter containing the MAF responsive region (MARE or MAF responsive element) in cells expressing MAF as described in WO2008098351 (incorporated herein by reference in its entirety), or based on the capacity of the so-called inhibitor for blocking reporter gene expression under the control of the IL-4 promoter in response to the stimulation with PMA/ionomycin in cells expressing NFATc2 and MAF as described in US2009048117A (incorporated herein by reference in its entirety).

The variants according to the present invention preferably have sequence similarity with the amino acid sequence of any of the c-Maf protein isoforms (SEQ ID NO: 4 or SEQ ID NO: 5) of at least about 50%, at least about 60%, at least about 70%, at least about 80%, at least about 90%, at least about 91%, at least about 92%, at least about 93%, at least about 94%, at least about 95%, at least about 96%, at least about 97%, at least about 98% or at least about 99%. The degree of similarity between the variants and the specific c-Maf protein sequences defined previously is determined using algorithms and computer processes which are widely known by the persons skilled in the art. The similarity between two amino acid sequences is preferably determined using the BLASTP algorithm [BLAST Manual, Altschul, S., et al., NCBI NLM NIH Bethesda, Md. 20894, Altschul, S., et al., J. Mol. Biol. 215: 403-410 (1990)].

The c-Maf protein expression level can be quantified by any conventional method which allows detecting and quantifying said protein in a sample from a subject. By way of non-limiting illustration, said protein levels can be quantified, for example, by using antibodies with c-Maf binding capacity (or a fragment thereof containing an antigenic determinant) and the subsequent quantification of the complexes formed. The antibodies used in these assays may or may not be labeled. Illustrative examples of markers that can be used include radioactive isotopes, enzymes, fluorophores, chemiluminescence reagents, enzyme substrates or cofactors, enzyme inhibitors, particles, dyes, etc. There is a wide range of known assays that can be used in the present invention which use unlabeled antibodies (primary antibody) and labeled antibodies (secondary antibody); these techniques include Western-blot or Western transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein microarrays or biochips including specific antibodies or assays based on colloidal precipitation in formats such as dipsticks. Other ways for detecting and quantifying said c-Maf protein include affinity chromatography techniques, ligand binding assays, etc. When an immunological method is used, any antibody or reagent that is known to bind to the c-Maf protein with a high affinity can be used for detecting the amount thereof. Nevertheless, the use of an antibody, for example, polyclonal sera, supernatants of hybridomas or monoclonal antibodies, antibody fragments, Fv,Fab, Fab' and F(ab')2, scFv, humanized diabodies, triabodies, tetrabodies, nanobodies, alphabodies, stapled peptides, cyclopeptides and antibodies is preferred. There are commercial anti-c-Maf protein antibodies on the market which can be used in the context of the present invention, such as for example antibodies ab427, ab55502, ab55502, ab72584, ab76817, ab77071 (Abcam plc, 330 Science Park, Cambridge CB4 0FL, United Kingdom), the O75444 monoclonal antibody (Mouse Anti-Human MAF Azide free Monoclonal antibody, Unconjugated, Clone 6b8) of AbD Serotec, etc. There are many commercial companies offering anti-c-Maf antibodies, such as Abnova Corporation, Bethyl Laboratories, Santa Cruz Biotechnology, Bioworld Technology, GeneTex, etc.

In a particular embodiment, the c-Maf protein levels are quantified by means of western blot, ELISA or a protein array.

In another particular embodiment, the c-Maf protein levels are quantified from exosomes, circulating DNA or circulating tumor cells. Exosomes are 40 - 100 nm membrane vesicles secreted by most cell types in *vivo* and *in vitro.* Exosomes form in a particular population of endosomes, called multivesicular bodies (MVBs) by inward budding into the lumen of the compartment. Upon fusion of MVBs with the plasma membrane, these internal vesicles are secreted. Exosomes can be isolated from diverse cell lines or body fluids by several methods well known in the art (Théry C. et al., Curr Protoc Cell Biol. 2006 Apr;Chapter 3:Unit 3.22) (the entire contents of which are incorporated by reference herein). Several commercial kits are available for the isolation of exosomes such as ExoQuick™ or ExoTest™.

The first method of the present invention comprises in a second step comparing the MAF gene expression level obtained in the sample (*e.g*., tumor sample) from the subject with a reference value.

Once the MAF gene expression level in a sample from a subject with breast cancer, for example HER2+ breast cancer, have been measured and compared with the reference value, if the expression level of said gene is increased with respect to said reference value, then it can be concluded that said subject has a greater tendency to develop bone metastasis.

The determination of the MAF gene expression level must be correlated with the reference value.

In an embodiment, reference value(s) as intended herein may convey absolute quantities of MAF. In another embodiment, the quantity of any one or more biomarkers in a sample from a tested subject may be determined directly relative to the reference value (*e.g*., in terms of increase or decrease, or fold-increase or fold-decrease). Advantageously, this may allow to compare the quantity of any one or more biomarkers in the sample from the subject with the reference value (in other words to measure the relative quantity of any one or more biomarkers in the sample from the subject vis-a-vis the reference value) without the need to first determine the respective absolute quantities of said one or more biomarkers.

In a preferred embodiment, the reference value is the MAF gene expression level in a control sample or reference sample. Depending on the type of tumor to be analyzed, the exact nature of the control or reference sample may vary. Thus, in the event that a prognosis is to be evaluated, then the reference sample is a sample from a subject with HER2+ breast cancer, that has not metastasized or that corresponds to the median value of the MAF gene expression level measured in a tumor tissue collection in biopsy samples from subjects with HER2+ breast cancer, which have not metastasized.

Said reference sample is typically obtained by combining equal amounts of samples from a subject population. Generally, the typical reference samples will be obtained from subjects who are clinically well documented and in whom the absence of metastasis is well characterized. In such samples, the normal concentrations (reference concentration) of the biomarker (MAF gene) can be determined, for example by providing the mean concentration over the reference population. Various considerations are taken into account when determining the reference concentration of the marker. Among such considerations are the age, weight, sex, general physical condition of the patient and the like. For example, equal amounts of a group of at least about 2, at least about 10, at least about 100 to preferably more than about 1000 subjects, preferably classified according to the foregoing considerations, for example according to various age categories, are taken as the reference group. The sample collection from which the reference level is derived will preferably be formed by subjects suffering from the same type of cancer as the patient object of the study.

In a particular embodiment the reference values for "increased" or "reduced" expression of the MAF expression are determined by calculating the percentiles by conventional means which involves performing assays in one or several samples isolated from subjects whose disease is well documented by any of the methods mentioned above the MAF expression level. The "reduced" level of MAF can then preferably be assigned to samples wherein the MAF expression level is equal to or lower than about the 50^{th} percentile in the normal population including, for example, expression level equal to or lower than about the 60^{th} percentile in the normal population, equal to or lower than about the 70^{th} percentile in the normal population, equal to or lower than about the 80^{th} percentile in the normal population, equal to or lower than about the 90^{th} percentile in the normal population, and equal to or lower than about the 95^{th} percentile in the normal population. The "increased" MAF gene expression level can then preferably be assigned to samples wherein the MAF gene expression level is equal to or greater than about the 50^{th} percentile in the normal population including, for example, expression level equal to or greater than about the 60^{th} percentile in the normal population, equal to or greater than about the 70^{th} percentile in the normal population, equal to or greater than about the 80^{th} percentile in the normal population, equal to or greater than about the 90^{th} percentile in the normal population, and equal to or greater than about the 95^{th} percentile in the normal population.

The person skilled in the art will understand that the prediction of the tendency for a primary breast tumor to metastasize is not needed to be correct for all the subjects to be identified (*i.e.,* for 100% of the subjects). Nevertheless, the term requires enabling the identification of a statistically significant part of the subjects (for example, a cohort in a cohort study). Whether a part is statistically significant can be determined in a simple manner by the person skilled in the art using various well known statistical evaluation tools, for example, the determination of confidence intervals, determination of p values, Student's T test, Mann-Whitney test, etc. Details are provided in Dowdy and Wearden, Statistics for Research, John Wiley and Sons, New York 1983. The preferred confidence intervals are at least about 90%, at least about 95%, at least about 97%, at least about 98% or at least about 99%. The p values are preferably 0.1, 0.05, 0.01, 0.005 or 0.0001. More preferably, at least about 60%, at least about 70%, at least about 80% or at least about 90% of the subjects of a population can be suitably identified by the method of the present invention.

In yet another embodiment, the metastasis to bone is an osteolytic bone metastasis.

In yet another embodiment, an expression level of MAF which is above the average indicates increased risk of bone metastasis, being said risk is proportional to the levels of MAF expression, Thus, the risk of bone metastasis in a subject suffering breast cancer is dose-dependent.

### Method for predicting the clinical outcome of a patient suffering bone metastasis from HER2+ breast cancer, based on the expression level of MAF

In another aspect, the present invention relates to an *in vitro* method (hereinafter second method of the present invention) for predicting the clinical outcome of a patient suffering bone metastatic HER2+ breast cancer which comprises:
i) quantifying the expression level of the MAF gene in a sample of said subject and
ii) comparing the expression level obtained in step i) with a reference value,
wherein increased expression level of said gene with respect to said reference value is indicative of a poor clinical outcome.

The second method of the present invention comprises in a first step, quantifying the MAF gene expression level in a sample -of a subject suffering HER2+ breast cancer. In a preferred embodiment, the sample is a tumor tissue sample.

In a preferred embodiment, the second method of the present invention comprises quantifying only the MAF gene expression level as a single marker, *i.e.,* the method does not involve determining the expression level of any additional marker.

In a second step, the MAF gene expression level obtained in the tumor sample of the subject is compared with a reference value. In a preferred embodiment, the reference value is the expression level of said gene in a control sample. The determination of the MAF gene expression level must be correlated to values of a control sample or reference sample. Depending on the type of tumor to be analyzed, the exact nature of the control sample may vary. Thus, in the case involving the second method of the present invention, then the reference sample is a sample of subject with breast cancer who has not suffered bone metastasis or that corresponds to the median value of the MAF gene expression level measured in a tumor tissue collection in biopsy samples of subjects with breast cancer who have not suffered metastasis.

Once the MAF gene expression level in the sample is measured and compared with the control sample, if the expression level of said gene is increased with respect to its expression level in the control sample, then it is indicative of a poor clinical outcome.

In a specific embodiment, the bone metastasis is osteolytic metastasis.

In another specific embodiment, the quantification of the MAF gene expression level comprises quantifying the messenger RNA (mRNA) of said gene, or a fragment of said mRNA, the complementary DNA (cDNA) of said gene, or a fragment of said cDNA. In a more preferred embodiment, the expression level is quantified by means of a quantitative polymerase chain reaction (PCR) or a DNA or RNA array.

In another embodiment, the quantification of the MAF gene expression level comprises quantifying the level of protein encoded by said gene or of a variant thereof. In a yet more preferred embodiment, the protein level is determined by means of Western blot, immunohistochemistry, ELISA or a protein array.

In another embodiment, the reference sample is a tumor tissue sample of HER2+ breast cancer, from a subject who has not suffered metastasis.

Any parameter which is widely accepted for determining clinical outcome of a patient can be used in the present invention including, without limitation:
- disease-free progression which, as used herein, describes the proportion of subjects in complete remission who have had no recurrence of disease during the time period under study.
- disease-free survival (DFS), as used herewith, is understood as the length of time after treatment for a disease during which a subject survives with no sign of the disease.
- objective response which, as used in the present invention, describes the proportion of treated subjects in whom a complete or partial response is observed.
- tumour control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- Time to progression (TTP), as used herein, relates to the time after a disease is treated until the disease starts to get worse. The term "progression" has been previously defined.
- six-month progression free survival or "PFS6" rate which, as used herein, relates to the percentage of subjects who are free of progression in the first six months after the initiation of the therapy and
- median survival which, as used herein, relates to the time at which half of the subjects enrolled in the study are still alive.

The terms "poor" or "good", as used herein to refer to a clinical outcome, mean that the subject will show a favourable or unfavourable outcome. As will be understood by those skilled in the art, such the assessment of the probability, although preferred to be, may not be correct for about 100% of the subjects to be diagnosed. The term, however, requires that a statistically significant portion of subjects can be identified as having a predisposition for a given outcome. Whether a portion is statistically significant can be determined readily by the person skilled in the art using various well known statistic evaluation tools, *e.g*., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least about 50%, at least about 60%, at least about 70%, at least about 80%, at least about 90% at least about 95%. The p-values are, preferably, 0.05, 0.01, 0.005, or 0.0001 or less. More preferably, at least about 60 percent, at least about 70 percent, at least about 80 percent or at least about 90 percent of the subjects of a population can be properly identified by the method of the present invention.

### Method for designing customized therapy - in patients with HER2+ breast tumors

As is known in the state of the art, the treatment to be administered to a subject suffering from cancer depends on whether the latter is a malignant tumor, *i.e.,* whether it has high probabilities of undergoing metastasis, or whether the latter is a benign tumor. In the first assumption, the treatment of choice is a systemic treatment such as chemotherapy and in the second assumption, the treatment of choice is a localized treatment such as radiotherapy.

Therefore, as described in the present invention, given that MAF gene overexpression in HER2+ cancer cells is related to the presence of bone metastasis, the expression level of the MAF gene is useful for making decisions in terms of the most suitable therapy for the subject suffering said cancer.

Thus, in another aspect the present invention relates to an *in vitro* method (hereinafter third method of the present invention) for designing a customized therapy for a subject suffering HER2+ breast cancer, which comprises
i) quantifying the MAF gene expression level in a tumor sample of said subject (*e.g.,* a tumor tissue sample, circulating tumor cell, circulating tumor DNA or tumor derived exosomes) and
ii) comparing the expression level obtained in i) with a reference value,
wherein if the expression level is increased with respect to said reference value, then said subject is susceptible to receive a therapy aiming to prevent and/or treat the bone metastasis. If the expression level is not increased with respect to said reference value, then said subject is not susceptible to receive a therapy aiming to prevent and/or treat the bone metastasis. In some embodiments, the sample is a tumor derived sample, including: a tumor sample, a circulating tumor sample, circulating tumor DNA, or tumor-derived exosomes.

In a particular embodiment, the bone metastasis is osteolytic metastasis.

The third method of the present invention comprises in a first step quantifying the MAF gene expression level in a sample in a subject suffering from HER2+ breast cancer. In a preferred embodiment, the sample is a tumor tissue sample.

In another particular embodiment, the third method of the present invention comprises quantifying only the MAF gene expression level as a single marker, *i.e.,* the method does not involve determining the expression level of any additional marker.

In the case of the third method of the present invention the sample can be a primary tumor tissue sample of the subject.

In a second step, the MAF gene expression level obtained in the tumor sample of the subject is compared with a reference value. In a preferred embodiment, the reference value is the MAF gene expression level of said gene in a control sample. The determination of the MAF gene expression level must be related to values of a control sample or reference sample. Depending on the type of tumor to be analyzed, the exact nature of the control sample may vary. Thus preferably the reference sample is a sample of a subject with HER2+ breast cancer, that has not metastasized or that corresponds to the median value of the MAF gene expression level measured in a tumor tissue collection in biopsy samples of subjects with HER2+ breast cancer, which has not metastasized.

Once the MAF gene expression level in the sample has been measured and compared with the reference value, if the expression level of said gene is increased with respect to the reference value, then it can be concluded that said subject is susceptible to receiving therapy or not receiving therapy aiming to prevent (if the subject has yet to undergo metastasis) and/or treat metastasis or not prevent and/or treat metastasis (if the subject has already experienced metastasis).

When the cancer has metastasized, systemic treatments including but not limited to chemotherapy, hormone treatment, immunotherapy, or a combination thereof can be used. Additionally, radiotherapy and/or surgery can be used. The choice of treatment generally depends on the type of primary cancer, the size, the location of the metastasis, the age, the general health of the patient and the types of treatments used previously.

The systemic treatments are those that reach the entire body and could represent therapies therapy aiming to prevent or inhibit (if the subject has yet to undergo metastasis) and/or treat metastasis (if the subject has already experienced metastasis), such as:
- Chemotherapy is the use of medicaments to destroy cancer cells. The medicaments are generally administered through oral or intravenous route. Sometimes, chemotherapy is used together with radiation treatment. Suitable chemotherapeutic treatments for breast cancer include, without limitation, anthracyclines (doxorubicin, epirubicin, pegylated liposomal doxorubicin), Taxanes (paclitaxel, docetaxel, albumin nano-particle bound paclitaxel), 5-fluorouracil (continuous infusion 5-FU, capecitabine), Vinca alkaloids (vinorelbine, vinblastine), Gemcitabine, Platinum salts (cisplatin, carboplatin), cyclophosphamide, Etoposide and combinations of one or more of the above such as Cyclophosphamide/anthracycline +/- 5-fluorouracil regimens (such as doxorubicin/ cyclophosphamide (AC), epirubicin/cyclophosphamide, (EC) cyclophosphamide/epirubicin/5-fluorouracil (CEF), cyclophosphamide/doxorubicin/5-fluorouracil (CAF), 5-fluorouracil /epirubicin/cyclophosphamide (FEC)), cyclophosphamide/metothrexate/5-fluorouracil (CMF), anthracyclines/taxanes (such as doxorubicin/paclitaxel or doxorubicin/docetaxel), Docetaxel/capecitabine, Gemcitabine/paclitaxel, Taxane/platinum regimens (such as paclitaxel/carboplatin or docetaxel/carboplatin).
- Immunotherapy is a treatment that aids the immune system itself of the patient to combat cancer. There are several types of immunotherapy which are used to treat metastasis in patients. These include but are not limited to cytokines, monoclonal antibodies and antitumor vaccines.

In another aspect, the treatment is Alpharadin (radium-223 dichloride). Alpharadin uses alpha radiation from radium-223 decay to kill cancer cells. Radium-223 naturally self-targets to bone metastases by virtue of its properties as a calcium-mimic. Alpha radiation has a very short range of 2-10 cells (when compared to current radiation therapy which is based on beta or gamma radiation), and therefore causes less damage to surrounding healthy tissues (particularly bone marrow). With similar properties to calcium, radium-223 is drawn to places where calcium is used to build bone in the body, including the site of faster, abnormal bone growth - such as that seen in the skeletal metastases of men with advanced, castration-resistant prostate cancer. Radium-223, after injection, is carried in the bloodstream to sites of abnormal bone growth. The place where a cancer starts in the body is known as the primary tumor. Some of these cells may break away and be carried in the bloodstream to another part of the body. The cancer cells may then settle in that part of the body and form a new tumor. If this happens it is called a secondary cancer or a metastasis. Most patients with late stage prostate cancer suffer the maximum burden of disease in their bones. The aim with radium-223 is to selectively target this secondary cancer. Any radium-223 not taken-up in the bones is quickly routed to the gut and excreted.

In another aspect, the treatment is an mTor inhibitor. In some aspects, the mTor inhibitor is a dual mTor/PI3kinase inhibitor. In some aspects, the mTor inhibitor is used to prevent or inhibit metastasis. In some aspects the mTor inhibitor is selected from the group consisting of: ABI009 (sirolimus), rapamycin (sirolimus), Abraxane (paclitaxel), Absorb (everolimus), Afinitor (everolimus), Afinitor with Gleevec, AS703026 (pimasertib), Axxess (umirolimus), AZD2014, BEZ235, Biofreedom (umirolimus), BioMatrix (umirolimus), BioMatrix flex (umirolimus), CC115, CC223, Combo Bioengineered Sirolimus Eluting Stent ORBUSNEICH (sirolimus), Curaxin CBLC102 (mepacrine), DE109 (sirolimus), DS3078, Endeavor DES (zotarolimus), Endeavor Resolute (zotarolimus), Femara (letrozole), Hocena (antroquinonol), INK128, Inspiron (sirolimus), IPI504 (retaspimycin hydrochloride), KRN951 (tivozanib), ME344, MGA031 (teplizumab), MiStent SES (sirolimus), MKC1, Nobori (umirolimus), OSI027, OVI123 (cordycepin), Palomid 529, PF04691502, Promus Element (everolimus), PWT33597, Rapamune (sirolimus), Resolute DES (zotarolimus), RG7422, SAR245409, SF1126, SGN75 (vorsetuzumab mafodotin), Synergy (everolimus), Taltorvic (ridaforolimus), Tarceva (erlotinib), Torisel (temsirolimus), Xience Prime (everolimus), Xience V (everolimus), Zomaxx (zotarolimus), Zortress (everolimus), Zotarolimus Eluting Peripheral Stent MEDTRONIC (zotarolimus), AP23841, AP24170, ARmTOR26, BN107, BN108, Canstatin GENZYME (canstatin), CU906, EC0371, EC0565, KI1004, LOR220, NV128, Rapamycin ONCOIMMUNE (sirolimus), SB2602, Sirolimus PNP SAMYANG BIOPHARMACEUTICALS (sirolimus), TOP216, VLI27, VS5584, WYE125132, XL388, Advacan (everolimus), AZD8055, Cypher Select Plus Sirolimus eluting Coronary Stent (sirolimus), Cypher Sirolimus eluting coronary stent (sirolimus), Drug Coated Balloon (sirolimus), E-Magic Plus (sirolimus), Emtor (sirolimus), Esprit (everolimus), Evertor (everolimus), HBF0079, LCP-Siro (sirolimus), Limus CLARIS (sirolimus), mTOR Inhibitor CELLZOME, Nevo Sirolimus eluting Coronary Stent (sirolimus), nPT-mTOR, Rapacan (sirolimus), Renacept (sirolimus), ReZolve (sirolimus), Rocas (sirolimus), SF1126, Sirolim (sirolimus), Sirolimus NORTH CHINA (sirolimus), Sirolimus RANBAXY (sirolimus), Sirolimus WATSON (sirolimus) Siropan (sirolimus), Sirova (sirolimus), Supralimus (sirolimus), Supralimus-Core (sirolimus), Tacrolimus WATSON (tacrolimus), TAFA93, Temsirolimus ACCORD (temsirolimus), Temsirolimus SANDOZ (temsirolimus), TOP216, Xience Prime (everolimus), Xience V (everolimus). In a specific aspect the mTor inhibitor is Afinitor (everolimus) (http://www.afinitor.com/index.jsp?usertrack.filter_applied=true&NovaId=40294620643 38207963; last accessed 11/28/2012). In another aspect, everolimus is combined with an aromatase inhibitor. (*See. e.g.,* Baselga, J., el al., Everolimus in Postmenopausal Hormone-Receptor Positive Advanced Breast Cancer. 2012. N. Engl. J. Med. 366(6): 520-529, which is herein incorporated by reference). In another aspect, mTor inhibitors can be identified through methods known in the art. (*See, e.g.,* Zhou, H. et al. Updates of mTor inhibitors. 2010. Anticancer Agents Med. Chem. 10(7): 571-81, which is herein incorporated by reference). In some aspects, the mTor inhibitor is used to treat or prevent or inhibit metastasis in a patient that is positive for a hormone receptor. (*See. e.g.,* Baselga, J., el al., Everolimus in Postmenopausal Hormone-Receptor Positive Advanced Breast Cancer. 2012. N. Engl. J. Med. 366(6): 520-529). In some embodiments, the patient is ER+. In some aspects, the mTor inhibitor is used to treat or prevent or inhibit metastasis in a patient with advanced breast cancer. In some aspects, the mTor inhibitor is used in combination with a second treatment. In some aspects, the second treatment is any treatment described herein.

In another aspect, the treatment is a Src kinase inhibitor. In some aspects, the Src inhibitor is used to prevent or inhibit metastasis. In some aspects, the Src kinase inhibitor is selected from the group: AZD0530 (saracatinib), Bosulif (bosutinib), ENMD981693, KD020, KX01, Sprycel (dasatinib), Yervoy (ipilimumab), AP23464, AP23485, AP23588, AZD0424, c-Src Kinase Inhibitor KISSEI, CU201, KX2361, SKS927, SRN004, SUNK706, TG100435, TG100948, AP23451, Dasatinib HETERO (dasatinib), Dasatinib VALEANT (dasatinib), Fontrax (dasatinib), Src Kinase Inhibitor KINEX, VX680,(tozasertib lactate), XL228, and SUNK706. In some embodiments, the Src kinase inhibitor is dasatinib. In another aspect, Src kinase inhibitors can be identified through methods known in the art (*See, e.g.,* Sen, B. and Johnson, F.M. Regulation of Src Family Kinases in Human Cancers. 2011. J. Signal Transduction. 2011: 14 pages, which is herein incorporated by reference). In some aspects, the Src kinase inhibitor is used to treat or prevent or inhibit metastasis in a patient that is positive for the SRC-responsive signature (SRS). In some aspects, the patient is SRS+ and ER-. (*See. e.g*.,Zhang, CH.-F, et al. Latent Bone Metastasis in Breast Cancer Tied to Src-Dependent survival signals. 2009. Cancer Cell. 16: 67-78, which is herein incorporated by reference.) In some aspects, the Src kinase inhibitor is used to treat or prevent or inhibit metastasis in a patient with advanced breast cancer. In some aspects, the Src kinase inhibitor is used in combination with a second treatment. In some aspects, the second treatment is any treatment described herein.

In another aspect, the treatment is a COX-2 inhibitor. In some aspects, the COX-2 inhibitor is used to prevent or inhibit metastasis. In some aspects, the COX-2 inhibitor is selected from the group: ABT963, Acetaminophen ER JOHNSON (acetaminophen), Acular X (ketorolac tromethamine), BAY1019036 (aspirin), BAY987111 (diphenhydramine, naproxen sodium), BAY11902 (piroxicam), BCIBUCH001 (ibuprofen), Capoxigem (apricoxib), CS502, CS670 (pelubiprofen), Diclofenac HPBCD (diclofenac), Diractin (ketoprofen), GW406381, HCT1026 (nitroflurbiprofen), Hyanalgese-D (diclofenac), HydrocoDex (acetaminophen, dextromethorphan, hydrocodone), Ibuprofen Sodium PFIZER (ibuprofen sodium), Ibuprofen with Acetaminophen PFIZER (acetaminophen, ibuprofen), Impracor (ketoprofen), IP880 (diclofenac), IP940 (indomethacin), ISV205 (diclofenac sodium), JNS013 (acetaminophen, tramadol hydrochloride), Ketoprofen TDS (ketoprofen), LTNS001 (naproxen etemesil), Mesalamine SALIX (mesalamine), Mesalamine SOFAR (mesalamine), Mesalazine (mesalamine), ML3000 (licofelone), MRX7EAT (etodolac), Naproxen IROKO (naproxen), NCX4016 (nitroaspirin), NCX701 (nitroacetaminophen), Nuprin SCOLR (ibuprofen), OMS103HP (amitriptyline hydrochloride, ketoprofen, oxymetazoline hydrochloride), Oralease (diclofenac), OxycoDex (dextromethorphan, oxycodone), P54, PercoDex (acetaminophen, dextromethorphan, oxycodone), PL3100 (naproxen, phosphatidyl choline), PSD508, R-Ketoprofen (ketoprofen), Remura (bromfenac sodium), ROX828 (ketorolac tromethamine), RP19583 (ketoprofen lysine), RQ00317076, SDX101 (R-etodolac), TDS943 (diclofenac sodium), TDT070 (ketoprofen), TPR100, TQ1011 (ketoprofen), TT063 (S-flurbiprofen), UR8880 (cimicoxib), V0498TA01A (ibuprofen), VT122 (etodolac, propranolol), XP20B (acetaminophen, dextropropoxyphene), XP21B (diclofenac potassium), XP21L (diclofenac potassium), Zoenasa (acetylcysteine, mesalamine), Acephen, Actifed Plus, Actifed-P, Acular, Acular LS, Acular PF, Acular X, Acuvail, Advil, Advil Allergy Sinus ,Advil Cold and Sinus ,Advil Congestion Relief ,Advil PM, Advil PM Capsule, Air Salonpas, Airtal, Alcohol-Free NyQuil Cold & Flu Relief, Aleve ,Aleve ABDI IBRAHIM ,Aleve-D, Alka-Seltzer ,Alka-Seltzer BAYER, Alka-Seltzer Extra Strength, Alka-Seltzer Lemon-Lime, Alka-Seltzer Original, Alka-Seltzer Plus, Alka-Seltzer plus Cold and Cough, Alka-Seltzer plus Cold and Cough Formula, Alka-Seltzer Plus Day and Night Cold Formula,, Alka-Seltzer Plus Day Non-Drowsy Cold Formula, Alka-Seltzer Plus Flu Formula, Alka-Seltzer Plus Night Cold Formula, Alka-Seltzer Plus Sinus Formula, Alka-Seltzer Plus Sparkling Original Cold Formula, Alka-Seltzer PM, Alka-Seltzer Wake-Up Call, Anacin, Anaprox, Anaprox MINERVA, Ansaid, Apitoxin, Apranax, Apranax abdi, Arcoxia, Arthritis Formula Bengay, Arthrotec, Asacol, Asacol HD, Asacol MEDUNA ARZNEIMITTEL, Asacol ORIFARM, Aspirin BAYER, Aspirin Complex, Aspirin Migran, AZD3582, Azulfidine, Baralgan M, BAY1019036, BAY987111, BAY11902, BCIBUCH001, Benadryl Allergy, Benadryl Day and Night, Benylin 4 Flu, Benylin Cold and Flu, Benylin Cold and Flu Day and Night, Benylin Cold and Sinus Day and Night, Benylin Cold and Sinus Plus, Benylin Day and Night Cold and Flu Relief, Benylin1 All-In-One, Brexin, Brexin ANGELINI, Bromday, Bufferin, Buscopan Plus, Caldolor, Calmatel, Cambia, Canasa, Capoxigem, Cataflam, Celebrex, Celebrex ORIFARM, Children's Advil Allergy Sinus, Children's Tylenol, Children's Tylenol Cough and Runny Nose, Children's Tylenol plus cold, Children's Tylenol plus Cold and Cough, Children's Tylenol plus cold and stuffy nose, Children's Tylenol plus Flu, Children's Tylenol plus cold & allergy, Children's Tylenol plus Cough & Runny Nose, Children's Tylenol plus Cough & Sore Throat, Children's Tylenol plus multi symptom cold, Clinoril, Codral Cold and Flu, Codral Day and Night Day Tablets, Codral Day and Night Night Tablets, Codral Nightime, Colazal, Combunox, Contac Cold plus Flu, Contac Cold plus Flu Non-Drowsy, Coricidin D, Coricidin HBP Cold and Flu, Coricidin HBP Day and Night Multi-Symptom Cold, Coricidin HBP Maximum Strength Flu, Coricidin HBP Nighttime Multi-Symptom Cold, Coricidin II Extra Strength Cold and Flu, CS502, CS670, Daypro, Daypro Alta, DDS06C, Demazin Cold and Flu, Demazin Cough, Cold and Flu, Demazin day/night Cold and Flu, Demazin PE Cold and Flu, Demazin PE day/night Cold and Flu, Diclofenac HPBCD, Dimetapp Day Relief, Dimetapp Multi-Symptom Cold and Flu, Dimetapp Night Relief, Dimetapp Pain and Fever Relief, Dimetapp PE Sinus Pain, Dimetapp PE Sinus Pain plus Allergy, Dipentum, Diractin, Disprin Cold 'n' Fever, Disprin Extra, Disprin Forte. Disprin Plus, Dristan Cold, Dristan Junior, Drixoral Plus, Duexis, Dynastat, Efferalgan, Efferalgan Plus Vitamin C, Efferalgan Vitamin C, Elixsure IB, Excedrin Back and Body, Excedrin Migraine, Excedrin PM, Excedrin Sinus Headache, Excedrin Tension Headache, Falcol, Fansamac, Feldene, FeverAll, Fiorinal, Fiorinal with Codeine, Flanax, Flector Patch, Flucam, Fortagesic, Gerbin, Giazo, Gladio, Goody's Back and Body Pain, Goody's Cool Orange, Goody's Extra Strength, Goody's PM, Greaseless Bengay, GW406381, HCT1026, He Xing Yi, Hyanalgese-D, HydrocoDex, Ibuprofen Sodium PFIZER, Ibuprofen with, Acetaminophen PFIZER, Icy Hot SANOFI AVENTIS, Impracor, Indocin, Indomethacin APP PHARMA, Indomethacin MYLAN, Infants' Tylenol, IP880, IP940, Iremod, ISV205, JNS013, Jr. Tylenol, Junifen, Junior Strength Advil, Junior Strength Motrin, Ketoprofen TDS, Lemsip Max, Lemsip Max All in One, Lemsip Max All Night, Lemsip Max Cold and Flu, Lialda, Listerine Mouth Wash, Lloyds Cream, Lodine, Lorfit P, Loxonin, LTNS001, Mersyndol, Mesalamine SALIX, Mesalamine SOFAR, Mesalazine, Mesasal GLAXO, Mesasal SANOFI, Mesulid, Metsal Heat Rub, Midol Complete, Midol Extended Relief, Midol Liquid Gels, Midol PM, Midol Teen Formula, Migranin COATED TABLETS, ML3000, Mobic, Mohrus, Motrin, Motrin Cold and Sinus Pain, Motrin PM, Movalis ASPEN, MRX7EAT, Nalfon, Nalfon PEDINOL, Naprelan, Naprosyn, Naprosyn RPG LIFE SCIENCE, Naproxen IROKO, NCX4016, NCX701, NeoProfen LUNDBECK, Nevanac, Nexcede, Niflan, Norgesic MEDICIS, Novalgin, Nuprin SCOLR, Nurofen, Nurofen Cold and Flu, Nurofen Max Strength Migraine, Nurofen Plus, Nuromol, NyQuil with Vitamin C, Ocufen, OMS103HP, Oralease, Orudis ABBOTT JAPAN, Oruvail, Osteluc, OxycoDex, P54, Panadol, Panadol Actifast, Paradine, Paramax, Parfenac, Pedea, Pennsaid, Pentasa, Pentasa ORIFARM, Peon, Percodan, Percodan-Demi, PercoDex, Percogesic, Perfalgan, PL2200, PL3100, Ponstel, Prexige, Prolensa, PSD508, R-Ketoprofen, Rantudil, Relafen, Remura, Robaxisal, Rotec, Rowasa, ROX828, RP19583, RQ00317076, Rubor, Salofalk, Salonpas, Saridon, SDX101, Seltouch, sfRowasa, Shinbaro, Sinumax, Sinutab, Sinutab,, sinus, Spalt, Sprix, Strefen, Sudafed Cold and Cough, Sudafed Head Cold and Sinus, Sudafed PE Cold plus Cough, Sudafed PE Pressure plus Pain, Sudafed PE, Severe Cold, Sudafed PE Sinus Day plus Night Relief Day Tablets, Sudafed PE Sinus Day plus Night Relief Night Tablets, Sudafed PE Sinus plus Anti-inflammatory Pain Relief, Sudafed Sinus Advance, Surgam, Synalgos-DC, Synflex, Tavist allergy/sinus/headache, TDS943, TDT070, Theraflu Cold and Sore Throat, Theraflu Daytime Severe Cold and Cough, Theraflu Daytime Warming Relief,Theraflu Warming Relief Caplets Daytime Multi-Symptom Cold, Theraflu Warming Relief Cold and Chest Congestion, Thomapyrin, Thomapyrin C, Thomapyrin Effervescent, Thomapyrin Medium, Tilcotil, Tispol, Tolectin, Toradol, TPR100, TQ1011, Trauma-Salbe, Trauma-Salbe Kwizda, Treo, Treximet, Trovex, TT063, Tylenol, Tylenol Allergy Multi-Symptom, Tylenol Back Pain, Tylenol Cold & Cough Daytime, Tylenol Cold & Cough Nighttime, Tylenol Cold and Sinus Daytime, Tylenol Cold and Sinus Nighttime, Tylenol Cold Head Congestion Severe, Tylenol Cold Multi Symptom Daytime, Tylenol Cold Multi Symptom Nighttime Liquid, Tylenol Cold Multi Symptom Severe, Tylenol Cold Non-Drowsiness Formula, Tylenol Cold Severe Congestion Daytime, Tylenol Complete Cold, Cough and Flu Night time, Tylenol Flu Nighttime, Tylenol Menstrual, Tylenol PM, Tylenol Sinus Congestion & Pain Daytime, Tylenol Sinus Congestion & Pain Nighttime, Tylenol Sinus Congestion & Pain Severe, Tylenol Sinus Severe Congestion Daytime, Tylenol Ultra Relief, Tylenol with Caffeine and Codeine phosphate, Tylenol with Codeine phosphate, Ultra Strength Bengay Cream, Ultracet, UR8880, V0498TA01A, Vicks NyQuil Cold and Flu Relief, Vicoprofen, Vimovo, Voltaren Emulgel, Voltaren GEL, Voltaren NOVARTIS CONSUMER HEALTH GMBH, Voltaren XR, VT122, Xefo, Xefo Rapid, Xefocam, Xibrom, XL3, Xodol, XP20B, XP21B, XP21L, Zipsor, and Zoenasa. In another aspect, COX-2 inhibitors can be identified through methods known in the art (*See, e.g.,* Dannhardt, G. and Kiefer, W. Cyclooxygenase inhibitors- current status and future prospects. 2001. Eur. J. Med. Chem. 36: 109-126, which is herein incorporated by reference). In some aspects, the COX-2 inhibitor is used to treat or prevent or inhibit metastasis in a patient with advanced breast cancer. In some aspects, the COX-2 inhibitor is used in combination with a second treatment. In some aspects, the second treatment is any treatment described herein. In some aspects, the COX-2 inhibitor is used in combination with a second treatment selected from the group consisting of: Denosumab, Zometa (http://www.us.zometa.com/index.jsp?usertrack.filter_applied=true&NovaId=293537693 4467633633; last accessed 12/2/2012), Carbozantinib or Cabozantinib, Antibody or peptide blocking PTHLH (parathyroid hormone like hormone) or PTHrP (parathyroid hormone related protein) and Everolimus.

In another aspect, the treatment agents used for avoiding and/or preventing bone degradation include, but are not limited to:
- Parathyroid hormone (PTH) and Parathyroid like hormone (PTHLH) inhibitors (including blocking antibodies) or recombinant forms thereof (teriparatide corresponding to the amino acids 7-34 of PTH). This hormone acts by stimulating the osteoclasts and increasing their activity.
- Strontium ranelate: is an alternative oral treatment, and forms part of the group of drugs called "dual action bone agents" (DABAs) because they stimulate the osteoblast proliferation and inhibit the osteoclast proliferation.
- "Estrogen receptor modulators" (SERM) refers to compounds which interfere or inhibit the binding of estrogens to the receptor, regardless of the mechanism. Examples of estrogen receptor modulators include, among others, estrogens progestagen, estradiol, droloxifene, raloxifene, lasofoxifene, TSE-424, tamoxifen, idoxifene, L Y353381, LY117081, toremifene, fluvestrant, 4-[7-(2,2-dimethyl-1-oxopropoxy-4-methyl-2-[4-[2-(1-piperidinyl)ethoxy]phenyl]-2H-1-benzopyran-3-yl]-phenyl-2,2-dimethylpropanoate 4,4'dihydroxybenzophenone-2,4-dinitrophenyl-hydrazone and SH646.
- Calcitonin: directly inhibits the osteoclast activity through the calcitonin receptor. The calcitonin receptors have been identified on the surface of the osteoclasts.
- Bisphosphonates: are a group of medicinal products used for the prevention and the treatment of diseases with bone resorption and reabsorption such as osteoporosis and cancer with bone metastasis, the latter being with or without hypercalcaemia, associated to breast cancer and prostate cancer. Examples of bisphosphonates which can be used in the therapy designed by means of the fifth method of the present invention include, although not limited to, nitrogenous bisphosphonates (such as pamidronate, neridronate, olpadronate, alendronate, ibandronate, risedronate, incadronate, zoledronate or zoledronic acid, etc.) and non-nitrogenous bisphosphonates (such as etidronate, clodronate, tiludronate, etc.).
- "Cathepsin K inhibitors" refers to compounds which interfere in the cathepsin K cysteine protease activity. Non-limiting examples of cathepsin K inhibitors include 4-amino-pyrimidine-2-carbonitrile derivatives (described in the International patent application WO 03/020278 under the name of Novartis Pharma GMBH), pyrrolo-pyrimidines described in the publication WO 03/020721 (Novartis Pharma GMBH) and the publication WO 04/000843 (ASTRAZENECA AB) as well as the inhibitors described in the publications PCT WO 00/55126 of Axys Pharmaceuticals, WO 01/49288 of Merck Frosst Canada & Co. and Axys Pharmaceuticals.
- "DKK-1(Dickkopf-1) inhibitor" as used herein refers to any compound which is capable of reducing DKK-1 activity. DKK-1 is a soluble Wnt pathway antagonist expressed predominantly in adult bone and upregulated in myeloma patients with osteolytic lesions. Agents targeting DKK-1 may play a role in preventing osteolytic bone disease in multiple myeloma patients. BHQ880 from Novartis is a first-in-class, fully human, anti-DKK-1 neutralizing antibody. Preclinical studies support the hypothesis that BHQ880 promotes bone formation and thereby inhibits tumor-induced osteolytic disease (Ettenberg S. et al., American Association for Cancer Research Annual Meeting. April 12-16, 2008; San Diego, Calif. Abstract).
- "Dual MET and VEGFR2 inhibitor" as used herein refers to any compound which is a potent dual inhibitor of the MET and VEGF pathways designed to block MET driven tumor escape. MET is expressed not only in tumor cells and endothelial cells, but also in osteoblasts (bone-forming cells) and osteoclasts (bone-removing cells). HGF binds to MET on all of these cell types, giving the MET pathway an important role in multiple autocrine and paracrine loops. Activation of MET in tumor cells appears to be important in the establishment of metastatic bone lesions. At the same time, activation of the MET pathway in osteoblasts and osteoclasts may lead to pathological features of bone metastases, including abnormal bone growth (ie, blastic lesions) or destruction (ie, lytic lesion. Thus, targeting the MET pathway may be a viable strategy in preventing the establishment and progression of metastatic bone lesions. Cabozantinib (Exelixis, Inc), formerly known as XL184 (CAS 849217-68-1), is a potent dual inhibitor of the MET and VEGF pathways designed to block MET driven tumor escape. In multiple preclinical studies cabozantinib has been shown to kill tumor cells, reduce metastases, and inhibit angiogenesis (the formation of new blood vessels necessary to support tumor growth). Another suitable dual inhibitors are E7050 (N-[2-Fluoro-4-({2-[4-(4-methylpiperazin-1-yl)piperidin-1-yl] carbonylaminopyridin-4-yl}oxy)phenyl]-N'-(4-fluorophenyl)cyclopropane-1,1-dicarboxamide (2R,3R)-tartrate) (CAS 928037-13-2) or Foretinib (also known as GSK1363089, XL880, CAS 849217-64-7).
- "RANKL inhibitors" as used herein refer to any compound which is capable of reducing the RANK activity. RANKL is found on the surface of the osteoblast membrane of the stroma and T-lymphocyte cells, and these T-lymphocyte cells are the only ones which have demonstrated the capacity for secreting it. Its main function is the activation of the osteoclasts, cells involved in the bone resorption. The RANKL inhibitors can act by blocking the binding of RANKL to its receptor (RANK), blocking the RANK-mediated signaling or reducing the expression of RANKL by blocking the transcription or the translation of RANKL. RANKL antagonists or inhibitors suitable for use in the present invention include, without limitation:
   ∘ a suitable RANK protein which is capable of binding RANKL and which comprises the entire or a fragment of the extracellular domain of a RANK protein. The soluble RANK may comprise the signal peptide and the extracellular domain of the murine or human RANK polypeptides, or alternatively, the mature form of the protein with the signal peptide removed can be used.
   ∘ Osteoprotegerin or a variant thereof with RANKL-binding capacity.
   ∘ RANKL-specific antisense molecules
   ∘ Ribozymes capable of processing the transcribed products of RANKL
   ∘ Specific anti-RANKL antibodies. "Anti-RANKL antibody or antibody directed against RANKL" is understood herein as all that antibody which is capable of binding specifically to the ligand of the activating receptor for the nuclear factor κB (RANKL) inhibiting one or more RANKL functions. The antibodies can be prepared using any of the methods which are known by the person skilled in the art. Thus, the polyclonal antibodies are prepared by means of immunizing an animal with the protein to be inhibited. The monoclonal antibodies are prepared using the method described by Kohler, Milstein et al. (Nature, 1975, 256: 495). Antibodies suitable in the context of the present invention include intact antibodies which comprise a variable antigen binding region and a constant region, fragments "Fab", "F(ab')2" and "Fab"', Fv, scFv, diabodies and bispecific antibodies.
   ∘ Specific anti-RANKL nanobodies. Nanobodies are antibody-derived therapeutic proteins that contain the unique structural and functional properties of naturally-occurring heavy-chain antibodies. The Nanobody technology was originally developed following the discovery that camelidae (camels and llamas) possess fully functional antibodies that lack light chains. The general structure of nanobodies is FR1-CDR1-FR2-CDR2-FR3-CDR3-FR4
wherein FR1 to FR4 are the framework regions 1 to 4 CDR1 to CDR3 are the complementarity determining regions 1 to 3. These heavy-chain antibodies contain a single variable domain (VHH) and two constant domains (CH2 and CH3). Importantly, the cloned and isolated VHH domain is a perfectly stable polypeptide harbouring the full antigen-binding capacity of the original heavy-chain antibody. These newly discovered VHH domains with their unique structural and functional properties form the basis of a new generation of therapeutic antibodies which Ablynx has named Nanobodies.

In one embodiment, the RANKL inhibitor is selected from the group consisting of a RANKL specific antibody, a RANKL specific nanobody and osteoprotegerin. In a specific embodiment, the anti-RANKL antibody is a monoclonal antibody. In a yet more specific embodiment, the anti-RANKL antibody is Denosumab (Pageau, Steven C. (2009). mAbs 1 (3): 210-215, CAS number 615258-40-7) (the entire contents of which are hereby incorporated by reference). Denosumab is a fully human monoclonal antibody which binds to RANKL and prevents its activation (it does not bind to the RANK receptor). Various aspects of Denosumab are covered by U.S. Pat. Nos. 6,740,522; 7,411,050; 7,097,834; 7,364,736 (the entire contents of each of which are hereby incorporated by reference in their entirety). In another embodiment, the RANKL inhibitor an antibody, antibody fragment, or fusion construct that binds the same epitope as Denosumab.

In a preferred embodiment, the anti-RANKL nanobody is any of the nanobodies as described in WO2008142164, (the contents of which are incorporated in the present application by reference). In a still more preferred embodiment, the anti-RANKL antibody is the ALX-0141 (Ablynx). ALX-0141 has been designed to inhibit bone loss associated with post-menopausal osteoporosis, reumatoid arthritis, cancer and certain medications, and to restore the balance of healthy bone metabolism.

In a preferred embodiment, the agent preventing the bone degradation is selected from the group consisting of a bisphosphonate, a RANKL inhibitor, PTH and PTHLH inhibitor or a PRG analog, strontium ranelate, a DKK-1 inhibitor, a dual MET and VEGFR2 inhibitor, an estrogen receptor modulator, Radium-223 calcitonin, and a cathepsin K inhibitor. In a more preferred embodiment the agent preventing the bone degradation is a bisphosphonate. In a yet more preferred embodiment, the bisphosphonate is the zoledronic acid.

In one embodiment, a CCR5 antagonist is administered to prevent or inhibit metastasis of the primary breast cancer tumor to bone. In one embodiment, the CCR5 antagonist is a large molecule. In another embodiment, the CCR5 antagonist is a small molecule. In some embodiments, the CCR5 antagonist is Maraviroc (Velasco-Veláquez, M. et al. 2012. CCR5 Antagonist Blocks Metastasis of Basal Breast Cancer Cells. Cancer Research. 72:3839-3850.). In some embodiments, the CCR5 antagonist is Vicriviroc. Velasco-Veláquez, M. et al. 2012. CCR5 Antagonist Blocks Metastasis of Basal Breast Cancer Cells. Cancer Research. 72:3839-3850.). In some aspects, the CCR5 antagonist is Aplaviroc (Demarest J.F. et al. 2005. Update on Aplaviroc: An HIV Entry Inhibitor Targeting CCR5. Retrovirology 2(Suppl. 1): S13). In some aspects, the CCR5 antagonist is a spiropiperidine CCR5 antagonist. (Rotstein D.M. et al. 2009. Spiropiperidine CCR5 antagonists. Bioorganic & Medicinal Chemistry Letters. 19 (18): 5401-5406. In some embodiments, the CCR5 antagonist is INCB009471 (Kuritzkes, D.R. 2009. HIV-1 entry inhibitors: an overview. Curr. Opin. HIV AIDS. 4(2): 82-7).

In a preferred embodiment the dual MET and VEGFR2 inhibitor is selected from the group consisting of Cabozantinib, Foretinib and E7050.

In a preferred embodiment the Radium 223 therapy is alpharadin.

Alternatively a combined treatment can be carried out in which more than one agent from those mentioned above are combined to treat and/or prevent the metastasis or said agents can be combined with other supplements, such as calcium or vitamin D or with a hormone treatment.

### Method for predicting early bone metastasis in breast cancer patients.

In another aspect, the present invention relates to an *in vitro* method for determining the risk of bone metastasis in a subject suffering breast cancer, such as HER2+ breast cancer, which comprises determining the expression level of the MAF gene in a sample of said subject wherein an expression level of said gene above the average value plus one standard deviation is indicative of an increased risk of early bone metastasis.

In a preferred embodiment, the bone metastasis is very early bone metastasis.

In a preferred embodiment, the bone metastasis is osteolytic metastasis.

"Early bone metastasis" as used herein, relates to a bone metastasis that appears before 5 years post surgery in a patient with breast cancer.

"Very early bone metastasis" as used herein, relates to a bone metastasis that appears before 3 years post surgery in a patient with breast cancer.

The fourth method of the present invention comprises in a first step, quantifying the MAF gene expression level in a sample of a subject suffering breast cancer, such as HER2+ breast cancer. In a preferred embodiment, the sample is a tumor tissue sample.

In a preferred embodiment, the fourth method of the present invention comprises quantifying only the MAF gene expression level as a single marker, *i.e.,* the method does not involve determining the expression level of any additional marker. The MAF gene expression level can be quantified as previously disclosed for the first method of the present invention.

In a preferred embodiment, the breast cancer is HER2+ breast cancer,.

In a second step, an expression level of said gene above the average value plus one standard deviation is indicative of an increased risk of early bone metastasis..

"Average level" as used herein relates to a single value of MAF expression level (as a mean, mode, or median) that summarizes or represents the general significance of a set of unequal values. In a preferred embodiment the average level corresponds to the average of expression levels obtained from a representative cohort of breast cancer tumors. The patient cohort is defined by age that is representative of the individual patient that one is attempting to evaluate.

"Standard deviation" as used herein relates to a measure of the dispersion of a collection of numbers. For example, the standard deviation for the average normal level of MAF is the dispersion of a collection of the MAF levels found in breast tumor samples The more spread apart the data, the higher the deviation. Standard deviation can be obtained by extracting the square root of the mean of squared deviations of observed values from their mean in a frequency distribution.

Once the MAF gene expression level in a sample from a subject with breast cancer, such as HER2+ breast cancer, has been measured and compared with the average level, if the expression level of said gene is above the average plus one standard deviation with respect to the average level, then it can be concluded that said subject has a greater tendency to develop early bone metastasis.

### Method for designing customized therapy in HER2+ breast cancer patients with bone metastasis

In another aspect, the present invention relates to an *in vitro* method for designing a customized therapy for a subject with HER2+ breast cancer with bone metastasis (hereinafter fifth method of the present invention) which comprises
i) quantifying the MAF gene expression level in a bone metastatic sample of said subject and
ii) comparing the expression level obtained in step (i) with a reference value,
wherein if the MAF gene expression level is increased with respect to said reference value, then said subject is susceptible to receive a therapy aiming to prevent the bone degradation.

In a preferred embodiment, the bone metastasis is osteolytic metastasis.

The fifth method of the present invention comprises in a first step, quantifying the MAF gene expression level (or MAF translocation or amplification) in a sample in a subject suffering HER2+ breast cancer. In the case of the fifth method of the present invention, the sample can be a tissue sample from bone metastasis.

In a preferred embodiment, the fifth method of the present invention comprises quantifying only the MAF gene expression level as a single marker, *i.e.,* the method does not involve determining the expression level of any additional marker.

In a second step the MAF gene expression level (or MAF translocation or amplification) obtained in the tumor sample of the subject is compared with the reference value. In a preferred embodiment, the reference value is the MAF gene expression level in a control sample. Depending on the type of tumor to be analyzed, the exact nature of the control sample may vary. Thus, in the case involving the fifth method of the present invention, then the reference sample is a sample of a subject with HER2+ breast cancer who has not suffered metastasis or that corresponds to the median value of the MAF gene expression level measured in a tumor tissue collection in biopsy samples of subjects with HER2+ breast cancer who have not suffered metastasis.

Once the MAF gene expression level in the sample is measured and compared with the reference value (*e.g.* the MAF gene expression level of a control sample), if the expression level of said gene is increased with respect to the reference value, then this is indicative that said subject is susceptible to receive a therapy aiming to avoid or prevent bone degradation.

Illustrative examples of agents used for avoiding and/or preventing bone degradation include, although not limited to:
- Parathyroid hormone (PTH) and Parathyroid like hormone (PTHLH) inhibitors (including blocking antibodies) or recombinant forms thereof (teriparatide corresponding to the amino acids 7-34 of PTH). This hormone acts by stimulating the osteoclasts and increasing their activity.
- Strontium ranelate: is an alternative oral treatment, and forms part of the group of drugs called "dual action bone agents" (DABAs) because they stimulate the osteoblast proliferation and inhibit the osteoclast proliferation.
- "Estrogen receptor modulators" (SERM) refers to compounds which interfere or inhibit the binding of estrogens to the receptor, regardless of the mechanism. Examples of estrogen receptor modulators include, among others, estrogens progestagen, estradiol, droloxifene, raloxifene, lasofoxifene, TSE-424, tamoxifen, idoxifene, L Y353381, LY117081, toremifene, fluvestrant, 4-[7-(2,2-dimethyl-1-oxopropoxy-4-methyl-2-[4-[2-(1-piperidinyl)ethoxy]phenyl]-2H-1-benzopyran-3-yl]-phenyl-2,2-dimethylpropanoate 4,4'dihydroxybenzophenone-2,4-dinitrophenyl-hydrazone and SH646.
- Calcitonin: directly inhibits the osteoclast activity through the calcitonin receptor. The calcitonin receptors have been identified on the surface of the osteoclasts.
- Bisphosphonates: are a group of medicinal products used for the prevention and the treatment of diseases with bone resorption and reabsorption such as osteoporosis and cancer with bone metastasis, the latter being with or without hypercalcaemia, associated to breast cancer and prostate cancer. Examples of bisphosphonates which can be used in the therapy designed by means of the fifth method of the present invention include, although not limited to, nitrogenous bisphosphonates (such as pamidronate, neridronate, olpadronate, alendronate, ibandronate, risedronate, incadronate, zoledronate or zoledronic acid, etc.) and non-nitrogenous bisphosphonates (such as etidronate, clodronate, tiludronate, etc.).
- Alpharadin (radium-223 dichloride). Alpharadin uses alpha radiation from radium-223 decay to kill cancer cells. Radium-223 naturally self-targets to bone metastases by virtue of its properties as a calcium-mimic. Alpha radiation has a very short range of 2-10 cells (when compared to current radiation therapy which is based on beta or gamma radiation), and therefore causes less damage to surrounding healthy tissues (particularly bone marrow). With similar properties to calcium, radium-223 is drawn to places where calcium is used to build bone in the body, including the site of faster, abnormal bone growth - such as that seen in the skeletal metastases of men with advanced, castration-resistant prostate cancer. Radium-223, after injection, is carried in the bloodstream to sites of abnormal bone growth. The place where a cancer starts in the body is known as the primary tumor. Some of these cells may break away and be carried in the bloodstream to another part of the body. The cancer cells may then settle in that part of the body and form a new tumor. If this happens it is called a secondary cancer or a metastasis. Most patients with late stage prostate cancer suffer the maximum burden of disease in their bones. The aim with radium-223 is to selectively target this secondary cancer. Any radium-223 not taken-up in the bones is quickly routed to the gut and excreted.
- "Cathepsin K inhibitors" refers to compounds which interfere in the cathepsin K cysteine protease activity. Non-limiting examples of cathepsin K inhibitors include 4-amino-pyrimidine-2-carbonitrile derivatives (described in the International patent application WO 03/020278 under the name of Novartis Pharma GMBH), pyrrolo-pyrimidines described in the publication WO 03/020721 (Novartis Pharma GMBH) and the publication WO 04/000843 (ASTRAZENECA AB) as well as the inhibitors described in the publications PCT WO 00/55126 of Axys Pharmaceuticals, WO 01/49288 of Merck Frosst Canada & Co. and Axys Pharmaceuticals.
- "DKK-1(Dickkopf-1) inhibitor" as used herein refers to any compound which is capable of reducing DKK-1 activity. DKK-1 is a soluble Wnt pathway antagonist expressed predominantly in adult bone and upregulated in myeloma patients with osteolytic lesions. Agents targeting DKK-1 may play a role in preventing osteolytic bone disease in multiple myeloma patients. BHQ880 from Novartis is a first-in-class, fully human, anti-DKK-1 neutralizing antibody. Preclinical studies support the hypothesis that BHQ880 promotes bone formation and thereby inhibits tumor-induced osteolytic disease (Ettenberg S. et al., American Association for Cancer Research Annual Meeting. April 12-16, 2008; San Diego, Calif. Abstract).
- "Dual MET and VEGFR2 inhibitor" as used herein refers to any compound which is a potent dual inhibitor of the MET and VEGF pathways designed to block MET driven tumor escape. MET is expressed not only in tumor cells and endothelial cells, but also in osteoblasts (bone-forming cells) and osteoclasts (bone-removing cells). HGF binds to MET on all of these cell types, giving the MET pathway an important role in multiple autocrine and paracrine loops. Activation of MET in tumor cells appears to be important in the establishment of metastatic bone lesions. At the same time, activation of the MET pathway in osteoblasts and osteoclasts may lead to pathological features of bone metastases, including abnormal bone growth (ie, blastic lesions) or destruction (ie, lytic lesion. Thus, targeting the MET pathway may be a viable strategy in preventing the establishment and progression of metastatic bone lesions. Cabozantinib (Exelixis, Inc), formerly known as XL184 (CAS 849217-68-1), is a potent dual inhibitor of the MET and VEGF pathways designed to block MET driven tumor escape. In multiple preclinical studies cabozantinib has been shown to kill tumor cells, reduce metastases, and inhibit angiogenesis (the formation of new blood vessels necessary to support tumor growth). Another suitable dual inhibitors are E7050 (N-[2-Fluoro-4-({2-[4-(4-methylpiperazin-1-yl)piperidin-1-yl] carbonylaminopyridin-4-yl}oxy)phenyl]-N'-(4-fluorophenyl)cyclopropane-1,1-dicarboxamide (2R,3R)-tartrate) (CAS 928037-13-2) or Foretinib (also known as GSK1363089, XL880, CAS 849217-64-7).
- "RANKL inhibitors" as used herein refer to any compound which is capable of reducing the RANK activity. RANKL is found on the surface of the osteoblast membrane of the stroma and T-lymphocyte cells, and these T-lymphocyte cells are the only ones which have demonstrated the capacity for secreting it. Its main function is the activation of the osteoclasts, cells involved in the bone resorption. The RANKL inhibitors can act by blocking the binding of RANKL to its receptor (RANK), blocking the RANK-mediated signaling or reducing the expression of RANKL by blocking the transcription or the translation of RANKL. RANKL antagonists or inhibitors suitable for use in the present invention include, without limitation:
   ∘ a suitable RANK protein which is capable of binding RANKL and which comprises the entire or a fragment of the extracellular domain of a RANK protein. The soluble RANK may comprise the signal peptide and the extracellular domain of the murine or human RANK polypeptides, or alternatively, the mature form of the protein with the signal peptide removed can be used.
   ∘ Osteoprotegerin or a variant thereof with RANKL-binding capacity.
   ∘ RANKL-specific antisense molecules
   ∘ Ribozymes capable of processing the transcribed products of RANKL
   ∘ Specific anti-RANKL antibodies. "Anti-RANKL antibody or antibody directed against RANKL" is understood herein as all that antibody which is capable of binding specifically to the ligand of the activating receptor for the nuclear factor κB (RANKL) inhibiting one or more RANKL functions. The antibodies can be prepared using any of the methods which are known by the person skilled in the art. Thus, the polyclonal antibodies are prepared by means of immunizing an animal with the protein to be inhibited. The monoclonal antibodies are prepared using the method described by Kohler, Milstein et al. (Nature, 1975, 256: 495). Antibodies suitable in the context of the present invention include intact antibodies which comprise a variable antigen binding region and a constant region, fragments "Fab", "F(ab')2" and "Fab"', Fv, scFv, diabodies and bispecific antibodies.
   ∘ Specific anti-RANKL nanobodies. Nanobodies are antibody-derived therapeutic proteins that contain the unique structural and functional properties of naturally-occurring heavy-chain antibodies. The Nanobody technology was originally developed following the discovery that camelidae (camels and llamas) possess fully functional antibodies that lack light chains. The general structure of nanobodies is FR1-CDR1-FR2-CDR2-FR3-CDR3-FR4
wherein FR1 to FR4 are the framework regions 1 to 4 CDR1 to CDR3 are the complementarity determining regions 1 to 3. These heavy-chain antibodies contain a single variable domain (VHH) and two constant domains (CH2 and CH3). Importantly, the cloned and isolated VHH domain is a perfectly stable polypeptide harbouring the full antigen-binding capacity of the original heavy-chain antibody. These newly discovered VHH domains with their unique structural and functional properties form the basis of a new generation of therapeutic antibodies which Ablynx has named Nanobodies.

In one embodiment, the RANKL inhibitor is selected from the group consisting of a RANKL specific antibody, a RANKL specific nanobody and osteoprotegerin. In a specific embodiment, the anti-RANKL antibody is a monoclonal antibody. In a yet more specific embodiment, the anti-RANKL antibody is Denosumab (Pageau, Steven C. (2009). mAbs 1 (3): 210-215, CAS number 615258-40-7) (the entire contents of which are hereby incorporated by reference). Denosumab is a fully human monoclonal antibody which binds to RANKL and prevents its activation (it does not bind to the RANK receptor). Various aspects of Denosumab are covered by U.S. Pat. Nos. 6,740,522; 7,411,050; 7,097,834; 7,364,736 (the entire contents of each of which are hereby incorporated by reference in their entirety). In another embodiment, the RANKL inhibitor an antibody, antibody fragment, or fusion construct that binds the same epitope as Denosumab.

In a preferred embodiment, the anti-RANKL nanobody is any of the nanobodies as described in WO2008142164, (the contents of which are incorporated in the present application by reference). In a still more preferred embodiment, the anti-RANKL antibody is the ALX-0141 (Ablynx). ALX-0141 has been designed to inhibit bone loss associated with post-menopausal osteoporosis, reumatoid arthritis, cancer and certain medications, and to restore the balance of healthy bone metabolism.

In a preferred embodiment, the agent preventing the bone degradation is selected from the group consisting of a bisphosphonate, a RANKL inhibitor, PTH and PTHLH inhibitor or a PRG analog, strontium ranelate, a DKK-1 inhibitor, a dual MET and VEGFR2 inhibitor, an estrogen receptor modulator, Radium-223, calcitonin, and a cathepsin K inhibitor. In a more preferred embodiment the agent preventing the bone degradation is a bisphosphonate. In a yet more preferred embodiment, the bisphosphonate is the zoledronic acid.

In one embodiment, a CCR5 antagonist is administered to prevent or inhibit metastasis of the primary breast cancer tumor to bone. In one embodiment, the CCR5 antagonist is a large molecule. In another embodiment, the CCR5 antagonist is a small molecule. In some embodiments, the CCR5 antagonist is Maraviroc (Velasco-Veláquez, M. et al. 2012. CCR5 Antagonist Blocks Metastasis of Basal Breast Cancer Cells. Cancer Research. 72:3839-3850.). In some embodiments, the CCR5 antagonist is Vicriviroc. Velasco-Veláquez, M. et al. 2012. CCR5 Antagonist Blocks Metastasis of Basal Breast Cancer Cells. Cancer Research. 72:3839-3850.). In some aspects, the CCR5 antagonist is Aplaviroc (Demarest J.F. et al. 2005. Update on Aplaviroc: An HIV Entry Inhibitor Targeting CCR5. Retrovirology 2(Suppl. 1): S13). In some aspects, the CCR5 antagonist is a spiropiperidine CCR5 antagonist. (Rotstein D.M. et al. 2009. Spiropiperidine CCR5 antagonists. Bioorganic & Medicinal Chemistry Letters. 19 (18): 5401-5406. In some embodiments, the CCR5 antagonist is INCB009471 (Kuritzkes, D.R. 2009. HIV-1 entry inhibitors: an overview. Curr. Opin. HIV AIDS. 4(2): 82-7).

In a preferred embodiment the dual MET and VEGFR2 inhibitor is selected from the group consisting of Cabozantinib, Foretinib and E7050.

In a preferred embodiment the Radium 223 therapy is alpharadin.

Alternatively a combined treatment can be carried out in which more than one agent from those mentioned above are combined to treat and/or prevent the metastasis or said agents can be combined with other supplements, such as calcium or vitamin D or with a hormone treatment.

### Method of prognosis of metastasis in HER2+ breast cancer, based on detecting the amplification of the MAF gene

In another aspect, the present invention relates to an *in vitro* method (hereinafter sixth method of the present invention) for predicting bone metastasis of a HER2+ breast cancer, in a subject suffering said cancer which comprises determining if the MAF gene is amplified in a sample of said subject relative to a reference gene copy number wherein an amplification of the MAF gene with respect to said reference gene copy number is indicative of increased risk of developing bone metastasis.

In some embodiments, the amplification is in region at the 16q23 locus. In some embodiments, the amplification is in any part of the chromosomal region between about Chr. 16 - about 79,392,959 bp to about 79,663,806 bp (from centromere to telomere). In some embodiments, the amplification is in the genomic region between about Chr. 16 - 79,392,959 bp to about 79,663,806 bp, but excluding DNA repeating elements. In some embodiments, amplification is measured using a probe specific for that region.

In a particular embodiment, the degree of amplification of the MAF gene can be determined by means of determining the amplification of a chromosome region containing said gene. Preferably, the chromosome region the amplification of which is indicative of the existence of amplification of the MAF gene is the locus 16q22-q24 which includes the MAF gene. The locus 16q22-q24 is located in chromosome 16, in the long arm of said chromosome and in a range between band 22 and band 24. This region corresponds in the NCBI database with the contigs NT_010498.15 and NT_010542.15. In another preferred embodiment, the degree of amplification of the MAF gene can be determined by means of using a probe specific for said gene. In another preferred embodiment, the amplification of the MAF gene is determined by means of using the Vysis LSI IGH/MAF Dual Color dual fusion probe,that comprises a probe against 14q32 and 16q23.

The sixth method of the present invention comprises, in a first step, determining if the MAF gene is amplified in a sample of a subject. In a preferred embodiment, the sample is a tumor tissue sample. To that end, the amplification of the MAF gene in the tumor sample is compared with respect to a control sample.

In a particular embodiment, the sixth method of the present invention for the prognosis of the tendency to develop bone metastasis in a subject with HER2+ breast cancer, comprises determining the MAF gene copy number in a sample of said subject and comparing said copy number with the copy number of a control or reference sample, wherein if the MAF copy number is greater with respect to the MAF copy number of a control sample, then the subject has a greater tendency to develop bone metastasis.

The control sample refers to a sample of a subject with HER2+ breast cancer, who has not suffered metastasis or that correspond to the median value of the MAF gene copy number measured in a tumor tissue collection in biopsy samples of subjects with HER2+ breast cancer, respectively, who have not suffered metastasis. Said reference sample is typically obtained by combining equal amounts of samples from a subject population. If the MAF gene copy number is increased with respect to the copy number of said gene in the control sample, then the subject has a greater tendency to develop metastasis.

In a preferred embodiment, the MAF gene is amplified with respect to a reference gene copy number when the MAF gene copy number is higher than the copy number that a reference sample or control sample has. In one example, the MAF gene is said to be "amplified" if the genomic copy number of the MAF gene is increased by at least about 2- (*i.e.,* about 6 copies), about 3- (*i.e.,* about 8 copies), about 4-, about 5-, about 6-, about 7-, about 8-, about 9-, about 10-, about 15-, about 20-, about 25-, about 30-, about 35-, about 40-, about 45-, or about 50-fold in a test sample relative to a control sample. In another example, a MAF gene is said to be "amplified" if the genomic copy number of the MAF gene per cell is at least about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, and the like.

In a particular embodiment, the amplification or the copy number is determined by means of *in situ* hybridization or PCR.

Methods for determining whether the MAF gene or the chromosome region 16q22-q24 is amplified are widely known in the state of the art. Said methods include, without limitation, *in situ* hybridization (ISH) (such as fluorescence *in situ* hybridization (FISH), chromogenic *in situ* hybridization (CISH) or silver *in situ* hybridization (SISH)), genomic comparative hybridization or polymerase chain reaction (such as real time quantitative PCR). For any ISH method, the amplification or the copy number can be determined by counting the number of fluorescent points, colored points or points with silver in the chromosomes or in the nucleus.

The fluorescence *in situ* hybridization (FISH) is a cytogenetic technique which is used for detecting and locating the presence or absence of specific DNA sequences in chromosomes. FISH uses fluorescence probes which only bind to some parts of the chromosome with which they show a high degree of sequence similarity. In a typical FISH method, the DNA probe is labeled with a fluorescent molecule or a hapten, typically in the form of fluor-dUTP, digoxigenin-dUTP, biotin-dUTP or hapten-dUTP which is incorporated in the DNA using enzymatic reactions, such as nick translation or PCR. The sample containing the genetic material (the chromosomes) is placed on glass slides and is denatured by a formamide treatment. The labeled probe is then hybridized with the sample containing the genetic material under suitable conditions which will be determined by the person skilled in the art. After the hybridization, the sample is viewed either directly (in the case of a probe labeled with fluorine) or indirectly (using fluorescently labeled antibodies to detect the hapten).

In the case of CISH, the probe is labeled with digoxigenin, biotin or fluorescein and is hybridized with the sample containing the genetic material in suitable conditions.

Any marking or labeling molecule which can bind to a DNA can be used to label the probes used in the fourth method of the present invention, thus allowing the detection of nucleic acid molecules. Examples of labels for the labeling include, although not limited to, radioactive isotopes, enzyme substrates, cofactors, ligands, chemiluminescence agents, fluorophores, haptens, enzymes and combinations thereof. Methods for labeling and guidelines for selecting suitable labels for different purposes can be found, for example, in Sambrook et al. (Molecular Cloning: A Laboratory Manual, Cold Spring Harbor, New York, 1989) and Ausubel et al. (Current Protocols in Molecular Biology, John Wiley and Sons, New York, 1998).

Once the existence of amplification is determined, either by directly determining the amplification of the MAF gene, the amplification of the 16q23 locus or by determining the amplification of the locus 16q22-q24, and after being compared with the amplification of said gene in the control sample, if amplification in the MAF gene is detected, it is indicative of the fact that the subject has a greater tendency to develop bone metastasis.

The determination of the amplification of the MAF gene needs to be correlated with values of a control sample or reference sample that correspond to the level of amplification of the MAF gene measured in a sample of a subject with HER2+ breast cancer who has not suffered metastasis or that correspond to the median value of the amplification of the MAF gene measured in a tumor tissue collection in biopsy samples of subjects with HER2+ breast cancer who have not suffered metastasis. Said reference sample is typically obtained by combining equal amounts of samples from a subject population. In general, the typical reference samples will be obtained from subjects who are clinically well documented and in whom the absence of metastasis is well characterized. The sample collection from which the reference level is derived will preferably be made up of subjects suffering the same type of cancer as the patient object of the study. Once this median value has been established, the level of amplification of c-MAF in tumor tissues of patients can be compared with this median value, and thus, if there is amplification, the subject has a greater tendency to develop metastasis.

In a preferred embodiment, the bone metastasis is osteolytic bone metastasis. As used herein, the expression "osteolytic bone metastasis" refers to a type of metastasis in which bone resorption (progressive loss of bone density) is produced in the proximity of the metastasis resulting from the stimulation of the osteoclast activity by the tumor cells and is characterized by severe pain, pathological fractures, hypercalcaemia, spinal cord compression and other syndromes resulting from nerve compression.

### Method of prognosis of metastasis in HER2+ breast cancer based on detecting the translocation of the MAF gene

In another aspect, the present invention relates to an *in vitro* method for predicting the clinical outcome of a patient suffering from HER2+ breast cancer, which comprises determining if the MAF gene is translocated in a sample of said subject wherein a translocation of the MAF gene is indicative of a poor clinical outcome.

In another aspect, the present invention relates to an *in vitro* method for predicting the clinical outcome of a patient suffering HER2+ breast cancer, which comprises determining if the MAF gene is translocated in a sample of said subject wherein a translocation of the MAF gene is indicative of a poor clinical outcome.

In some embodiments, the translocated gene is from the region at the 16q23 locus. In some embodiments, the translocated gene is from any part of the chromosomal region between about Chr. 16 - about 79,392,959 bp to 79,663,806 bp (from centromere to telomere). In some embodiments, the translocated gene is from the genomic region between about Chr. 16 - about 79,392,959 bp to 79,663,806 bp, but excluding DNA repeating elements. In some embodiments, the translocation is measured using a probe specific for that region.

In a particular embodiment, the translocation of the MAF gene can be determined by means of determining the translocation of a chromosome region containing said gene. In one embodiment, the translocation is the t(14,16) translocation. In another embodiment, the chromosome region that is translocated is from locus 16q22-q24. The locus 16q22-q24 is located in chromosome 16, in the long arm of said chromosome and in a range between band 22 and band 24. This region corresponds in the NCBI database with the contigs NT_010498.15 and NT_010542.15. In a preferred embodiment, the MAF gene translocates to chromosome 14 at the locus 14q32, resulting in the translocation t(14,16)(q32,q23). This translocation places the MAF gene next to the strong enhancers in the IgH locus, which, in some cases, leads to overexpression of MAF. (Eychène, A., Rocques, N., and Puoponnot, C., A new MAFia in cancer. 2008. Nature Reviews: Cancer. 8: 683-693.)

In a preferred embodiment, the translocation of the MAF gene can be determined by means of using a probe specific for said translocation. In some embodiments, the translocation is measured using a dual color probe. In some embodiments, the translocation is measured using a dual fusion probe. In some embodiments, the translocation is measured using a dual color, dual fusion probe. In some embodiments, the translocation is measured using two separate probes.

In another preferred embodiment, the translocation of the MAF gene is determined using the Vysis LSI IGH/MAF Dual Color dual fusion probe (http://www.abbottmolecular.com/us/products/analyte-specific-reagent/fish/vysis-lsi-igh-maf-dual-color-dual-fusion-probe.html; last accessed 11/5/2012), which comprises a probe against 14q32 and 16q23. In another preferred embodiment, the translocation of the MAF gene is determined using a Kreatech diagnostics MAF/IGH gt(14;16) Fusion probe (http://www.kreatech.com/products/repeat-freetm-poseidontm-fish-probes/hematology/maf-igh-gt1416-fusion-probe.html; last accessed 11/5/2012), an Abnova MAF FISH probe (http://www.abnova.com/products/products_detail.asp?Catalog_id=FA0375; last accessed 11/5/2012), a Cancer Genetics Italia IGH/MAF Two Color, Two Fusion translocation probe (http://www.cancergeneticsitalia.com/dna-fish-probe/ighmaf/; last accessed 11/5/2012), a Creative Bioarray IGH/MAF-t(14;16)(q32;q23) FISH probe (http://www.creative-bioarray.com/products.asp?cid=35&page=10; last accessed 11/5/2012), a Arup Laboratories multiple myeloma panel by FISH (http://www.aruplab.com/files/technical-bulletins/Multiple%20Myeloma%20%28MM%29%20by%20FISH.pdf; last accessed 11/5/2012), an Agilent probe specific to 16q23 or 14q32 (http://www.genomics.agilent.com/ProductSearch.aspx?chr=16&start=79483700&end=7 9754340; last accessed 11/5/2012; http://www.genomics.agilent.com/ProductSearch.aspx?Pageid=3000&ProductID=637; last accessed 11/5/2012), a Dako probe specific to 16q23 or 14q32 (http://www.dako.com/us/ar42/psg42806000/baseproducts_surefish.htm?setCountry=true &purl=ar42/psg42806000/baseproducts_surefish.htm?undefined&submit=Accept%20co untry; last accessed 11/5/2012), a Cytocell IGH/MAF Translocation, Dual Fusion Probe (http://www.zentech.be/uploads/docs/products_info/prenatalogy/cytocell%202012-2013%20catalogue%5B3%5D.pdf; last accessed 11/5/2012), a Metasystems XL IGH / MAF Translocation - Dual Fusion Probe (http://www.metasystems-international.com/index.php?option=com_joodb&view=article&joobase=5&id=12%3Ad -5029-100-og&Itemid=272; last accessed 11/5/2012), a Zeiss FISH Probes XL, 100µl, IGH / MAFB (https://www.micro-shop.zeiss.com/?s=440675675dedc6&1=en&p=uk&f=r&i=5000&o=&h=25&n=1&sd=0 00000-0528-231-uk; last accessed 11/5/2012) or a Genycell Biotech IGH/MAF Dual Fusion Probe (http://www.google.com/url?sa=t&rct=j&q=&esrc=s&source=web&cd=1&ved=0CCQQ FjAA&url=http%3A%2F%2Fwww.genycell.es%2Fimages%2Fproductos%2Fbrochures %2Flphmie6_86.ppt&ei=MhGYUOi3GKWH0QGlt4DoDw&usg=AFQjCNEqQMbT8v QGjJbi9riEf3lVgoFTFQ&sig2=V5IS8juEMVHB18Mv2Xx_Ww; last accessed 11/5/2012)

In some embodiments, the label on the probe is a fluorophore. In some embodiments, the fluorophore on the probe is orange. In some embodiments, the fluorophore on the probe is green. In some embodiments, the fluorophore on the probe is red. In some cases, the fluorophore on the probe is yellow. In some embodiments, one probe is labeled with a red fluorophore, and one with a green fluorophore. In some embodiments, one probe is labeled with a green fluorophore and one with an orange fluorophore. In some cases, the fluorophore on the probe is yellow. For instance, if the MAF-specific probe is labeled with a red fluorophore, and the IGH-specific probe is labeled with a green fluorophore, if white is seen it indicates that the signals overlap and translocation has occurred.

In some embodiments, the fluorophore is SpectrumOrange. In some embodiments, the fluorophore is SpectrumGreen. In some embodiments, the fluorophore is DAPI. In some embodiments, the fluorophore is Platinum*Bright*405 In some embodiments, the fluorophore is Platinum*Bright*415. In some embodiments, the fluorophore is Platinum*Bright*495. In some embodiments, the fluorophore is Platinum*Bright*505. In some embodiments, the fluorophore is Platinum*Bright*550. In some embodiments, the fluorophore is Platinum*Bright*547. In some embodiments, the fluorophore is Platinum*Bright*570. In some embodiments, the fluorophore is Platinum*Bright*590. In some embodiments, the fluorophore is Platinum*Bright*647. In some embodiments, the fluorophore is Platinum*Bright*495/550. In some embodiments, the fluorophore is Platinum*Bright*415/495/550. In some embodiments, the fluorophore is DAPI/Platinum*Bright*495/550. In some embodiments, the fluorophore is FITC. In some embodiments, the fluorophore is Texas Red. In some embodiments, the fluorophore is DEAC. In some embodiments, the fluorophore is R6G. In some embodiments, the fluorophore is Cy5. In some embodiments, the fluorophore is FITC, Texas Red and DAPI. In some embodiments, a DAPI counterstain is used to visualize the translocation, amplification or copy number alteration.

One embodiment of the present invention comprises a method in which in a first step it is determined if the MAF gene is translocated in a sample of a subject. In a preferred embodiment, the sample is a tumor tissue sample.

In a particular embodiment, a method of the present invention for the prognosis of the tendency to develop bone metastasis in a subject with HER2+ breast cancer comprises determining the MAF gene copy number in a sample of said subject wherein the MAF gene is translocated and comparing said copy number with the copy number of a control or reference sample, wherein if the MAF copy number is greater with respect to the MAF copy number of a control sample, then the subject has a greater tendency to develop bone metastasis.

Methods for determining whether the MAF gene or the chromosome region 16q22-q24 is translocated are widely known in the state of the art and include those described previously for the amplification of MAF. Said methods include, without limitation, *in situ* hybridization (ISH) (such as fluorescence *in situ* hybridization (FISH), chromogenic *in situ* hybridization (CISH) or silver *in situ* hybridization (SISH)), genomic comparative hybridization or polymerase chain reaction (such as real time quantitative PCR). For any ISH method, the amplification, the copy number, or the translocation can be determined by counting the number of fluorescent points, colored points or points with silver in the chromosomes or in the nucleus. In other embodiments, the detection of copy number alterations and translocations can be detected through the use of whole genome sequencing, exome sequencing or by the use of any PCR derived technology. For instance, PCR can be performed on samples of genomic DNA to detect translocation. In one embodiment, quantitative PCR is used. In one embodiment, PCR is performed with a primer specific to the MAF gene and a primer specific to the IGH promoter region; if a product is produced, translocation has occurred.

In some embodiments, the amplification and copy number of the MAF gene are determined after translocation of the MAF gene is determined. In some embodiments, the probe is used to determine if the cell is polyploid for the MAF gene. In some embodiments, a determination of polyploidy is made by determining if there are more than 2 signals from the gene of interest. In some embodiments, polyploidy is determined by measuring the signal from the probe specific for the gene of interest and comparing it with a centromeric probe or other probe.

### Method of prognosis of clinical outcome in a HER2+ breast cancer, based on detecting the amplification of the MAF gene

In another aspect, the present invention relates to an *in vitro* method (hereinafter seventh method of the present invention) for predicting the clinical outcome of a patient suffering HER2+ breast cancer, which comprises determining if the MAF gene is amplified in a sample of said subject relative to a reference gene copy number wherein an amplification of the MAF gene with respect to said reference gene copy number is indicative of a poor clinical outcome.

The seventh method of the present invention comprises, in a first step, determining if the MAF gene is amplified in a sample of a subject. The determination of the amplification of the MAF is carried out essentially as described in the fifth method of the present invention. In a preferred embodiment the sample is a tumor tissue sample. In a preferred embodiment, the amplification of the MAF gene is determined by means of determining the amplification of the locus 16q23 or 16q22-q24. In another preferred embodiment, the amplification of the MAF gene is determined by means of using a MAF gene-specific probe.

In a second step, the seventh method of the present invention comprises comparing said copy number with the copy number of a control or reference sample, wherein if the MAF copy number is greater with respect to the MAF copy number of a control sample, then this is indicative of a poor clinical outcome.

In a preferred embodiment, the MAF gene is amplified with respect to a reference gene copy number when the MAF gene copy number is higher than the copy number that a reference sample or control sample has. In one example, the MAF gene is said to be "amplified" if the genomic copy number of the MAF gene is increased by at least about *2-* (*i.e.,* about 6 copies), about 3- (*i.e.,* about 8 copies), about 4-, about 5-, about 6-, about 7-, about 8-, about 9-, about 10-, about 15-, about 20-, about 25-, about 30-, about 35-, about 40-, about 45-, or about 50-fold in a test sample relative to a control sample. In another example, a MAF gene is said to be "amplified" if the genomic copy number of the MAF gene per cell is at least about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, and the like.

In another embodiment, the reference gene copy number is the gene copy number in a sample of HER2+ breast cancer, from a subject who has not suffered bone metastasis.

In another embodiment, the amplification is determined by means of *in situ* hybridization or PCR.

### Method for designing customized therapy - in patients with HER2+ breast tumors

As is known in the state of the art, the treatment to be administered to a subject suffering from cancer depends on whether the latter is a malignant tumor, *i.e.,* whether it has high probabilities of undergoing metastasis, or whether the latter is a benign tumor. In the first assumption, the treatment of choice is a systemic treatment such as chemotherapy and in the second assumption, the treatment of choice is a localized treatment such as radiotherapy.

Therefore, as described in the present application, given that MAF gene amplification or translocation in HER2+ breast cancer cells is related to the presence of bone metastasis, the MAF gene amplification or translocation is useful for making decisions in terms of the most suitable therapy for the subject suffering said cancer. In a preferred embodiment, the amplification of the MAF gene is determined by means of determining the amplification of the locus 16q23 or 16q22-q24. In another preferred embodiment, the amplification of the MAF gene is determined by means of using a MAF gene-specific probe.

Thus, in another aspect the present invention relates to an *in vitro* method (hereinafter third method of the present invention) for designing a customized therapy for a subject suffering HER2+ breast cancer, which comprises
iii) quantifying the MAF gene amplification or translocation in a sample of said subject and
iv) comparing the gene amplification or translocation obtained in i) with a reference value,
wherein if the MAF gene amplification or translocation is increased with respect to said reference value, then said subject is susceptible to receive a therapy or not aiming to prevent and/or treat the bone metastasis. If the MAF gene amplification or translocation is not increased with respect to said reference value, then said subject is not susceptible to receive a therapy aiming to prevent and/or treat the bone metastasis.
In a preferred embodiment, the amplification of the MAF gene is determined by means of determining the amplification of the locus 16q23 or 16q22-q24. In another preferred embodiment, the amplification of the MAF gene is determined by means of using a MAF gene-specific probe.

In a particular embodiment, the bone metastasis is osteolytic metastasis.

Another method of the present invention comprises quantifying the MAF gene amplification or translocation in a sample in a subject suffering from HER2+ breast cancer. In a preferred embodiment, the sample is a tumor tissue sample.

In another particular embodiment, the method of the present invention comprises quantifying only the MAF gene amplification or translocation as a single marker, *i.e.,* the method does not involve determining the expression level of any additional marker.

In the case of this particular method of the present invention the sample can be a primary tumor tissue sample of the subject.

In a second step, the MAF gene amplification or translocation obtained in the tumor sample of the subject is compared with a reference value. In a preferred embodiment, the reference value is the MAF gene amplification or translocation of said gene in a control sample. The determination of the MAF gene amplification or translocation must be related to values of a control sample or reference sample. Depending on the type of tumor to be analyzed, the exact nature of the control sample may vary. Thus preferably the reference sample is a sample of a subject with HER2+ breast cancer, that has not metastasized or that corresponds to MAF gene amplification or translocation measured in a tumor tissue collection in biopsy samples of subjects with HER2+ breast cancer, which has not metastasized.

Once the MAF gene amplification or translocation in the sample has been measured and compared with the reference value, if the gene amplification or translocation of said gene is increased with respect to the reference value, then it can be concluded that said subject is susceptible to receiving therapy or not receiving a therapy aiming to prevent (if the subject has yet to undergo metastasis) and/or treat metastasis or not prevent and/or treat metastasis (if the subject has already experienced metastasis).

When the cancer has metastasized, systemic treatments including but not limited to chemotherapy, hormone treatment, immunotherapy, or a combination thereof can be used. Additionally, radiotherapy and/or surgery can be used. The choice of treatment generally depends on the type of primary cancer, the size, the location of the metastasis, the age, the general health of the patient and the types of treatments used previously.

The systemic treatments are those that reach the entire body and represent therapies aiming to prevent (if the subject has yet to undergo metastasis) and/or treat metastasis (if the subject has already experienced metastasis), such as:
- Chemotherapy is the use of medicaments to destroy cancer cells. The medicaments are generally administered through oral or intravenous route. Sometimes, chemotherapy is used together with radiation treatment. Suitable chemotherapeutic treatments for breast cancer include, without limitation, anthracyclines (doxorubicin, epirubicin, pegylated liposomal doxorubicin), Taxanes (paclitaxel, docetaxel, albumin nano-particle bound paclitaxel), 5-fluorouracil (continuous infusion 5-FU, capecitabine), Vinca alkaloids (vinorelbine, vinblastine), Gemcitabine, Platinum salts (cisplatin, carboplatin), cyclophosphamide, Etoposide and combinations of one or more of the above such as Cyclophosphamide/anthracycline +/- 5-fluorouracil regimens (such as doxorubicin/ cyclophosphamide (AC), epirubicin/cyclophosphamide, (EC) cyclophosphamide/epirubicin/5-fluorouracil (CEF), cyclophosphamide/doxorubicin/5-fluorouracil (CAF), 5-fluorouracil /epirubicin/cyclophosphamide (FEC)), cyclophosphamide/metothrexate/5-fluorouracil (CMF), anthracyclines/taxanes (such as doxorubicin/paclitaxel or doxorubicin/docetaxel), Docetaxel/capecitabine, Gemcitabine/paclitaxel, Taxane/platinum regimens (such as paclitaxel/carboplatin or docetaxel/carboplatin).
- Immunotherapy is a treatment that aids the immune system itself of the patient to combat cancer. There are several types of immunotherapy which are used to treat metastasis in patients. These include but are not limited to cytokines, monoclonal antibodies and antitumor vaccines.

In another aspect, the treatment is Alpharadin (radium-223 dichloride). Alpharadin uses alpha radiation from radium-223 decay to kill cancer cells. Radium-223 naturally self-targets to bone metastases by virtue of its properties as a calcium-mimic. Alpha radiation has a very short range of 2-10 cells (when compared to current radiation therapy which is based on beta or gamma radiation), and therefore causes less damage to surrounding healthy tissues (particularly bone marrow). With similar properties to calcium, radium-223 is drawn to places where calcium is used to build bone in the body, including the site of faster, abnormal bone growth - such as that seen in the skeletal metastases of men with advanced, castration-resistant prostate cancer. Radium-223, after injection, is carried in the bloodstream to sites of abnormal bone growth. The place where a cancer starts in the body is known as the primary tumor. Some of these cells may break away and be carried in the bloodstream to another part of the body. The cancer cells may then settle in that part of the body and form a new tumor. If this happens it is called a secondary cancer or a metastasis. Most patients with late stage prostate cancer suffer the maximum burden of disease in their bones. The aim with radium-223 is to selectively target this secondary cancer. Any radium-223 not taken-up in the bones is quickly routed to the gut and excreted.

In another aspect, the treatment is an mTor inhibitor. In some aspects, the mTor inhibitor is a dual mTor/PI3kinase inhibitor. In some aspects, the mTor inhibitor is used to prevent or inhibit metastasis. In some aspects the mTor inhibitor is selected from the group consisting of: ABI009 (sirolimus), rapamycin (sirolimus), Abraxane (paclitaxel), Absorb (everolimus), Afinitor (everolimus), Afinitor with Gleevec, AS703026 (pimasertib), Axxess (umirolimus), AZD2014, BEZ235, Biofreedom (umirolimus), BioMatrix (umirolimus), BioMatrix flex (umirolimus), CC115, CC223, Combo Bioengineered Sirolimus Eluting Stent ORBUSNEICH (sirolimus), Curaxin CBLC102 (mepacrine), DE109 (sirolimus), DS3078, Endeavor DES (zotarolimus), Endeavor Resolute (zotarolimus), Femara (letrozole), Hocena (antroquinonol), INK128, Inspiron (sirolimus), IPI504 (retaspimycin hydrochloride), KRN951 (tivozanib), ME344, MGA031 (teplizumab), MiStent SES (sirolimus), MKC1, Nobori (umirolimus), OSI027, OVI123 (cordycepin), Palomid 529, PF04691502, Promus Element (everolimus), PWT33597, Rapamune (sirolimus), Resolute DES (zotarolimus), RG7422, SAR245409, SF1126, SGN75 (vorsetuzumab mafodotin), Synergy (everolimus), Taltorvic (ridaforolimus), Tarceva (erlotinib), Torisel (temsirolimus), Xience Prime (everolimus), Xience V (everolimus), Zomaxx (zotarolimus), Zortress (everolimus), Zotarolimus Eluting Peripheral Stent MEDTRONIC (zotarolimus), AP23841, AP24170, ARmTOR26, BN107, BN108, Canstatin GENZYME (canstatin), CU906, EC0371, EC0565, KI1004, LOR220, NV128, Rapamycin ONCOIMMUNE (sirolimus), SB2602, Sirolimus PNP SAMYANG BIOPHARMACEUTICALS (sirolimus), TOP216, VLI27, VS5584, WYE125132, XL388, Advacan (everolimus), AZD8055, Cypher Select Plus Sirolimus eluting Coronary Stent (sirolimus), Cypher Sirolimus eluting coronary stent (sirolimus), Drug Coated Balloon (sirolimus), E-Magic Plus (sirolimus), Emtor (sirolimus), Esprit (everolimus), Evertor (everolimus), HBF0079, LCP-Siro (sirolimus), Limus CLARIS (sirolimus), mTOR Inhibitor CELLZOME, Nevo Sirolimus eluting Coronary Stent (sirolimus), nPT-mTOR, Rapacan (sirolimus), Renacept (sirolimus), ReZolve (sirolimus), Rocas (sirolimus), SF1126, Sirolim (sirolimus), Sirolimus NORTH CHINA (sirolimus), Sirolimus RANBAXY (sirolimus), Sirolimus WATSON (sirolimus) Siropan (sirolimus), Sirova (sirolimus), Supralimus (sirolimus), Supralimus-Core (sirolimus), Tacrolimus WATSON (tacrolimus), TAFA93, Temsirolimus ACCORD (temsirolimus), Temsirolimus SANDOZ (temsirolimus), TOP216, Xience Prime (everolimus), Xience V (everolimus). In a specific aspect the mTor inhibitor is Afinitor (everolimus) (http://www.afinitor.com/index.jsp?usertrack.filter_applied=true&Novald=40294620643 38207963; last accessed 11/28/2012). In another aspect, everolimus is combined with an aromatase inhibitor. (*See. e.g.,* Baselga, J., el al., Everolimus in Postmenopausal Hormone-Receptor Positive Advanced Breast Cancer. 2012. N. Engl. J. Med. 366(6): 520-529, which is herein incorporated by reference). In another aspect, mTor inhibitors can be identified through methods known in the art. (*See, e.g.,* Zhou, H. et al. Updates of mTor inhibitors. 2010. Anticancer Agents Med. Chem. 10(7): 571-81, which is herein incorporated by reference). In some aspects, the mTor inhibitor is used to treat or prevent or inhibit metastasis in a patient that is positive for a hormone receptor. (*See. e.g.,* Baselga, J., el al., Everolimus in Postmenopausal Hormone-Receptor Positive Advanced Breast Cancer. 2012. N. Engl. J. Med. 366(6): 520-529). In some embodiments, the patient is ER+. In some aspects, the mTor inhibitor is used to treat or prevent or inhibit metastasis in a patient with advanced breast cancer. In some aspects, the mTor inhibitor is used in combination with a second treatment. In some aspects, the second treatment is any treatment described herein.

In another aspect, the treatment is a Src kinase inhibitor. In some aspects, the Src inhibitor is used to prevent or inhibit metastasis. In some aspects, the Src kinase inhibitor is selected from the group: AZD0530 (saracatinib), Bosulif (bosutinib), ENMD981693, KD020, KX01, Sprycel (dasatinib), Yervoy (ipilimumab), AP23464, AP23485, AP23588, AZD0424, c-Src Kinase Inhibitor KISSEI, CU201, KX2361, SKS927, SRN004, SUNK706, TG100435, TG100948, AP23451, Dasatinib HETERO (dasatinib), Dasatinib VALEANT (dasatinib), Fontrax (dasatinib), Src Kinase Inhibitor KINEX, VX680,(tozasertib lactate), XL228, and SUNK706. In some embodiments, the Src kinase inhibitor is dasatinib. In another aspect, Src kinase inhibitors can be identified through methods known in the art (*See, e.g.,* Sen, B. and Johnson, F.M. Regulation of Src Family Kinases in Human Cancers. 2011. J. Signal Transduction. 2011: 14 pages, which is herein incorporated by reference). In some aspects, the Src kinase inhibitor is used to treat or prevent or inhibit metastasis in a patient that is positive for the SRC-responsive signature (SRS). In some aspects, the patient is SRS+ and ER-. (*See.* e.g.,Zhang, CH.-F, et al. Latent Bone Metastasis in Breast Cancer Tied to Src-Dependent survival signals. 2009. Cancer Cell. 16: 67-78, which is herein incorporated by reference.) In some aspects, the Src kinase inhibitor is used to treat or prevent or inhibit metastasis in a patient with advanced breast cancer. In some aspects, the Src kinase inhibitor is used in combination with a second treatment. In some aspects, the second treatment is any treatment described herein.

In another aspect, the treatment is a COX-2 inhibitor. In some aspects, the COX-2 inhibitor is used to prevent or inhibit metastasis. In some aspects, the COX-2 inhibitor is selected from the group: ABT963, Acetaminophen ER JOHNSON (acetaminophen), Acular X (ketorolac tromethamine), BAY1019036 (aspirin), BAY987111 (diphenhydramine, naproxen sodium), BAY11902 (piroxicam), BCIBUCH001 (ibuprofen), Capoxigem (apricoxib), CS502, CS670 (pelubiprofen), Diclofenac HPBCD (diclofenac), Diractin (ketoprofen), GW406381, HCT1026 (nitroflurbiprofen), Hyanalgese-D (diclofenac), HydrocoDex (acetaminophen, dextromethorphan, hydrocodone), Ibuprofen Sodium PFIZER (ibuprofen sodium), Ibuprofen with Acetaminophen PFIZER (acetaminophen, ibuprofen), Impracor (ketoprofen), IP880 (diclofenac), IP940 (indomethacin), ISV205 (diclofenac sodium), JNS013 (acetaminophen, tramadol hydrochloride), Ketoprofen TDS (ketoprofen), LTNS001 (naproxen etemesil), Mesalamine SALIX (mesalamine), Mesalamine SOFAR (mesalamine), Mesalazine (mesalamine), ML3000 (licofelone), MRX7EAT (etodolac), Naproxen IROKO (naproxen), NCX4016 (nitroaspirin), NCX701 (nitroacetaminophen), Nuprin SCOLR (ibuprofen), OMS103HP (amitriptyline hydrochloride, ketoprofen, oxymetazoline hydrochloride), Oralease (diclofenac), OxycoDex (dextromethorphan, oxycodone), P54, PercoDex (acetaminophen, dextromethorphan, oxycodone), PL3100 (naproxen, phosphatidyl choline), PSD508, R-Ketoprofen (ketoprofen), Remura (bromfenac sodium), ROX828 (ketorolac tromethamine), RP19583 (ketoprofen lysine), RQ00317076, SDX101 (R-etodolac), TDS943 (diclofenac sodium), TDT070 (ketoprofen), TPR100, TQ1011 (ketoprofen), TT063 (S-flurbiprofen), UR8880 (cimicoxib), V0498TA01A (ibuprofen), VT122 (etodolac, propranolol), XP20B (acetaminophen, dextropropoxyphene), XP21B (diclofenac potassium), XP21L (diclofenac potassium), Zoenasa (acetylcysteine, mesalamine), Acephen, Actifed Plus, Actifed-P, Acular, Acular LS, Acular PF, Acular X, Acuvail, Advil, Advil Allergy Sinus, Advil Cold and Sinus, Advil Congestion Relief ,Advil PM, Advil PM Capsule, Air Salonpas, Airtal, Alcohol-Free NyQuil Cold & Flu Relief, Aleve, Aleve ABDI IBRAHIM, Aleve-D, Alka-Seltzer ,Alka-Seltzer BAYER, Alka-Seltzer Extra Strength, Alka-Seltzer Lemon-Lime, Alka-Seltzer Original, Alka-Seltzer Plus, Alka-Seltzer plus Cold and Cough, Alka-Seltzer plus Cold and Cough Formula, Alka-Seltzer Plus Day and Night Cold Formula,, Alka-Seltzer Plus Day Non-Drowsy Cold Formula, Alka-Seltzer Plus Flu Formula, Alka-Seltzer Plus Night Cold Formula, Alka-Seltzer Plus Sinus Formula, Alka-Seltzer Plus Sparkling Original Cold Formula, Alka-Seltzer PM, Alka-Seltzer Wake-Up Call, Anacin, Anaprox, Anaprox MINERVA, Ansaid, Apitoxin, Apranax, Apranax abdi, Arcoxia, Arthritis Formula Bengay, Arthrotec, Asacol, Asacol HD, Asacol MEDUNA ARZNEIMITTEL, Asacol ORIFARM, Aspirin BAYER, Aspirin Complex, Aspirin Migran, AZD3582, Azulfidine, Baralgan M, BAY1019036, BAY987111, BAYl1902, BCIBUCH001, Benadryl Allergy, Benadryl Day and Night, Benylin 4 Flu, Benylin Cold and Flu, Benylin Cold and Flu Day and Night, Benylin Cold and Sinus Day and Night, Benylin Cold and Sinus Plus, Benylin Day and Night Cold and Flu Relief, Benylin1 All-In-One, Brexin, Brexin ANGELINI, Bromday, Bufferin, Buscopan Plus, Caldolor, Calmatel, Cambia, Canasa, Capoxigem, Cataflam, Celebrex, Celebrex ORIFARM, Children's Advil Allergy Sinus, Children's Tylenol, Children's Tylenol Cough and Runny Nose, Children's Tylenol plus cold, Children's Tylenol plus Cold and Cough, Children's Tylenol plus cold and stuffy nose, Children's Tylenol plus Flu, Children's Tylenol plus cold & allergy, Children's Tylenol plus Cough & Runny Nose, Children's Tylenol plus Cough & Sore Throat, Children's Tylenol plus multi symptom cold, Clinoril, Codral Cold and Flu, Codral Day and Night Day Tablets, Codral Day and Night Night Tablets, Codral Nightime, Colazal, Combunox, Contac Cold plus Flu, Contac Cold plus Flu Non-Drowsy, Coricidin D, Coricidin HBP Cold and Flu, Coricidin HBP Day and Night Multi-Symptom Cold, Coricidin HBP Maximum Strength Flu, Coricidin HBP Nighttime Multi-Symptom Cold, Coricidin II Extra Strength Cold and Flu, CS502, CS670, Daypro, Daypro Alta, DDS06C, Demazin Cold and Flu, Demazin Cough, Cold and Flu, Demazin day/night Cold and Flu, Demazin PE Cold and Flu, Demazin PE day/night Cold and Flu, Diclofenac HPBCD, Dimetapp Day Relief, Dimetapp Multi-Symptom Cold and Flu, Dimetapp Night Relief, Dimetapp Pain and Fever Relief, Dimetapp PE Sinus Pain, Dimetapp PE Sinus Pain plus Allergy, Dipentum, Diractin, Disprin Cold 'n' Fever, Disprin Extra, Disprin Forte. Disprin Plus, Dristan Cold, Dristan Junior, Drixoral Plus, Duexis, Dynastat, Efferalgan, Efferalgan Plus Vitamin C, Efferalgan Vitamin C, Elixsure IB, Excedrin Back and Body, Excedrin Migraine, Excedrin PM, Excedrin Sinus Headache, Excedrin Tension Headache, Falcol, Fansamac, Feldene, FeverAll, Fiorinal, Fiorinal with Codeine, Flanax, Flector Patch, Flucam, Fortagesic, Gerbin, Giazo, Gladio, Goody's Back and Body Pain, Goody's Cool Orange, Goody's Extra Strength, Goody's PM, Greaseless Bengay, GW406381, HCT1026, He Xing Yi, Hyanalgese-D, HydrocoDex, Ibuprofen Sodium PFIZER, Ibuprofen with, Acetaminophen PFIZER, Icy Hot SANOFI AVENTIS, Impracor, Indocin, Indomethacin APP PHARMA, Indomethacin MYLAN, Infants' Tylenol, IP880, IP940, Iremod, ISV205, JNS013, Jr. Tylenol, Junifen, Junior Strength Advil, Junior Strength Motrin, Ketoprofen TDS, Lemsip Max, Lemsip Max All in One, Lemsip Max All Night, Lemsip Max Cold and Flu, Lialda, Listerine Mouth Wash, Lloyds Cream, Lodine, Lorfit P, Loxonin, LTNS001, Mersyndol, Mesalamine SALIX, Mesalamine SOFAR, Mesalazine, Mesasal GLAXO, Mesasal SANOFI, Mesulid, Metsal Heat Rub, Midol Complete, Midol Extended Relief, Midol Liquid Gels, Midol PM, Midol Teen Formula, Migranin COATED TABLETS, ML3000, Mobic, Mohrus, Motrin, Motrin Cold and Sinus Pain, Motrin PM, Movalis ASPEN, MRX7EAT, Nalfon, Nalfon PEDINOL, Naprelan, Naprosyn, Naprosyn RPG LIFE SCIENCE, Naproxen IROKO, NCX4016, NCX701, NeoProfen LUNDBECK, Nevanac, Nexcede, Niflan, Norgesic MEDICIS, Novalgin, Nuprin SCOLR, Nurofen, Nurofen Cold and Flu, Nurofen Max Strength Migraine, Nurofen Plus, Nuromol, NyQuil with Vitamin C, Ocufen, OMS103HP, Oralease, Orudis ABBOTT JAPAN, Oruvail, Osteluc, OxycoDex, P54, Panadol, Panadol Actifast, Paradine, Paramax, Parfenac, Pedea, Pennsaid, Pentasa, Pentasa ORIFARM, Peon, Percodan, Percodan-Demi, PercoDex, Percogesic, Perfalgan, PL2200, PL3100, Ponstel, Prexige, Prolensa, PSD508, R-Ketoprofen, Rantudil, Relafen, Remura, Robaxisal, Rotec, Rowasa, ROX828, RP19583, RQ00317076, Rubor, Salofalk, Salonpas, Saridon, SDX101, Seltouch, sfRowasa, Shinbaro, Sinumax, Sinutab, Sinutab,, sinus, Spalt, Sprix, Strefen, Sudafed Cold and Cough, Sudafed Head Cold and Sinus, Sudafed PE Cold plus Cough, Sudafed PE Pressure plus Pain, Sudafed PE, Severe Cold, Sudafed PE Sinus Day plus Night Relief Day Tablets, Sudafed PE Sinus Day plus Night Relief Night Tablets, Sudafed PE Sinus plus Anti-inflammatory Pain Relief, Sudafed Sinus Advance, Surgam, Synalgos-DC, Synflex, Tavist allergy/sinus/headache, TDS943, TDT070, Theraflu Cold and Sore Throat, Theraflu Daytime Severe Cold and Cough, Theraflu Daytime Warming Relief,Theraflu Warming Relief Caplets Daytime Multi-Symptom Cold, Theraflu Warming Relief Cold and Chest Congestion, Thomapyrin, Thomapyrin C, Thomapyrin Effervescent, Thomapyrin Medium, Tilcotil, Tispol, Tolectin, Toradol, TPR100, TQ1011, Trauma-Salbe, Trauma-Salbe Kwizda, Treo, Treximet, Trovex, TT063, Tylenol, Tylenol Allergy Multi-Symptom, Tylenol Back Pain, Tylenol Cold & Cough Daytime, Tylenol Cold & Cough Nighttime, Tylenol Cold and Sinus Daytime, Tylenol Cold and Sinus Nighttime, Tylenol Cold Head Congestion Severe, Tylenol Cold Multi Symptom Daytime, Tylenol Cold Multi Symptom Nighttime Liquid, Tylenol Cold Multi Symptom Severe, Tylenol Cold Non-Drowsiness Formula, Tylenol Cold Severe Congestion Daytime, Tylenol Complete Cold, Cough and Flu Night time, Tylenol Flu Nighttime, Tylenol Menstrual, Tylenol PM, Tylenol Sinus Congestion & Pain Daytime, Tylenol Sinus Congestion & Pain Nighttime, Tylenol Sinus Congestion & Pain Severe, Tylenol Sinus Severe Congestion Daytime, Tylenol Ultra Relief, Tylenol with Caffeine and Codeine phosphate, Tylenol with Codeine phosphate, Ultra Strength Bengay Cream, Ultracet, UR8880, V0498TA01A, Vicks NyQuil Cold and Flu Relief, Vicoprofen, Vimovo, Voltaren Emulgel, Voltaren GEL, Voltaren NOVARTIS CONSUMER HEALTH GMBH, Voltaren XR, VT122, Xefo, Xefo Rapid, Xefocam, Xibrom, XL3, Xodol, XP20B, XP21B, XP21L, Zipsor, and Zoenasa. In another aspect, COX-2 inhibitors can be identified through methods known in the art (*See, e.g.,* Dannhardt, G. and Kiefer, W. Cyclooxygenase inhibitors- current status and future prospects. 2001. Eur. J. Med. Chem. 36: 109-126, which is herein incorporated by reference). In some aspects, the COX-2 inhibitor is used to treat or prevent or inhibit metastasis in a patient with advanced breast cancer. In some aspects, the COX-2 inhibitor is used in combination with a second treatment. In some aspects, the second treatment is any treatment described herein. In some aspects, the COX-2 inhibitor is used in combination with a second treatment selected from the group consisting of: Denosumab, Zometa (http://www.us.zometa.com/index.jsp?usertrack.filter_applied=true&NovaId=293537693 4467633633; last accessed 12/2/2012), Carbozantinib or Cabozantinib, Antibody or peptide blocking PTHLH (parathyroid hormone like hormone) or PTHrP (parathyroid hormone related protein) and Everolimus.

In another aspect, the treatment agents used for avoiding and/or preventing bone degradation include, although not limited to:
- Parathyroid hormone (PTH) and Parathyroid like hormone (PTHLH) inhibitors (including blocking antibodies) or recombinant forms thereof (teriparatide corresponding to the amino acids 7-34 of PTH). This hormone acts by stimulating the osteoclasts and increasing their activity.
- Strontium ranelate: is an alternative oral treatment, and forms part of the group of drugs called "dual action bone agents" (DABAs) because they stimulate the osteoblast proliferation and inhibit the osteoclast proliferation.
- "Estrogen receptor modulators" (SERM) refers to compounds which interfere or inhibit the binding of estrogens to the receptor, regardless of the mechanism. Examples of estrogen receptor modulators include, among others, estrogens progestagen, estradiol, droloxifene, raloxifene, lasofoxifene, TSE-424, tamoxifen, idoxifene, L Y353381, LY117081, toremifene, fluvestrant, 4-[7-(2,2-dimethyl-1-oxopropoxy-4-methyl-2-[4-[2-(1-piperidinyl)ethoxy]phenyl]-2H-1-benzopyran-3-yl]-phenyl-2,2-dimethylpropanoate 4,4'dihydroxybenzophenone-2,4-dinitrophenyl-hydrazone and SH646.
- Calcitonin: directly inhibits the osteoclast activity through the calcitonin receptor. The calcitonin receptors have been identified on the surface of the osteoclasts.
- Bisphosphonates: are a group of medicinal products used for the prevention and the treatment of diseases with bone resorption and reabsorption such as osteoporosis and cancer with bone metastasis, the latter being with or without hypercalcaemia, associated to breast cancer and prostate cancer. Examples of bisphosphonates which can be used in the therapy designed by means of the fifth method of the present invention include, although not limited to, nitrogenous bisphosphonates (such as pamidronate, neridronate, olpadronate, alendronate, ibandronate, risedronate, incadronate, zoledronate or zoledronic acid, etc.) and non-nitrogenous bisphosphonates (such as etidronate, clodronate, tiludronate, etc.).
- "Cathepsin K inhibitors" refers to compounds which interfere in the cathepsin K cysteine protease activity. Non-limiting examples of cathepsin K inhibitors include 4-amino-pyrimidine-2-carbonitrile derivatives (described in the International patent application WO 03/020278 under the name of Novartis Pharma GMBH), pyrrolo-pyrimidines described in the publication WO 03/020721 (Novartis Pharma GMBH) and the publication WO 04/000843 (ASTRAZENECA AB) as well as the inhibitors described in the publications PCT WO 00/55126 of Axys Pharmaceuticals, WO 01/49288 of Merck Frosst Canada & Co. and Axys Pharmaceuticals.
- "DKK-1(Dickkopf-1) inhibitor" as used herein refers to any compound which is capable of reducing DKK-1 activity. DKK-1 is a soluble Wnt pathway antagonist expressed predominantly in adult bone and upregulated in myeloma patients with osteolytic lesions. Agents targeting DKK-1 may play a role in preventing osteolytic bone disease in multiple myeloma patients. BHQ880 from Novartis is a first-in-class, fully human, anti-DKK-1 neutralizing antibody. Preclinical studies support the hypothesis that BHQ880 promotes bone formation and thereby inhibits tumor-induced osteolytic disease (Ettenberg S. et al., American Association for Cancer Research Annual Meeting. April 12-16, 2008; San Diego, Calif. Abstract).
- "Dual MET and VEGFR2 inhibitor" as used herein refers to any compound which is a potent dual inhibitor of the MET and VEGF pathways designed to block MET driven tumor escape. MET is expressed not only in tumor cells and endothelial cells, but also in osteoblasts (bone-forming cells) and osteoclasts (bone-removing cells). HGF binds to MET on all of these cell types, giving the MET pathway an important role in multiple autocrine and paracrine loops. Activation of MET in tumor cells appears to be important in the establishment of metastatic bone lesions. At the same time, activation of the MET pathway in osteoblasts and osteoclasts may lead to pathological features of bone metastases, including abnormal bone growth (ie, blastic lesions) or destruction (ie, lytic lesion. Thus, targeting the MET pathway may be a viable strategy in preventing the establishment and progression of metastatic bone lesions. Cabozantinib (Exelixis, Inc), formerly known as XL184 (CAS 849217-68-1), is a potent dual inhibitor of the MET and VEGF pathways designed to block MET driven tumor escape. In multiple preclinical studies cabozantinib has been shown to kill tumor cells, reduce metastases, and inhibit angiogenesis (the formation of new blood vessels necessary to support tumor growth). Another suitable dual inhibitors are E7050 (N-[2-Fluoro-4-({2-[4-(4-methylpiperazin-1-yl)piperidin-1-yl] carbonylaminopyridin-4-yl} oxy) phenyl]-N'-(4-fluorophenyl) cyclopropane-1,1-dicarboxamide (2R,3R)-tartrate) (CAS 928037-13-2) or Foretinib (also known as GSK1363089, XL880, CAS 849217-64-7).
- "RANKL inhibitors" as used herein refer to any compound which is capable of reducing the RANK activity. RANKL is found on the surface of the osteoblast membrane of the stroma and T-lymphocyte cells, and these T-lymphocyte cells are the only ones which have demonstrated the capacity for secreting it. Its main function is the activation of the osteoclasts, cells involved in the bone resorption. The RANKL inhibitors can act by blocking the binding of RANKL to its receptor (RANK), blocking the RANK-mediated signaling or reducing the expression of RANKL by blocking the transcription or the translation of RANKL. RANKL antagonists or inhibitors suitable for use in the present invention include, without limitation:
   ∘ a suitable RANK protein which is capable of binding RANKL and which comprises the entire or a fragment of the extracellular domain of a RANK protein. The soluble RANK may comprise the signal peptide and the extracellular domain of the murine or human RANK polypeptides, or alternatively, the mature form of the protein with the signal peptide removed can be used.
   ∘ Osteoprotegerin or a variant thereof with RANKL-binding capacity.
   ∘ RANKL-specific antisense molecules
   ∘ Ribozymes capable of processing the transcribed products of RANKL
   ∘ Specific anti-RANKL antibodies. "Anti-RANKL antibody or antibody directed against RANKL" is understood herein as all that antibody which is capable of binding specifically to the ligand of the activating receptor for the nuclear factor κB (RANKL) inhibiting one or more RANKL functions. The antibodies can be prepared using any of the methods which are known by the person skilled in the art. Thus, the polyclonal antibodies are prepared by means of immunizing an animal with the protein to be inhibited. The monoclonal antibodies are prepared using the method described by Kohler, Milstein et al. (Nature, 1975, 256: 495). Antibodies suitable in the context of the present invention include intact antibodies which comprise a variable antigen binding region and a constant region, fragments "Fab", "F(ab')2" and "Fab'", Fv, scFv, diabodies and bispecific antibodies.
   ∘ Specific anti-RANKL nanobodies. Nanobodies are antibody-derived therapeutic proteins that contain the unique structural and functional properties of naturally-occurring heavy-chain antibodies. The Nanobody technology was originally developed following the discovery that camelidae (camels and llamas) possess fully functional antibodies that lack light chains. The general structure of nanobodies is
      FR1-CDR1-FR2-CDR2-FR3-CDR3-FR4 wherein FR1 to FR4 are the framework regions 1 to 4 CDR1 to CDR3 are the complementarity determining regions 1 to 3. These heavy-chain antibodies contain a single variable domain (VHH) and two constant domains (CH2 and CH3). Importantly, the cloned and isolated VHH domain is a perfectly stable polypeptide harbouring the full antigen-binding capacity of the original heavy-chain antibody. These newly discovered VHH domains with their unique structural and functional properties form the basis of a new generation of therapeutic antibodies which Ablynx has named Nanobodies.

In one embodiment, the RANKL inhibitor is selected from the group consisting of a RANKL specific antibody, a RANKL specific nanobody and osteoprotegerin. In a specific embodiment, the anti-RANKL antibody is a monoclonal antibody. In a yet more specific embodiment, the anti-RANKL antibody is Denosumab (Pageau, Steven C. (2009). mAbs 1 (3): 210-215, CAS number 615258-40-7) (the entire contents of which are hereby incorporated by reference). Denosumab is a fully human monoclonal antibody which binds to RANKL and prevents its activation (it does not bind to the RANK receptor). Various aspects of Denosumab are covered by U.S. Pat. Nos. 6,740,522; 7,411,050; 7,097,834; 7,364,736 (the entire contents of each of which are hereby incorporated by reference in their entirety). In another embodiment, the RANKL inhibitor an antibody, antibody fragment, or fusion construct that binds the same epitope as Denosumab.

In a preferred embodiment, the anti-RANKL nanobody is any of the nanobodies as described in WO2008142164, (the contents of which are incorporated in the present application by reference). In a still more preferred embodiment, the anti-RANKL antibody is the ALX-0141 (Ablynx). ALX-0141 has been designed to inhibit bone loss associated with post-menopausal osteoporosis, reumatoid arthritis, cancer and certain medications, and to restore the balance of healthy bone metabolism.

In a preferred embodiment, the agent preventing the bone degradation is selected from the group consisting of a bisphosphonate, a RANKL inhibitor, PTH and PTHLH inhibitor or a PRG analog, strontium ranelate, a DKK-1 inhibitor, a dual MET and VEGFR2 inhibitor, an estrogen receptor modulator, Radium-223 calcitonin, and a cathepsin K inhibitor. In a more preferred embodiment the agent preventing the bone degradation is a bisphosphonate. In a yet more preferred embodiment, the bisphosphonate is the zoledronic acid.

In one embodiment, a CCR5 antagonist is administered to prevent or inhibit metastasis of the primary breast cancer tumor to bone. In one embodiment, the CCR5 antagonist is a large molecule. In another embodiment, the CCR5 antagonist is a small molecule. In some embodiments, the CCR5 antagonist is Maraviroc (Velasco-Veláquez, M. et al. 2012. CCR5 Antagonist Blocks Metastasis of Basal Breast Cancer Cells. Cancer Research. 72:3839-3850.). In some embodiments, the CCR5 antagonist is Vicriviroc. Velasco-Veláquez, M. et al. 2012. CCR5 Antagonist Blocks Metastasis of Basal Breast Cancer Cells. Cancer Research. 72:3839-3850.). In some aspects, the CCR5 antagonist is Aplaviroc (Demarest J.F. et al. 2005. Update on Aplaviroc: An HIV Entry Inhibitor Targeting CCR5. Retrovirology 2(Suppl. 1): S13). In some aspects, the CCR5 antagonist is a spiropiperidine CCR5 antagonist. (Rotstein D.M. et al. 2009. Spiropiperidine CCR5 antagonists. Bioorganic & Medicinal Chemistry Letters. 19 (18): 5401-5406. In some embodiments, the CCR5 antagonist is INCB009471 (Kuritzkes, D.R. 2009. HIV-1 entry inhibitors: an overview. Curr. Opin. HIV AIDS. 4(2): 82-7).

In a preferred embodiment the dual MET and VEGFR2 inhibitor is selected from the group consisting of Cabozantinib, Foretinib and E7050.

In a preferred embodiment the Radium 223 therapy is alpharadin.

Alternatively a combined treatment can be carried out in which more than one agent from those mentioned above are combined to treat and/or prevent the metastasis or said agents can be combined with other supplements, such as calcium or vitamin D or with a hormone treatment.

### Methods for treating bone metastasis from HER2+ breast cancer, using c-Maf inhibitory agents

In another aspect, the present invention relates to a c-Maf inhibitory agent (hereinafter, inhibitory agent of the present invention) for use in the treatment or prevention of bone metastasis from HER2+ breast cancer.

In another aspect, the present invention relates to the use of a c-Maf inhibitory agent for the manufacture of a medicament for the treatment or prevention of bone metastasis from HER2+ breast cancer.

In another aspect, the present invention relates to a method for the treatment or prevention of bone metastasis from HER2+ breast cancer in a subject in need thereof comprising the administration to said subject of a c-Maf inhibitory agent.

In another aspect, the present invention relates to a method for preventing or reducing the risk of bone metastasis in a subject suffering from HER2+ breast cancer, said method comprising administering to said subject an agent that prevents or reduces bone metastasis, wherein said agent is administered in accordance with a treatment regimen determined from quantifying the expression level of c-Maf in said subject.

By way of non-limiting illustration, c-Maf inhibitory agents suitable for use in the present invention include antisense oligonucleotides, interference RNAs (siRNAs), catalytic RNAs, specific ribozymes, inhibitory antibodies or nanobodies, a dominant negative c-Maf variant or a compound from Table 1 or 2.

### Antisense oligonucleotides

An additional aspect of the present invention relates to the use of isolated "antisense" nucleic acids to inhibit expression, for example, for inhibiting transcription and/or translation of a nucleic acid which encodes c-Maf the activity of which is to be inhibited. The antisense nucleic acids can be bound to the potential target of the drug by means of conventional base complementarity or, for example, in the case of binding to Double stranded DNA through specific interaction in the large groove of the double helix. Generally, these methods refer to a range of techniques generally used in the art and they include any method which is based on the specific binding to oligonucleotide sequences.

An antisense construct of the present invention can be distributed, for example, as an expression plasmid which, when it is transcribed in a cell, produces RNA complementary to at least one unique part of the cellular mRNA encoding c-Maf. Alternatively, the antisense construct is a oligonucleotide probe generated *ex vivo* which, when introduced into the cell, produces inhibition of gene expression hybridizing with the mRNA and/or gene sequences of a target nucleic acid. Such oligonucleotide probes are preferably modified oligonucleotides which are resistant to endogenous nucleases, for example, exonucleases and/or endonucleases and are therefore stable *in vivo.* Examples of nucleic acids molecules for use thereof as antisense oligonucleotides are DNA analogs of phosphoramidate, phosphothionate and methylphosphonate (see also US patent Nos. 5,176,996; 5,264,564; and 5,256,775) (each of which is incorporated herein by reference in its entirety). Additionally, the general approximations for constructing oligomers useful in the antisense therapy have been reviewed, for example, in Van der Krol et al., BioTechniques 6: 958-976, 1988; and Stein et al., Cancer Res 48: 2659-2668, 1988.

With respect to the antisense oligonucleotide, the oligodeoxyribonucleotide regions derived from the starting site of the translation, for example, between about -10 and about +10 of the target gene are preferred. The antisense approximations involve the oligonucleotide design (either DNA or RNA) that are complementary to the mRNA encoding the target polypeptide. The antisense oligonucleotide will be bound to the transcribed mRNA and translation will be prevented.

The oligonucleotides which are complementary to the 5' end of the mRNA, for example the non translated 5' sequence up to and including the start codon AUG must function in the most efficient manner to inhibit translation. Nevertheless, it has been shown recently that the sequences complementary to the non translated 3' sequences of the mRNA are also efficient for inhibiting mRNA translation (Wagner, Nature 372: 333, 1994). Therefore, complementary oligonucleotides could be used at the non translated 5' or 3' regions, non coding regions of a gene in an antisense approximation to inhibit the translation of that mRNA. The oligonucleotides complementary to the non translated 5' region of the mRNA must include the complement of the start codon AUG. The oligonucleotides complementary to the coding region of the mRNA are less efficient translation inhibitors but they could also be used according to the present invention. If they are designed to hybridize with the 5' region, 3' region or the coding region of the mRNA, the antisense nucleic acids must have at least six nucleotides long and preferably have less than approximately 100 and more preferably less than approximately 50, 25, 17 or 10 nucleotides long.

Preferably, *in vitro* studies are performed first to quantify the capacity of the antisense oligonucleotides for inhibiting gene expression. Preferably these studies use controls which distinguish between antisense gene inhibition and non specific biological effects of the oligonucleotides. Also preferably these studies compared the levels of target RNA or protein with that of an internal control of RNA or protein. The results obtained using the antisense oligonucleotides can be compared with those obtained using a control oligonucleotide. Preferably the control oligonucleotide is approximately of the same length as the oligonucleotide to be assayed and the oligonucleotide sequence does not differ from the antisense sequence more than it is deemed necessary to prevent the specific hybridization to the target sequence.

The antisense oligonucleotide can be a single or double stranded DNA or RNA or chimeric mixtures or derivatives or modified versions thereof. The oligonucleotide can be modified in the base group, the sugar group or the phosphate backbone, for example, to improve the stability of the molecule, its hybridization capacity etc. The oligonucleotide may include other bound groups, such as peptides (for example, for directing them to the receptors of the host cells) or agents for facilitating transport through the cell membrane (see, for example, Letsinger et al., Proc. Natl. Acad. Sci. U.S.A. 86: 6553-6556, 1989; Lemaitre et al., Proc. Natl. Acad. Sci. 84: 648-652, 1987; PCT Publication No. WO 88/09810) or the blood-brain barrier (see, for example, PCT Publication No. WO 89/10134), intercalating agents (see, for example, Zon, Pharm. Res. 5: 539-549, 1988). For this purpose, the oligonucleotide can be conjugated to another molecule, for example, a peptide, a transporting agent, hybridization triggered cleaving agent, etc.

The antisense oligonucleotides may comprise at least one group of modified base. The antisense oligonucleotide may also comprise at least a modified sugar group selected from the group including but not limited to arabinose, 2-fluoroarabinose, xylulose, and hexose. The antisense oligonucleotide may also contain a backbone similar to a neutral peptide. Such molecules are known as peptide nucleic acid (PNA) oligomers and are described, for example, in Perry-O'Keefe et al., Proc. Natl. Acad. Sci. U.S.A. 93: 14670, 1996, and in Eglom et al., Nature 365: 566, 1993.

In yet another embodiment, the antisense oligonucleotide comprises at least one modified phosphate backbone. In yet another embodiment, the antisense oligonucleotide is an alpha-anomeric oligonucleotide.

While antisense oligonucleotides complementary to the coding region of the target mRNA sequence can be used, those complementary to the transcribed non translated region can also be used.

In some cases, it may be difficult to reach the sufficient intracellular concentrations of the antisense to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (*i.e.,* the promoter and/or enhancers) to form triple helix structures preventing gene transcription in the target cells in the body (see in general, Helene, Anticancer Drug Des. 6(6): 569-84, 1991). In certain embodiments, the antisense oligonucleotides are antisense morpholines.

### siRNA

Small interfering RNA or siRNA are agents which are capable of inhibiting the expression of a target gene by means of RNA interference. A siRNA can be chemically synthesized, can be obtained by means of *in vitro* transcription or can be synthesized *in vivo* in the target cell. Typically, the siRNA consist of a double stranded RNA between 15 and 40 nucleotide long and may contain a 3' and/or 5' protruding region of 1 to 6 nucleotides. The length of the protruding region is independent of the total length of the siRNA molecule. The siRNA acts by means of degrading or silencing the target messenger after transcription.

The siRNA of the present invention are substantially homologous to the mRNA of the MAF encoding gene or to the gene sequence which encodes said protein. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as:
- siRNA in which the bonds between the nucleotides are different than those that appear in nature, such as phosphorothionate bonds.
- Conjugates of the RNA strand with a functional reagent, such as a fluorophore.
- Modifications of the ends of the RNA strands, particularly of the 3' end by means of the modification with different hydroxyl functional groups in 2' position.
- Nucleotides with modified sugars such as O-alkylated residues on 2' position like 2'-O-methylribose or 2'-O-fluororibose.
- Nucleotides with modified bases such as halogenated bases (for example 5-bromouracil and 5-iodouracil), alkylated bases (for example 7-methylguanosine).

The siRNA can be used as is, *i.e.,* in the form of a double stranded RNA with the aforementioned characteristics. Alternatively, the use of vectors containing the sense and antisense strand sequence of the siRNA is possible under the control of suitable promoters for the expression thereof in the cell of interest.

Vectors suitable for expressing siRNA are those in which the two DNA regions encoding the two strands of siRNA are arranged in tandem in one and the same DNA strand separated by a spacer region which, upon transcription, forms a loop and wherein a single promoter directs the transcription of the DNA molecule giving rise to shRNA.

Alternatively, the use of vectors in which each of the strands forming the siRNA is formed from the transcription of a different transcriptional unit is possible. These vectors are in turn divided into divergent and convergent transcription vectors. In divergent transcription vectors, the transcriptional units encoding each of the DNA strands forming the siRNA are located in tandem in a vector such that the transcription of each DNA strand depends on its own promoter which may be the same or different (Wang, J. et al., 2003, Proc. Natl. Acad. Sci. USA., 100:5103-5106 and Lee, N.S., et al., 2002, Nat. Biotechnol., 20:500-505). In convergent transcription vectors, the DNA regions giving rise to the siRNA form the sense and antisense strands of a DNA region which are flanked by two reverse promoters. After the transcription of the sense and antisense RNA strands, the latter will form the hybrid for forming a functional siRNA. Vectors with reverse promoter systems in which 2 U6 promoters (Tran, N. et al., 2003, BMC Biotechnol., 3:21), a mouse U6 promoter and a human HI promoter (Zheng, L., et al., 2004, Proc. Natl. Acad. Sci. USA., 135-140 and WO 2005026322) and a human U6 promoter and a mouse HI promoter (Kaykas, A. and Moon, R., 2004, BMC Cell Biol., 5:16) are used have been described.

Promoters suitable for use thereof in the expression of siRNA from convergent or divergent expression vectors include any promoter or pair of promoters compatible with the cells in which the siRNA is to be expressed. Thus, promoters suitable for the present invention include but are not necessarily limited to constitutive promoters such as those derived from the genomes of eukaryotic viruses such as the polyoma virus, adenovirus, SV40, CMV, avian sarcoma virus, hepatitis B virus, the metallothionein gene promoter, the thymidine kinase gene promoter of the herpes simplex virus, retrovirus LTR regions, the immunoglobulin gene promoter, the actin gene promoter, the EF-1alpha gene promoter as well as inducible promoters in which the protein expression depends on the addition of a molecule or an exogenous signal such as the tetracycline system, the NFkappaB/UV light system, the Cre/Lox system and the heat shock gene promoter, the regulatable RNA polymerase II promoters described in WO/2006/135436 as well as specific tissue promoters (for example, the PSA promoter described in WO2006012221). In a preferred embodiment, the promoters are RNA polymerase III promoters which act constitutively. The RNA polymerase III promoters are found in a limited number of genes such as 5S RNA, tRNA, 7SL RNA and U6 snRNA. Unlike other RNA polymerase III promoters, type III promoters do not require any intragenic sequence but rather need sequences in 5' direction comprising a TATA box in positions -34 and -24, a proximal sequence element or PSE between -66 and -47 and, in some cases, a distal sequence element or DSE between positions -265 and -149. In a preferred embodiment, the type III RNA polymerase III promoters are the human or murine H1 and U6 gene promoters. In a yet more preferred embodiment, the promoters are 2 human or murine U6 promoters, a mouse U6 promoter and a human HI promoter or a human U6 promoter and a mouse HI promoter. In the context of the present invention, the ER alpha gene promoters or cyclin D1 gene promoters are especially suitable and therefore they are especially preferred to specifically express the genes of interest in breast tumors, preferably in HER2+ breast tumors.

The siRNA can be generated intracellularly from the so called shRNA (short hairpin RNA) characterized in that the antiparallel strands forming the siRNA are connected by a loop or hairpin region. The shRNAs can be encoded by plasmids or viruses, particularly retroviruses, and are under the control of a promoter. Promoters suitable for expressing shRNA are those indicated in the paragraph above for expressing siRNA.

Vectors suitable for expressing siRNA and shRNA include prokaryotic expression vectors such as pUC18, pUC19, Bluescript and the derivatives thereof, mp18, mp19, pBR322, pMB9, CoIEl, pCRl, RP4, phages and shuttle vectors such as pSA3 and pAT28, yeast expression vectors such as 2-micron plasmid type vectors, integration plasmids, YEP vectors, centromeric plasmids and the like, insect cell expression vectors such as pAC series vectors and pVL series vectors, plant expression vectors such as pIBI, pEarleyGate, pAVA, pCAMBIA, pGSA, pGWB, pMDC, pMY, pORE series vectors and the like and viral vector-based (adenovirus, viruses associated with adenoviruses as well as retroviruses and particularly lentiviruses) higher eukaryotic cell expression vectors or non-viral vectors such as pcDNA3, pHCMV/Zeo, pCR3.1, pEFl/His, pIND/GS, pRc/HCMV2, pSV40/Zeo2, pTRACER-HCMV, pUB6/V5-His, pVAXl, pZeoSV2, pCI, pSVL and pKSV-10, pBPV-1, pML2d and pTDT1. In a preferred embodiment, the vectors are lentiviral vectors.

The siRNA and shRNA of the present invention can be obtained using a series of techniques known by the person skilled in the art. The region of the nucleotide sequence taken as a basis for designing the siRNA is not limiting and it may contain a region of the coding sequence (between the start codon and the end codon) or it may alternatively contain sequences of the non-translated 5' or 3' region preferably between 25 and 50 nucleotides long and in any position in 3' direction position with respect to the start codon. One way of designing an siRNA involves the identification of the AA(N19)TT motifs wherein N can be any nucleotide in the MAF gene sequence, and the selection of those having a high G/C content. If said motif is not found, it is possible to identify the NA(N21) motif wherein N can be any nucleotide.

MAF specific siRNAs include the siRNA described in WO2005046731, one of the strands of which is ACGGCUCGAGCAGCGACAA (SEQ ID NO: 6). Other MAF specific siRNA sequences include, but are not limited to, CUUACCAGUGUGUUCACAA (SEQ ID NO: 7), UGGAAGACUACUACUGGAUG (SEQ ID NO: 8), AUUUGCAGUCAUGGAGAACC (SEQ ID NO: 9), CAAGGAGAAAUACGAGAAGU (SEQ ID NO: 10), ACAAGGAGAAAUACGAGAAG (SEQ ID NO: 11) and ACCUGGAAGACUACUACUGG (SEQ ID NO: 12).

### DNA Enzymes

On the other hand, the present invention also contemplates the use of DNA enzymes to inhibit the expression of the MAF gene of the present invention. DNA enzymes incorporate some of the mechanistic features of both antisense and ribozyme technologies. DNA enzymes are designed such that they recognize a particular target nucleic acid sequence similar to the antisense oligonucleotide, nevertheless like the ribozyme they are catalytic and specifically cleave the target nucleic acid.

### Ribozymes

Ribozyme molecules designed for catalytically cleaving transcription products of a target mRNA to prevent the translation of the mRNA which encodes MAF the activity of which is to be inhibited, can also be used. Ribozymes are enzymatic RNA molecules capable of catalyzing specific RNA cleaving (For a review, see, Rossi, Current Biology 4: 469-471, 1994). The mechanism of ribozyme action involves a specific hybridization of a ribozyme molecule sequence to a complementary target RNA followed by an endonucleolytic cleavage event. The composition of the ribozyme molecules preferably includes one or more sequences complementary to the target mRNA and the well known sequence responsible for cleaving the mRNA or a functionally equivalent sequence (see, for example, US patent No. 5093246).

The ribozymes used in the present invention include hammer-head ribozymes, endoribonuclease RNA (hereinafter "Cech type ribozymes") (Zaug et al., Science 224:574-578, 1984.

The ribozymes can be formed by modified oligonucleotides (for example to improve the stability, targeting, etc.) and they should be distributed to cells expressing the target gene *in vivo.* A preferred distribution method involves using a DNA construct which "encodes" the ribozyme under the control of a strong constitutive pol III or pol II promoter such that the transfected cells will produce sufficient amounts of the ribozyme to destroy the endogenous target messengers and to inhibit translation. Since the ribozymes are catalytic, unlike other antisense molecules, a low intracellular concentration is required for its efficiency.

### Inhibitory antibodies

In the context of the present invention, "inhibitory antibody" is understood as any antibody capable of binding specifically to the c-Maf protein and inhibiting one or more of the functions of said protein, preferably those related to transcription. The antibodies can be prepared using any of the methods which are known by the person skilled in the art, some of which have been mentioned above. Thus, the polyclonal antibodies are prepared by means of immunizing an animal with the protein to be inhibited. The monoclonal antibodies are prepared using the method described by Kohler, Milstein et al. (Nature, 1975, 256: 495). In the context of the present invention, suitable antibodies include intact antibodies comprising a variable antigen binding region and a constant region, "Fab", "F(ab')2" and "Fab'", Fv, scFv fragments, diabodies, bispecific antibodies, alphabodies, cyclopeptides and stapled peptides. Once antibodies with c-Maf protein binding capacity are identified, those capable of inhibiting the activity of this protein will be selected using an inhibitory agent identification assay.

### Inhibitory peptides

As used herein, the term "inhibitory peptide" refers to those peptides capable of binding to the c-M protein and inhibiting its activity as has been explained above, *i.e.,* preventing the c-Maf from being able to activate gene transcription.

### Negative c-Maf dominants

Since the proteins from the MAF family are capable of homodimerizing and heterodimerizing with other members of the AP-1 family such as Fos and Jun, one way of inhibiting c-Maf activity is by means of using negative dominants capable of dimerizing with c-Maf but lacking the capacity for activating transcription. Thus, the negative c-Maf dominants can be any of the small maf proteins existing in the cell and lacking two-thirds of the amino terminal end containing the transactivation domain (for example, mafK, mafF, mafg and pi 8) (Fujiwara et al (1993) Oncogene 8, 2371-2380; Igarashi et al. (1995) J. Biol.Chem. 270, 7615-7624; Andrews et al. (1993) Proc. Natl. Acad. Sci. USA 90, 11488-11492; Kataoka et al. (1995) Mol. Cell. Biol. 15, 2180-2190) (Kataoka et al. (1996) Oncogene 12, 53-62).

Alternatively, the negative c-Maf dominants include c-Maf variants which maintain the capacity for dimerizing with other proteins but lack the capacity for activating transcription. These variants are, for example, those lacking the c-Maf transactivation domain located at the N-terminal end of the protein. Thus, negative c-Maf dominant variants include in an illustrative manner the variants in which at least amino acids 1 to 122, at least amino acids 1-187 or at least amino acids 1 to 257 (by considering the numbering of human MAF as described in US6274338) have been removed.

The present invention contemplates the use of both the negative c-Maf dominant variants and of polynucleotides encoding MAF under the operative control of a promoter suitable for expression in target cell. The promoters that can be used for regulating the polynucleotide transcription of the present invention can be constitutive promoters, *i.e.,* promoters directing the transcription at a basal level, or inducible promoters in which the transcriptional activity requires an external signal. Constitutive promoters suitable for regulating transcription are, among others, the CMV promoter, the SV40 promoter, the DHFR promoter, the mouse mammary tumor virus (MMTV) promoter, the 1a elongation factor (EFla) promoter, the albumin promoter, the ApoA1 promoter, the keratin promoter, the CD3 promoter, the immunoglobulin heavy or light chain promoter, the neurofilament promoter, the neuron specific enolase promoter, the L7 promoter, the CD2 promoter, the myosin light chain kinase promoter, the HOX gene promoter, the thymidine kinase promoter, the RNA polymerase II promoter, the MyoD gene promoter, the phosphoglyceratekinase (PGK) gene promoter, the low density lipoprotein (LDL) promoter, the actin gene promoter. In a preferred embodiment, the promoter regulating the expression of the transactivator is the PGK gene promoter. In a preferred embodiment, the promoter regulating the polynucleotide transcription of the present invention is the RNA polymerase promoter of the T7 phage.

Preferably, the inducible promoters that can be used in the context of the present invention are those responding to an inducer agent showing zero or negligible basal expression in the absence of an inducer agent and are capable of promoting the activation of gene located in the 3' position. Depending on the type of inducer agent, the inducible promoters are classified as Tet on/off promoters (Gossen, M. and H. Bujard (1992) Proc. Natl. Acad. Sci. USA, 89:5547-5551; Gossen, M. et al., 1995, Science 268:1766-1769; Rossi, F.M.V. and H.M. Blau, 1998, Curr. Opin. Biotechnol. 9:451-456); Pip on/off promoters (US 6287813); antiprogestin-dependent promoters (US 2004132086), ecdysone-dependent promoters (Christopherson et al., 1992, Proc. Natl. Acad. Sci. USA, 89:6314-6318; No et al., 1996, Proc. Natl. Acad. Sci. USA, 93:3346-3351, Suhr et al., 1998, Proc. Natl. Acad. Sci. USA, 95:7999-8004 and WO9738117), a metallothionein-dependent promoter (WO8604920) and rapamycin-dependent promoters (Rivera et al., 1996, Nat. Med. 2:1028-32).

Vectors suitable for expressing the polynucleotide encoding the negative c-Maf dominant variant include vectors derived from prokaryotic expression vectors such as pUC18, pUC19, Bluescript and derivatives thereof, mp18, mp19, pBR322, pMB9, ColEl, pCR1, RP4, phages and shuttle vectors such as pSA3 and pAT28, yeast expression vectors such as 2-micron type plasmid vectors, integration plasmids, YEP vectors, centromeric plasmids and the like, insect cell expression vectors such as pAC series vectors and pVL series vectors, plant expression vectors such as pIBI, pEarleyGate, pAVA, pCAMBIA, pGSA, pGWB, pMDC, pMY, pORE series vectors and the like and viral vector-based (adenoviruses, viruses associated with adenoviruses as well as retroviruses and particularly lentiviruses) higher eukaryotic cell expression vectors OR non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDTl.

### Small molecules

Other c-Maf inhibitory compounds suitable for use in the present invention include:

**Table 1: Small molecules with c-Maf inhibiting capacity**

| | |
|---|---|
| I | Endiandric acid H derivatives such as those described in WO2004014888 corresponding to the general formula |
| | 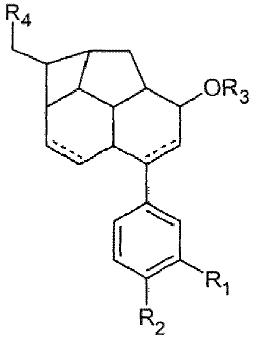 |
| | wherein |
| | R₁ and R₂ are, independently of one another, |
| | 1.0 H or |
| | 2.0 a O-C₁-C₆-alkyl, -O-C₂-C₆-alkenyl, -O-C₂-C₆-alkynyl or -O-C₆-C₁₀-aryl group, in which alkyl, alkenyl and alkynyl are straight-chain or branched, and in which the alkyl, alkenyl and alkynyl groups are mono- or disubstituted with: |
| | 2.1 -OH, |
| | 2.2 =O, |
| | 2.3 -O-C₁-C₆-alkyl, in which alkyl is straight-chain or branched, |
| | 2.4 -O-C₂-C₆-alkenyl, in which alkenyl is straight-chain or branched, |
| | 2.5 C₆-C₁₀-aryl, |
| | 2.6 -NH-C₁-C₆-alkyl, in which alkyl is straight-chain or branched, |
| | 2.7 -NH-C₂-C₆-alkenyl, in which alkenyl is straight-chain or branched, |
| | 2.8 -NH₂ or |
| | 2.9 halogen, |
| | and in which the aryl group, is optionally mono- or disubstituted with the substituent 2.1 or 2.3 to 2.9, |
| | in which the substituents 2.3, 2.4, 2.6 and 2.7 may be further substituted with - CN, -amide or -oxime functions, and 2.5 may be further substituted with -CN or amide functions, or R₁ and R₂ together form a ring, wherein R₁ and R₂ mean a - O-[(C₁-C₆)-alkylene]-O-group, |
| | R₃ is |
| | 1.0 H or |
| | 2.0 a -O-C₁-C₆-alkyl, -O-C₂-C₆-alkenyl, -O-C₂-C₆-alkynyl or -O-C₆-C₁₀-aryl group, in which alkyl, alkenyl and alkynyl are straight-chain or branched, and in which the alkyl, alkenyl and alkynyl groups are mono-or disubstituted with: |
| | 2.1 -OH, |
| | 2.2 =O, |
| | 2.3 -O-C₁-C₆-alkyl, in which alkyl is straight-chain or branched, |
| | 2.4 -O-C₂-C₆-alkenyl, in which alkenyl is straight-chain or branched, |
| | 2.5 -C₆-C₁₀-aryl, |
| | 2.6 -NH-C₁-C₆-alkyl, in which alkyl is straight-chain or branched, |
| | 2.7 -NH-C₂-C₆-alkenyl, in which alkenyl is straight-chain or branched, |
| | 2.8 -NH₂ or |
| | 2.9 halogen, |
| | and in which the aryl group, is optionally mono-or disubstituted with the substituent 2.1 or 2.3 to 2.9, |
| | in which the substituents 2.3, 2.4, 2.6 and 2.7 may be further substituted with - CN, -amide or -oxime functions, and 2.5 may be further substituted with -CN or amide functions |
| | R₄ is CO₂R₃, CO₂NHR₃, CHO, CH₂OR₃, CH₂OSi(R₃)₃, CH₂Br, CH₂CN, in which R₃ is as defined above, |
| | and, in particular, the compounds |
| | 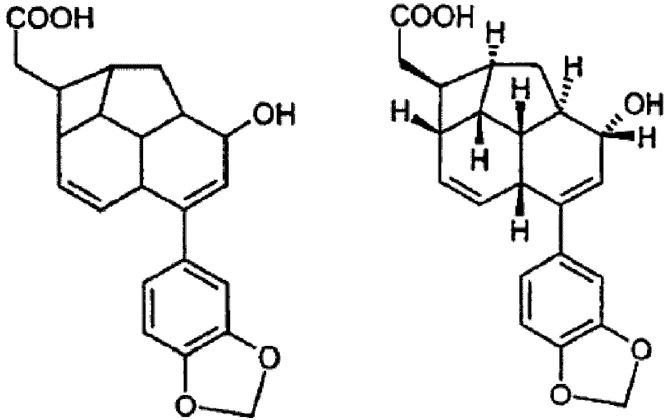 |
| II | 8-hydroxyquinoline derivatives such as those described in WO2009146546 of general formula |
| | 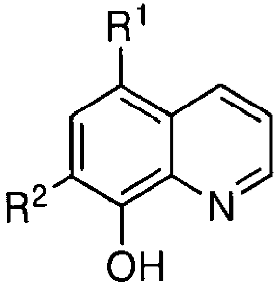 |
| | wherein |
| | R₁ is selected from the group consisting of NO₂, NH₂, NH(C₁-C₆-alkyl) and N(C₁-C₆-alkyl)(C₁-C₆-alkyl); |
| | R₂ is selected from H, halogen, C₁-C₆ alkyl, and fluoro-substituted C₁-C₆ alkyl, or |
| | R₁ is C1 and R₂ is Br or H, |
| | and, preferably, the compounds |
| | 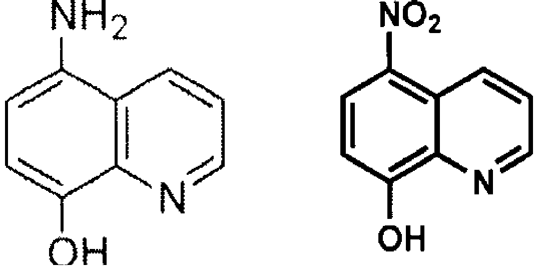 |
| | 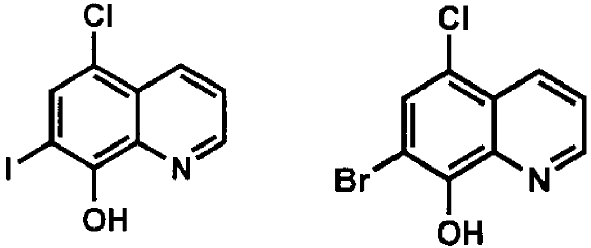 |
| | 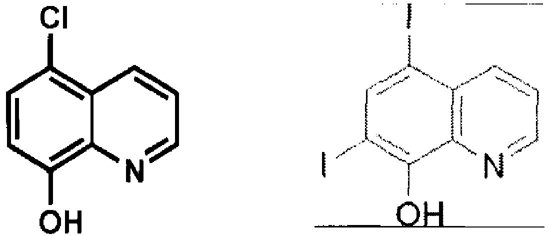 |
| III | Clioquinol (5-chloro-7-iodoquinolin-8-ol) as described in WO09049410 |
| IV | Compounds such as those described in WO08098351 of general formula |
| | 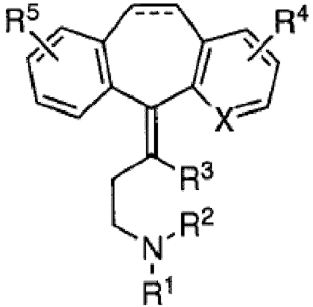 |
| | wherein |
| | ==-:-:-: is a single or double bond, |
| | R¹ is selected from the group consisting of H, C₁-C₄ alkyl, C(O)O C₁-C₄ alkyl, |
| | C(O) C₁-C₄ alkyl and C(O)NH C₁-C₄ alkyl; |
| | R² is selected from H and C₁-C₄ alkyl; |
| | R³ is selected from H and C₁-C₄ alkyl; |
| | or R² and R³ are bound together along with the carbon and nitrogen atoms to which they are bound to form a piperidine ring, |
| | R⁴ and R⁵ are independently selected from H, halogen, hydroxy, C₁-C₄ alkyl, fluoro-substituted C₁-C₄ alkyl and C₁-C₄ alkoxy; and |
| | X is selected from C and N, |
| | and preferred compounds such as |
| | Cyproheptadine (4-(5H-dibenzo-[a,d]cyclohepten-5-ylidene)-1-methylpiperidine hydrochloride), |
| | Amitriptyline (3-(10,11-dihydro-5H-dibenzo[[*a*,*d*]]cycloheptene-5-ylidene)-N,N-dimethyl-1-propanamine), |
| | Loratadine (Ethyl 4-(8-chloro-5,6-dihydro-11H-benzo[5,6]cyclohepta[1,2-b]pyridin-11-ylidene)-1-piperidinecarboxylate, |
| | Cyclobenzrapine (3-(5H-dibenzo[a,d]cyclohepten-5-ylidene)-N,N-dimethyl-1-propanamine). |
| V | Nivalenol (12,13-epoxy-3,4,7,15-tetrahydroxytrichothec-9-en-8-one) as described in WO0359249 |

Other c-Maf inhibitors are described in the patent application WO2005063252 (incorporated by reference herein in its entirety), such as shown in the following table (Table 2).

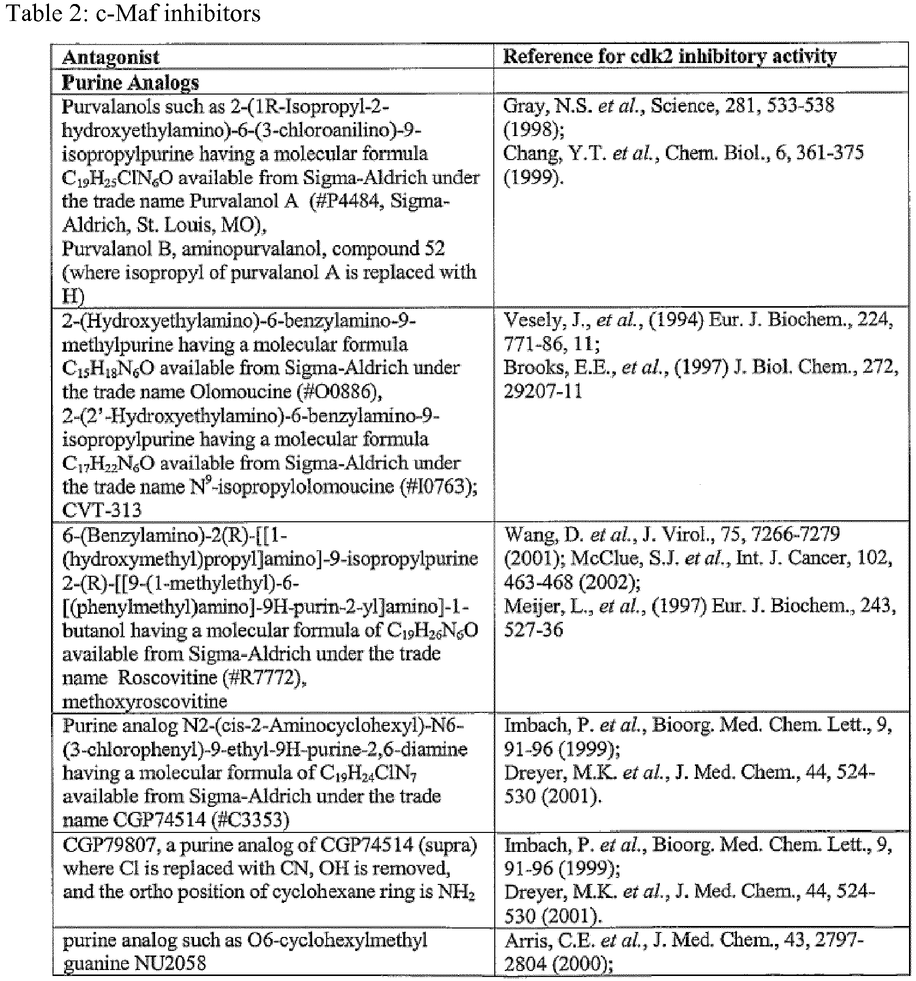

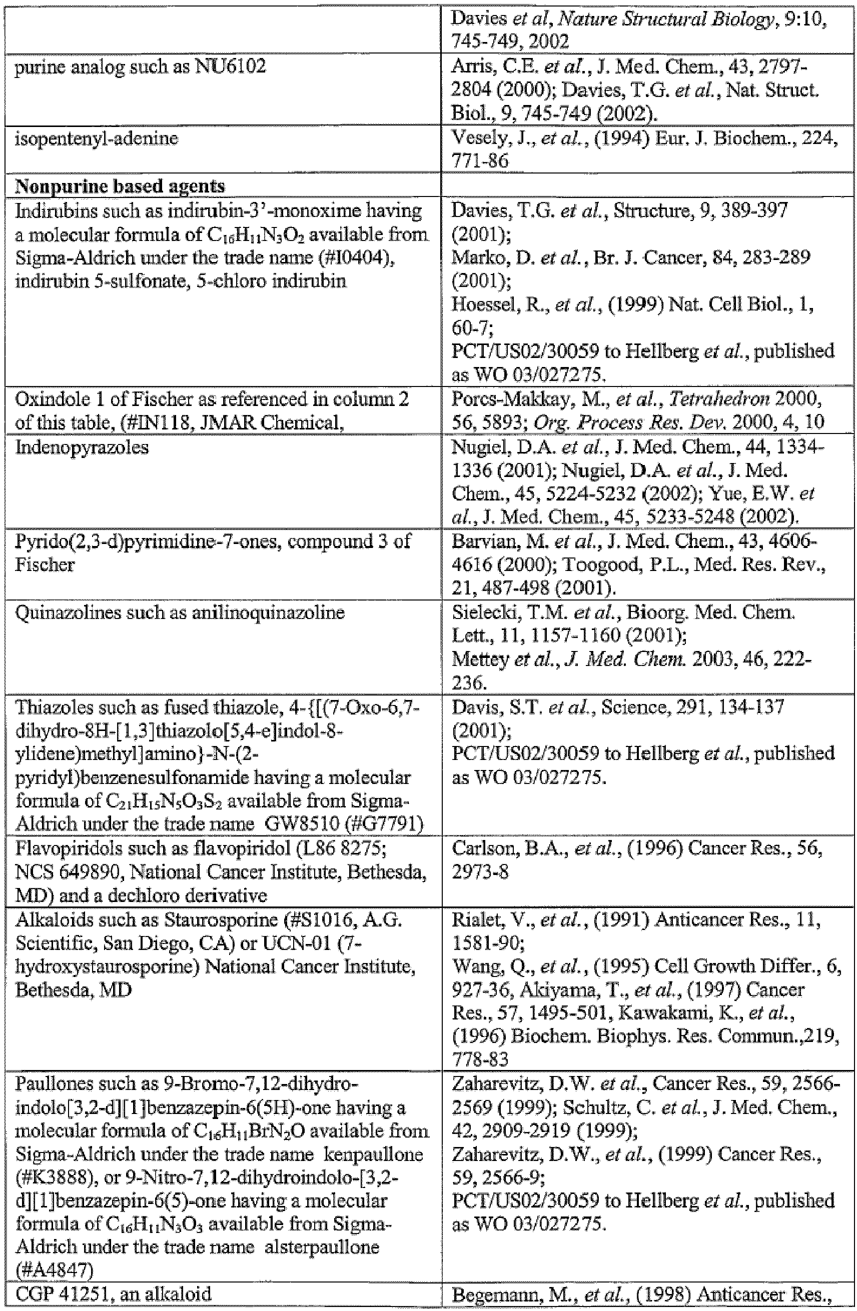

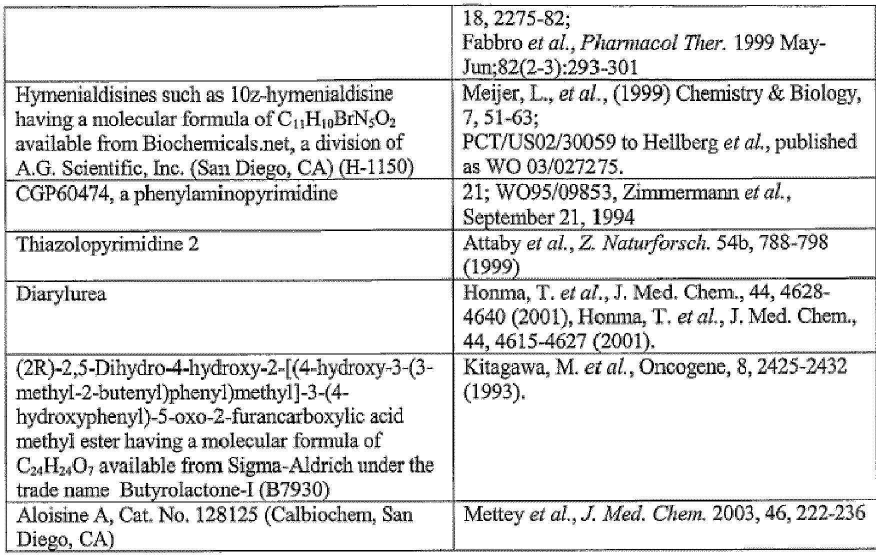

In a preferred embodiment, the bone metastasis is osteolytic metastasis.

The c-Maf inhibitory agents are typically administered in combination with a pharmaceutically acceptable carrier.

The term "carrier" refers to a diluent or an excipient whereby the active ingredient is administered. Such pharmaceutical carriers can be sterile liquids such as water and oil, including those of a petroleum, animal, plant or synthetic origin such as peanut oil, soy oil, mineral oil, sesame oil and the like. Water or aqueous saline solutions and aqueous dextrose and glycerol solutions, particularly for injectable solutions, are preferably used as carriers. Suitable pharmaceutical carriers are described in "Remington's Pharmaceutical Sciences" by E.W. Martin, 1995. Preferably, the carriers of the present invention are approved by the state or federal government regulatory agency or are listed in the United States Pharmacopeia or other pharmacopeia generally recognized for use thereof in animals and more particularly in human beings.

The carriers and auxiliary substances necessary for manufacturing the desired pharmaceutical dosage form of the pharmaceutical composition of the present invention will depend, among other factors, on the pharmaceutical dosage form chosen. Said pharmaceutical dosage forms of the pharmaceutical composition will be manufactured according to the conventional methods known by the person skilled in the art. A review of the different methods for administering active ingredients, excipients to be used and processes for producing them can be found in "Tratado de Farmacia Galenica", C. Faulí i Trillo, Luzán 5, S.A. 1993 Edition. Examples of pharmaceutical compositions include any solid composition (tablets, pills, capsules, granules, etc.) or liquid composition (solutions, suspensions or emulsions) for oral, topical or parenteral administration. Furthermore, the pharmaceutical composition may contain, as deemed necessary, stabilizers, suspensions, preservatives, surfactants and the like.

For use in medicine, the c-Maf inhibitory agents can be found in the form of a prodrug, salt, solvate or clathrate, either isolated or in combination with additional active agents and can be formulated together with a pharmaceutically acceptable excipient. Excipients preferred for use thereof in the present present invention include sugars, starches, celluloses, rubbers and proteins. In a particular embodiment, the pharmaceutical composition of the present invention will be formulated in a solid pharmaceutical dosage form (for example tablets, capsules, pills, granules, suppositories, sterile crystal or amorphous solids that can be reconstituted to provide liquid forms, etc.), liquid pharmaceutical dosage form (for example solutions, suspensions, emulsions, elixirs, lotions, ointments, etc.) or semisolid pharmaceutical dosage form (gels, ointments, creams and the like). The pharmaceutical compositions of the present invention can be administered by any route, including but not limited to the oral route, intravenous route, intramuscular route, intraarterial route, intramedularry route, intrathecal route, intraventricular router, transdermal route, subcutaneous route, intraperitoneal route, intranasal route, enteric route, topical route, sublingual route or rectal route. A review of the different ways for administering active ingredients, of the excipients to be used and of the manufacturing processes thereof can be found in Tratado de Farmacia Galénica, C. Faulí i Trillo, Luzán 5, S.A., 1993 Edition and in Remington's Pharmaceutical Sciences (A.R. Gennaro, Ed.), 20th edition, Williams & Wilkins PA, USA (2000). Examples of pharmaceutically acceptable carriers are known in the state of the art and include phosphate buffered saline solutions, water, emulsions such as oil/water emulsions, different types of wetting agents, sterile solutions, etc. The compositions comprising said carriers can be formulated by conventional processes known in the state of the art.

In the event that nucleic acids (siRNA, polynucleotides encoding siRNA or shRNA or polynucleotides encoding negative MAF dominants) are administered, the present invention contemplates pharmaceutical compositions particularly prepared for administering said nucleic acids. The pharmaceutical compositions can comprise said naked nucleic acids, *i.e.,* in the absence of compounds protecting the nucleic acids from degradation by the nucleases of the body, which entails the advantage that the toxicity associated with the reagents used for transfection is eliminated. Administration routes suitable for naked compounds include the intravascular route, intratumor route, intracranial route, intraperitoneal route, intrasplenic route, intramuscular route, subretinal route, subcutaneous route, mucosal route, topical route and oral route (Templeton, 2002, DNA Cell Biol., 21:857-867). Alternatively, the nucleic acids can be administered forming part of liposomes conjugated to cholesterol or conjugated to compounds capable of promoting the translocation through cell membranes such as the Tat peptide derived from the HIV-1 TAT protein, the third helix of the homeodomain of the *D. melanogaster* antennapedia protein, the herpes simplex virus VP22 protein, arginine oligomers and peptides as described in WO07069090 (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol Therapy 8:143-150 and Snyder, E.L. and Dowdy, S.F., 2004, Pharm. Res. 21:389-393). Alternatively, the polynucleotide can be administered forming part of a plasmid vector or viral vector, preferably adenovirus-based vectors, in adeno-associated viruses or in retroviruses such as viruses based on murine leukemia virus (MLV) or on lentivirus (HIV, FIV, EIAV).

The c-Maf inhibitory agents or the pharmaceutical compositions containing them can be administered at a dose of less than 10 mg per kilogram of body weight, preferably less than about , about 2, about 1, about 0.5, about 0.1, about 0.05, about 0.01, about 0.005, about 0.001, about 0.0005, about 0.0001, about 0.00005 or about 0.00001 mg per kg of body weight. The unit dose can be administered by injection, inhalation or topical administration.

The dose depends on the severity and the response of the condition to be treated and it may vary between several days and months or until the condition subsides. The optimal dosage can be determined by periodically measuring the concentrations of the agent in the body of the patient. The optimal dose can be determined from the EC50 values obtained by means of previous *in vitro* or *in vivo* assays in animal models. The unit dose can be administered once a day or less than once a day, preferably less than once about every 2, about every 4, about every 8 or about every 30 days. Alternatively, it is possible to administer a starting dose followed by one or several maintenance doses, generally of a lesser amount than the starting dose. The maintenance regimen may involve treating the patient with a dose ranging between about 0.01 µg and about 1.4 mg/kg of body weight per day, for example about 10, about 1, about 0.1, about 0.01, about 0.001, or about 0.00001 mg per kg of body weight per day. The maintenance doses are preferably administered at the most once about every 5, about every 10 or about every 30 days. The treatment must be continued for a time that will vary according to the type of disorder the patient suffers, the severity thereof and the condition of the patient. After treatment, the progress of the patient must be monitored to determine if the dose should be increased in the event that the disease does not respond to the treatment or the dose is reduced if an improvement of the disease is observed or if unwanted side effects are observed.

### Treatment or prevention of the bone degradation in breast cancer patients with bone metastasis having elevated MAF levels

In another aspect, the present invention relates to a c-Maf inhibitory agent or an agent capable of avoiding or preventing bone degradation for use in the treatment of bone metastasis in a subject suffering from HER2+ breast cancer, and having elevated MAF levels in a metastatic sample with respect to a control sample.

In another aspect, the present invention relates to the use of a c-Maf inhibitory agent or an agent capable of avoiding or preventing bone degradation for the manufacture of a medicament for the treatment of bone metastasis in a subject suffering HER2+ breast cancer, and having elevated MAF levels in a metastatic sample with respect to a control sample.

Alternatively, the present invention relates to a method of prevention and/or treatment of the degradation in a subject suffering HER2+ breast cancer and having elevated MAF levels in a metastatic sample with respect to a control sample, which comprises administering a c-Maf inhibitory agent or an agent for avoiding or preventing bone degradation to said subject.

In a particular embodiment the bone metastasis is osteolytic metastasis.

c-Maf inhibitory agents and agents capable of avoiding or preventing bone degradation suitable for the therapeutic method described in the present invention have been described in detail above in the context of the customized therapy method.

The reference or control sample is a sample of a subject with HER2+ breast cancer, who has not suffered metastasis or that corresponds to the median value of the MAF gene expression level measured in a tumor tissue collection in biopsy samples of subjects with HER2+ breast cancer who have not suffered metastasis.

Methods for determining or quantifying if the MAF levels are elevated with respect to a control sample have been described in detail in relation to the first method of the present invention and are equally applicable to the agent for avoiding or preventing bone degradation.

Alternatively, a combined treatment can be carried out, in which more than one agent for avoiding or preventing bone degradation from those mentioned above are combined to treat and/or prevent the metastasis or said agents can be combined with other supplements, such as calcium or vitamin D or with a hormone.

The agents for avoiding or preventing bone degradation are typically administered in combination with a pharmaceutically acceptable carrier. The term "carrier" and the types of carriers have been defined above for the c-Maf inhibitory agent, as well as the form and the dose in which they can be administered and are equally applicable to the agent for avoiding or preventing bone degradation.

The following examples illustrate the present invention and do not limit the scope thereof.

### Kits of the present invention

In another aspect, the present invention relates to a kit for predicting bone metastasis of a HER2+ breast cancer, in a subject suffering from said cancer, the kit comprising: a) means for quantifying the expression level of MAF in a sample of said subject; and b) means for comparing the quantified level of expression of MAF in said sample to a reference MAF expression level.

In another aspect, the present invention relates to a kit for predicting the clinical outcome of a subject suffering from bone metastasis from a HER2+ breast cancer, the kit comprising: a) means for quantifying the expression level of MAF in a sample of said subject; and b) means for comparing the quantified expression level of MAF in said sample to a reference MAF expression level.

In another aspect the present invention relates to a kit for determining a therapy for a subject suffering from HER2+ breast cancer, the kit comprising: a) means for quantifying the expression level of MAF in a sample of said subject; b) means for comparing the quantified expression level of MAF in said sample to a reference MAF expression level; and c) means for determining a therapy for preventing and/or reducing bone metastasis in said subject based on the comparison of the quantified expression level to the reference expression level.

In another aspect the present invention relates to a kit comprising: i) a reagent for quantifying the expression level of MAF in a sample of a subject suffering from HER2+ breast cancer, and ii) one or more MAF gene expression level indices that have been predetermined to correlate with the risk of bone metastasis.

Means for quantifying the expression level of MAF in a sample of said subject have been previously described in detail including 16q23 and 16q22-24 locus amplification and translocation.

In a preferred embodiment, means for quantifying expression comprise a set of probes and/or primers that specifically bind and/or amplify the MAF gene.

In particular embodiment the breast cancer is HER2+ breast cancer.

All the particular embodiments of the methods of the present invention are applicable to the kits of the present invention and to their uses.

### Method for typing a sample of a subject suffering breast cancer

In another aspect, the present invention relates to an *in vitro* method for typing a sample of a subject suffering from breast cancer, the method comprising:
a) providing a sample from said subject;
b) quantifying the expression level of MAF in said sample;
c) typing said sample by comparing the quantified expression level of MAF to a predetermined reference level of MAF expression;
wherein said typing provides prognostic information related to the risk of bone metastasis in said subject.

Means for quantifying the expression level of MAF in a sample of said subject have been previously described in detail including 16q23 and 16q22-24 locus amplification and translocation.

In particular embodiment the breast cancer is HER2+ breast cancer.

In a preferred embodiment the sample is a tumor tissue sample.

### Method for classifying a subject suffering from breast cancer

In another aspect, the present invention relates to a method for classifying a subject suffering from breast cancer into a cohort, comprising: a) determining the expression level of MAF in a sample of said subject; b) comparing the expression level of MAF in said sample to a predetermined reference level of MAF expression; and c) classifying said subject into a cohort based on said expression level of MAF in the sample.

Means for quantifying the expression level of MAF in a sample of said subject have been previously described in detail including 16q23 and 16q22-24 locus amplification and translocation.

In particular embodiment the breast cancer is HER2+ breast cancer.

In a preferred embodiment the sample is a tumor tissue sample.

In a preferred embodiment said cohort comprises at least one other individual who has been determined to have a comparable expression level of MAF in comparison to said reference expression level.

In another preferred embodiment said expression level of MAF in said sample is increased relative to said predetermined reference level, and wherein the members of the cohort are classified as having increased risk of bone metastasis.

In another preferred embodiment said cohort is for conducting a clinical trial. In a preferred embodiment, the sample is a tumor tissue sample.

### EXAMPLES

### EXAMPLE 1

### Experimental procedures and data sets

### Breast cancer primary tumor sample cohort:

The breast cancer cohort used was composed of more than 334 primary breast cancer specimens from patients with stage I, II or III BC and clinical annotated follow up, including site of metastasis (Rojo F., Ann Oncol (2012) 23 (5): 1156-1164). Tissue microarrays were processed as per standard procedures. Breast cancer tumors were classified in various subtypes including ER+, Triple Negative and HER2+ and then the appropriate statistical analyses were performed to test if MAF (MAF) expression and the 16q22-24 amplification in these tumors correlates with bone metastasis events in the subtypes of interest.

The Statistical analyses in this cohort were based on the following premises:

### A) Cumulative incidence

For figure 1b p-values were obtained after fitting Cox cause-specific proportional hazard models with competing events (death before bone metastasis at any time) and performing likelihood ratio tests.

### B) Comparison of baseline characteristics (Table S1).

Differences in age were tested with Student's t test. All other variables were tested with Fisher's exact test.

### Validation of the prognostic capacity to predict bone metastasis of the 16q22-24 DNA genomic amplification by FISH determination.

To further validate the ability of 16q22-24 genomic amplification to specifically predict bone metastasis risk, we analyzed 16q22-24 chromosome region genomic gain by means of FISH (we used a commercially available diagnostic probe that determines the 16q23 genomic region, IGH/MAF Abbot Vysis probe, and normalized the number of 16q23 copies using a centromeric chr 16 probe, 16q11.2 (CEP16)) in a set composed of 334 primary breast cancer specimens from patients with stage I, II or III BC and annotated follow up (Rojo F., Ann Oncol (2012) 23 (5): 1156-1164). Tissue microarrays were processed as per standard procedures. The slides were incubated with MAF (16q23) and independently stained using CEP16 probe. DAPI counterstain was applied and images were acquired with adequate microscope. The patients were stratified according to 16q23/CEP16 <1.5 FISH as negative and 16q23/CEP16 >1.5 FISH as positive group based on the average of 50 cells per tumor (Figure 1a).

Cumulative incidence plot (Figure 1b) for bone metastasis at any time (continuous line) and death before recurrence in bone (dashed line) in stage I, II, and III BC human primary tumor set (n=334) was determined and showed statistical significance.

### Determination of Treatment Regimen in Subject Diagnosed with HER2+ Based on MAF Expression Levels

A tumor tissue sample is obtained from a subject diagnosed as having HER2+ breast cancer. The sample is sectioned into thin slices of tissue and embedded in paraffin. Each paraffin section is mounted on a slide. The slides are incubated with anti-MAF antibody. For visualization and detection of antibodies bound to MAF, antibodies conjugated with fluorescent dye are used. The slides are visualized by providing excitation beams to the fluorescent dyes. Images of fluorescent signals are taken by fluorescent microscopes. The relative expression level of MAF in the tumor sample is obtained by comparing the fluorescent signal in the tumor sample to that of a reference sample. The intensity in the tumor sample is correlated with the intensity in the reference sample, wherein a higher intensity in the tumor sample compared to the reference sample correlates with an increased risk of the subject having primary breast cancer metastasis to the bone. Alternatively, 16q22-24 locus, 16q23 locus or MAF gene amplification or translocation is determined using an in situ hybridization technique or similar

Based on the prognosis of increased risk of bone metastasis, the subject is administered the anti-RANKL antibody Denosumab as a prophylactic treatment for bone metastasis. 120mg of Denosumab is administered to the subject subcutaneously (SC) once monthly for 6 months. 120mg SC every 3 months for the next 4 and a half years. Oral calcium (at least 500 mg) and vitamin D (at least 400 IU) for 5 years. After 5 years, the subject is free of any evidence of bone-metastasis. Based on the prognosis of a lack of an increased risk of bone metastasis the patient is not administered this anti-RANKL antibody.

It is understood that the examples and embodiments described herein are for illustrative purposes only and that various modifications or changes in light thereof will be suggested to persons skilled in the art and are to be included with the spirit and purview of this application.

All publications, patents, patent applications, internet sites, and accession numbers/database sequences including both polynucleotide and polypeptide sequences cited herein are hereby incorporated by reference herein in their entirety for all purposes to the same extent as if each individual publication, patent, patent application, internet site, or accession number/database sequence were specifically and individually indicated to be so incorporated by reference.

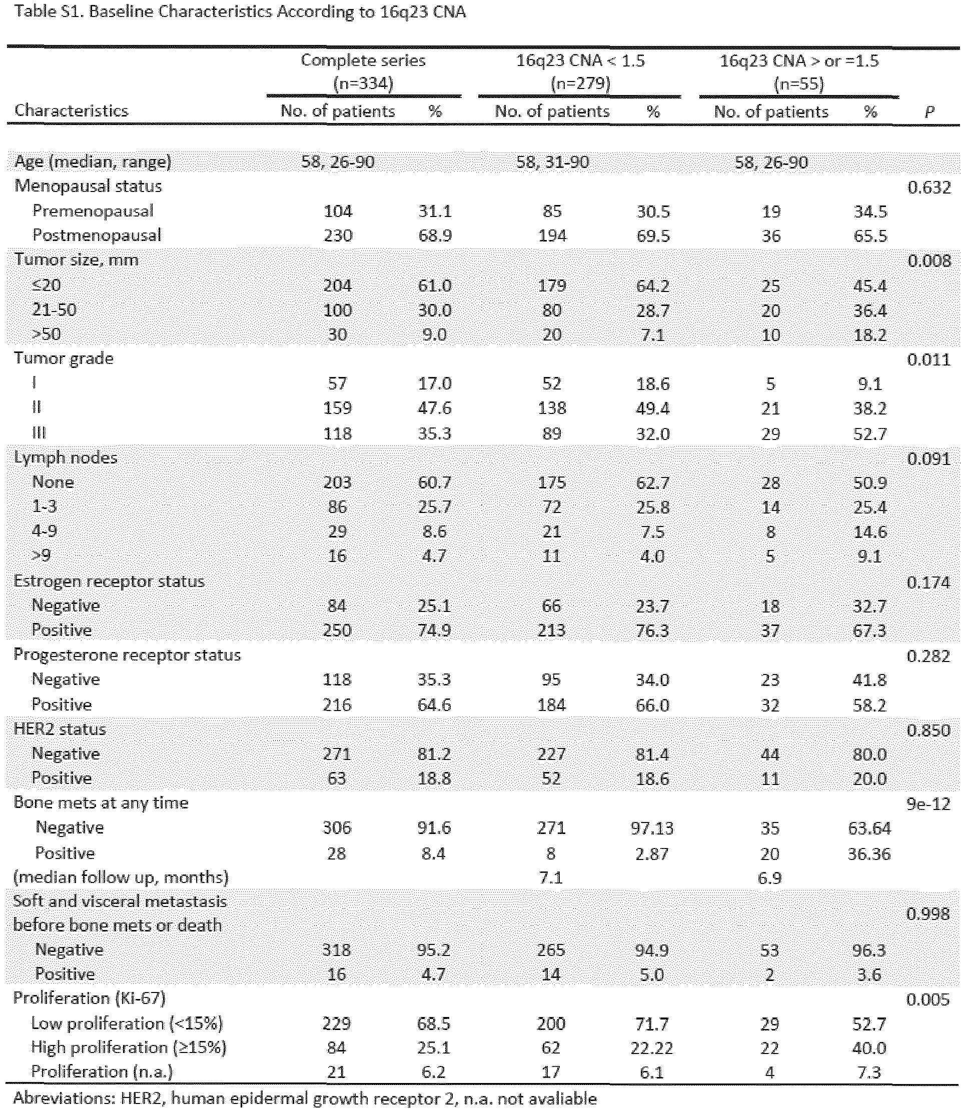

## Claims

1. An *in vitro* method for designing a customized therapy for a subject suffering HER2+ breast cancer which comprises
i) quantifying the c-MAF gene expression level, amplification or copy number in a HER2+ sample of said subject and
ii) comparing the expression level, amplification or copy number obtained in i) with a reference value,
wherein if the expression level, copy number, or degree of amplification is increased with respect to said reference value, then said subject is susceptible to receive clodronate, wherein if the expression level, copy number or degree of amplification is not increased with respect to said reference value, then said subject is not susceptible to receive clodronate.

2. An *in vitro* method for predicting the risk of bone metastasis and/or clinical outcome of a HER2+ breast cancer in a subject suffering from said cancer which comprises
ii) determining the expression level of the c-MAF gene in a HER2+ sample of said subject and
iii) comparing the expression level obtained in step i) with a reference value,
wherein an increased expression level of said gene with respect to said reference value is indicative of increased risk of developing bone metastasis and/or a poor clinical outcome.

3. An *in vitro* method for designing a customized therapy for a subject suffering HER2+ breast cancer which comprises
i) quantifying the c-MAF gene expression level in a HER2+ sample of said subject and
ii) comparing the expression level obtained in i) with a reference value,
wherein if the expression level is increased with respect to said reference value, then said subject is susceptible to receive a therapy aiming to prevent and/or treat bone metastasis and/or a therapy aiming to avoid and/or prevent bone degradation,
wherein if the expression level is not increased with respect to said reference value, then said subject is not susceptible to receive a therapy aiming to prevent and/or treat bone metastasis and/or a therapy aiming to avoid and/or prevent bone degradation.

4. The method according to claim 3, wherein the therapy to avoid or prevent bone degradation is selected from the group consisting of a bisphosphonate, a RANKL inhibitor, a PTH inhibitor, a PTHLH inhibitor, a PRG analog, strontium ranelate, a DKK-1 inhibitor, a dual MET and VEGFR2 inhibitor, an estrogen receptor modulator, calcitonin, radium-223 and a cathepsin K inhibitor and/or the therapy to prevent and/or treat metastasis is selected from the group consisting of: a c-MAF inhibitory agent, systemic treatments including but not limited to: chemotherapy, hormone therapy, immunotherapy, radiotherapy, surgery, an mTor inhibitor, a Src kinase inhibitor, a CCR5 antagonist, a COX-2 inhibitor, and Alpharadin

5. The method according to claim 4, wherein the RANKL inhibitor is selected from the group consisting of a RANKL specific antibody, a RANKL-specific nanobody and osteoprotegerin, the bisphosphonate is zoledronic acid or clodronate, and the dual MET and VEGFR2 inhibitor Cabozantinib.

6. The method according to claim 5, wherein the RANKL specific antibody is denosumab.

7. The method of any one of claims 1-6, wherein determining the c-MAF gene expression level, amplification or copy number comprises quantifying the expression level, copy number or amplification of protein encoded by said gene or of a variant thereof, wherein the level, copy number or amplification of protein is quantified by means of western blot, ELISA, FISH, immunohistochemistry or a protein array or quantifying the messenger RNA (mRNA) of said gene, or a fragment of said mRNA, the complementary DNA (cDNA) of said gene, or a fragment of said cDNA.

8. A c-Maf inhibitory agent for use in the prevention, inhibition, or treatment of bone metastasis in HER2+ breast cancer in a subject that has been identified as having an amplification of the c-MAF gene or an increased expression level or copy number in comparison to a reference value, wherein said c-Maf inhibitory agent is selected from the group consisting of a MAF specific siRNA, a MAF specific antisense oligonucleotide, a c-Maf specific ribozyme, a c-Maf inhibitory antibody or nanobody, a dominant negative c-Maf variant, a compound from Table 1 or from Table 2, catalytic RNAs, DNA enzymes, inhibitory antibodies, inhibitory peptides, a c-Maf specific small molecule, a c-Maf specific antibody, a c-Maf specific antibody-like molecule, a c-Maf specific structurally constrained (cyclical) peptide, a c-Maf specific stapled peptide, and a c-Maf specific alphabody.

9. An agent for use in the prevention, inhibition, or treatment of bone metastasis or avoiding or preventing bone degradation in HER2+ breast cancer in a subject that has been identified as having an amplification of the c-MAF gene or an increased copy number in comparison to a reference value, wherein the agent is clodronate.

10. An agent for use in the prevention, inhibition, or treatment of bone metastasis or avoiding or preventing bone degradation in HER2+ breast cancer in a subject that has been identified as having an increased expression level in comparison to a reference value, wherein said agent is selected from the group consisting of a bisphosphonate, a RANKL inhibitor, a PTH or a PTHLH inhibitor or a PRG analog, strontium ranelate, a DKK-1 inhibitor, a dual MET and VEGFR2 inhibitor, an estrogen receptor modulator, calcitonin, Radium-223 and a cathepsin K inhibitor.

11. The agent of claim 10, wherein the RANKL inhibitor is selected from the group consisting of a RANKL specific antibody, a RANKL-specific nanobody and osteoprotegerin, the bisphosphonate is zoledronic acid or clodronate, and the dual MET and VEGFR2 inhibitor Cabozantinib.

12. The agent of claim 11, wherein the RANKL specific antibody is denosumab.

13. The agent of any one of claims 8-12, wherein determining the c-MAF gene expression level, amplification or copy number comprises quantifying the expression level, copy number or amplification of protein encoded by said gene or of a variant thereof, wherein the level, copy number or amplification of protein is quantified by means of western blot, ELISA, FISH, immunohistochemistry or a protein array or quantifying the messenger RNA (mRNA) of said gene, or a fragment of said mRNA, the complementary DNA (cDNA) of said gene, or a fragment of said cDNA.

14. A kit for predicting bone metastasis and/or clinical outcome of a HER2+ breast cancer in a subject suffering from said cancer and/or for determining a therapy for a subject suffering from breast cancer, the kit comprising: a) means for quantifying the expression level, amplification or copy number of c-MAF in a sample of said subject; b) means for comparing the quantified level of expression, amplification or copy number of c-MAF in said sample to a reference c-MAF level and, optionally, c) means for determining a therapy for preventing and/or reducing bone metastasis and/or avoiding or preventing bone degradation in said subject based on the comparison of the quantified expression level, amplification or copy number to the reference level.

15. An *in vitro* method for typing a sample of a subject suffering HER2+ breast cancer, the method comprising:
**(a)** providing a HER2+ breast cancer sample from said subject;
**(b)** quantifying the expression level of c-MAF in said sample;
**(c)** typing said sample by comparing the quantified expression level of c-MAF to a predetermined reference level;
wherein said typing provides prognostic information related to the risk of bone metastasis in said subject.

16. A method of classifying a subject suffering from HER2+ breast cancer into a cohort, comprising: a) determining the expression level of c-MAF in a HER2+ breast cancer sample of said subject; b) comparing the expression level of c-MAF in said sample to a predetermined reference level; and c) classifying said subject into a cohort based on said expression level of c-MAF in the sample.
